# EUROPÄISCHE PATENTSCHRIFT

(11) **EP 2 255 007 B1**
(45) Veröffentlichungstag und Bekanntmachung des Hinweises auf die Patenterteilung: **02.11.2016**
(21) Anmeldenummer: 09713531.3
(22) Anmeldetag: 19.02.2009
(51) Int. Cl.: C12N 15/52, C12P 7/16, C12N 9/04, C12N 9/88, C12N 9/10

(54) **FERMENTATIVE PRODUKTION VON ISOBUTANOL MIT HEFE**
FERMENTATIVE PRODUCTION OF ISOBUTANOL USING YEAST
PRODUCTION D'ISOBUTANOL PAR FERMENTATION AU MOYEN DE LEVURE

(30) Priorität: 20.02.2008 DE 102008010121
(43) Veröffentlichungstag der Anmeldung: 01.12.2010
(73) Patentinhaber: Butalco GmbH, 6363 Fürigen (CH)
(72) Erfinder: FESTEL, Gunter, CH-6331 Hünenberg/Zug (CH); BOLES, Eckhard, 64293 Darmstadt (DE); WEBER, Christian, 65779 Kelkheim am Taunus (DE); BRAT, Dawid, 65929 Frankfurt am Main (DE)
(74) Vertreter: Stolmár & Partner Patentanwälte PartG mbB
(86) Internationale Anmeldenummer: PCT/EP2009/001191
(87) Internationale Veröffentlichungsnummer: WO 2009/103533

(56) Entgegenhaltungen:
- VILLA K D ET AL: "CONTROL OF VICINAL DIKETONE PRODUCTION BY BREWER'S YEAST. I. EFFECTS OF ILV5 AND ILV3 GENE AMPLIFICATION ON VICINAL DIKETONE PRODUCTION AND ILV ENZYME ACTIVITY" JOURNAL OF THE AMERICAN SOCIETY OF BREWING CHEMISTS, AMERICAN SOCIETY OF BREWING CHEMISTS, ST PAUL, MN, US, Bd. 53, Nr. 2, 1. Januar 1995 (1995-01-01), Seiten 49-53, XP008070962 ISSN: 0361-0470
- VURALHAN ZEYNEP ET AL: "Identification and characterization of phenylpyruvate decarboxylase genes in Saccharomyces cerevisiae." APPLIED AND ENVIRONMENTAL MICROBIOLOGY AUG 2003, Bd. 69, Nr. 8, August 2003 (2003-08), Seiten 4534-4541, XP002532188 ISSN: 0099-2240
- DICKINSON J RICHARD ET AL: "An investigation of the metabolism of valine to isobutyl alcohol in Saccharomyces cerevisiae" JOURNAL OF BIOLOGICAL CHEMISTRY, AMERICAN SOCIETY OF BIOLOCHEMICAL BIOLOGISTS, BIRMINGHAM, US, Bd. 273, Nr. 40, 2. Oktober 1998 (1998-10-02), Seiten 25751-25756, XP002425679 ISSN: 0021-9258
- EDEN A ET AL: "Involvement of branched-chain amino acid aminotransferases in the production of fusel alcohols during fermentation in yeast." APPLIED MICROBIOLOGY AND BIOTECHNOLOGY APR 2001, Bd. 55, Nr. 3, April 2001 (2001-04), Seiten 296-300, XP002532189 ISSN: 0175-7598
- MATSUDA F ET AL: "Construction of an Artificial Pathway for Isobutanol Biosynthesis in the Cytosol of Saccharomyces cerevisiae", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 76, no. 11, 1 November 2012 (2012-11-01), pages 2139-2141, XP002698893, ISSN: 0916-8451, DOI: 10.1271/BBB.120420 [retrieved on 2012-11-07]
- DAWID BRAT ET AL: "Cytosolic re-localization and optimization of valine synthesis and catabolism enables increased isobutanol production with the yeast Saccharomyces cerevisiae Background", BIOTECHNOLOGY FOR BIOFUELS, vol. 5, 6 September 2012 (2012-09-06), pages 65-80, XP055084476,
- ATSUMI SHOTA ET AL: "Non-fermentative pathways for synthesis of branched-chain higher alcohols as biofuels", NATURE, NATURE PUBLISHING GROUP, UNITED KINGDOM, vol. 451, no. 7174, 3 January 2008 (2008-01-03), pages 86-89, XP002568324, ISSN: 0028-0836, DOI: 10.1038/NATURE06450

## Beschreibung

Die Erfindung betrifft einen fermentativen Weg zur Produktion von Isobutanol aus Zuckern.

Isobutanol hat hervorragende Eigenschaften als Kraftstoff. Darüberhinaus ist es auch eine interessante Chemikalie z.B. als Basischemikalie für die Produktion anderer Chemikalien oder als Lösungsmittel. Isobutanol wird heutzutage vornehmlich petrochemisch aus fossilen Ressourcen hergestellt. Wesentlich zukunftsträchtiger wäre dagegen seine Herstellung aus erneuerbaren Ressourcen wie z.B. pflanzlichen Zuckern oder Pflanzenabfällen. Kürzlich wurden zwei mikrobielle, nichtfermentative Verfahren vorgestellt, mit denen sich Isobutanol aus Zuckern herstellen lässt (Atsumi et al., 2008; Patentanmeldung US 2007/0092957). In beiden Verfahren wurden Wirtszellen durch Einführung heterologer DNA dazu gebracht, aus dem Stoffwechselintermediat Pyruvat, welches durch den Abbau von Zuckern entsteht, Isobutanol und auch andere verzweigtkettige Alkohole zu produzieren. Beiden beschriebenen Verfahren ist jedoch gemein, dass sie nicht-fermentativ ablaufen, d.h. dass ihre Redoxbalancen beim Abbau der Zucker zum Isobutanol nicht ausgeglichen sind. Daher können sie nur in komplexen Medien, durch gleichzeitige Umsetzung von Cosubstraten, durch die Bildung von Nebenprodukten oder unter aeroben Bedingungen ablaufen. Das schränkt die Ausführbarkeit der Verfahren stark ein bzw. macht sie wirtschaftlich unattraktiv.

In Villa K. D. et al. (J. Am. Soc. Brew. Chem. 53(2): 49-53, 1995) wird die Amplifizierung des ILV3 Gens oder des ILV5 Gens oder beider Gene gemeinsam in Bierhefen beschrieben, wobei die Amplifizierung durch Übertragung eines Plasmids herbeigeführt wird, das bakteriellen Ursprungs ist und in das entweder das *ILV3* Gen oder das *ILV5* Gen oder beide Gene gemeinsam kloniert wurden. Desweiteren wird eine Hefezelle beschrieben, in der die Gene *ILV3* oder *ILV5* oder beide Gene gemeinsam überexprimiert sind. Dadurch soll in der Hefezelle der metabolische Stofffluss durch den Isoleucin-Valin-Biosyntheseweg gesteigert werden, um die Produktion von vicinalen Diketonen und Vorläuferverbindungen davon zu verringern.

Ein Ausweg wäre die Entwicklung eines fermentativen mikrobiellen Prozesses, der in Minimalmedien, ohne Cosubstrate und auch unter anaeroben oder Sauerstoff-limitierten Bedingungen ablaufen könnte. Dafür würden sich als Mikroorganismen insbesondere Hefen und dabei insbesondere solche der Gattung *Saccharomyces* wie z.B. *Saccharomyces cerevisiae* anbieten. Interessanterweise besitzen Hefen bereits alle Enzyme, die für die Bildung von Isobutanol aus Zuckern notwendig sind. Allerdings sind diese Enzyme in verschiedenen Kompartimenten der Hefezellen lokalisiert (Cytosol und Mitochondrien), sie benutzen verschiedene Cofaktoren, die nicht oder nicht effektiv ineinander überführbar sind (NAD⁺/NADH und NADP⁺/NADPH) und die Enzyme werden nur schwach oder unter speziellen Bedingungen exprimiert bzw. besitzen eine niedrige Enzymaktivität. Um eine effektive Produktion von Isobutanol aus Zuckern zu erreichen, müssten die vorhandenen Stoffwechselwege so modifiziert werden, dass mit ihrer Hilfe redoxneutral und mit Energiegewinn in Form von ATP Isobutanol produziert werden könnte und das auch unter anaeroben oder Sauerstoff-limitierten Bedingungen. Die Entwicklung eines solchen fermentativen Weges zur Produktion von Isobutanol aus Zuckern ist Aufgabe und Ziel dieser Erfindung.

Zucker wie z.B. Glucose werden in Wirtszellen wie z.B. Hefen vornehmlich durch den Stoffwechselweg der Glykolyse zu Pyruvat abgebaut. Dabei entstehen aus einem Molekül Glucose zwei Moleküle Pyruvat. Zusätzlich entstehen dabei 2 energiereiche Verbindungen in Form von ATP und es werden 2 Moleküle NAD⁺ zu NADH+H⁺ reduziert. Pyruvat wird dann normalerweise entweder durch die Pyruvatdecarboxylasen und Alkoholdehydrogenasen zu Ethanol umgewandelt oder es wird in die Mitochondrien transportiert und dort durch die Pyruvatdehydrogenase in Acetyl-CoA umgewandelt und letztendlich in den Zitronensäurezyklus eingeschleust. Pyruvat kann darüber hinaus auch noch in einigen anderen Reaktionen umgesetzt werden. Einer dieser Reaktionswege ist der Biosyntheseweg zur Aminosäure Valin. Andererseits kann Valin aber auch abgebaut werden und zwar u.a. zum Produkt Isobutanol. Wenn es nun gelänge, den Biosyntheseweg und den Abbauweg des Valins kurzzuschließen, dann ließe sich direkt aus Zuckern über das Pyruvat Isobutanol produzieren. Solch ein Stoffwechselweg kombiniert die Enzyme, die an der Biosynthese von Valin beteiligt sind (vom Pyruvat zum α-Ketoisovalerat), mit denen, die am Valin-Abbau beteiligt sind (vom α-Ketoisovalerat zum Isobutanol). Die Hefe *Saccharomyces cerevisiae* besitzt selber alle dazu benötigten Gene. Dabei kodiert *ILV2* (*YMR108W*) (SEQ.ID. Nr. 1) die Acetolactat Synthase, die zwei Pyruvat-Moleküle zum Acetolactat umwandelt. Das Ilv2-Enzym (SEQ.ID. Nr. 2) wird vom Ilv6-Protein (=YCL009C) (SEQ.ID. Nr. 4) aktiviert. *ILV5* (*YLR355C*) (SEQ.ID. Nr. 5) kodiert die Acetohydroxysäure Reductoisomerase, die Acetolactat zum 2,3-Dihydroxyisovalerat umsetzt. *ILV3* (*YJR016C*) (SEQ.ID. Nr. 7) kodiert die Dihydroxysäure Dehydratase, die 2,3-Dihydroxyisovalerat zum 2-Ketoisovalerat umsetzt. 2-Ketoisovalerat wird dann normalerweise zum Valin transaminiert; durch die Transaminasen Bat1 (SEQ.ID. Nr. 10) und Bat2 (SEQ.ID. Nr. 12). Umgeht oder reduziert man diese Reaktion, dann könnte 2-Ketoisovalerat aber auch durch verschiedene 2-Ketosäure Decarboxylasen zum Isobutyraldehyd umgesetzt werden, z.B. durch die Enzyme Pdc1 (SEQ.ID. Nr. 14), Pdc5 (SEQ.ID. Nr. 16), Pdc6 (SEQ.ID. Nr. 18), Aro10 (SEQ.ID. Nr. 20), Thi3 (SEQ.ID. Nr. 22) (Dickinson et al., 1998; 2003). Diese direkte Umwandlung wird normalerweise unter anderem durch die unterschiedliche Kompartimentierung der Enzyme (Mitochondrien, Cytosol) verhindert. Isobutyraldehyd kann dann durch verschiedene Alkoholdehydrogenasen schließlich zum Isobutanol reduziert werden (Dickinson et al., 2003). Dazu gehören u.a. Adh1-7 (SEQ.ID. Nr. 24), (SEQ.ID. Nr. 26), (SEQ.ID. Nr. 28), (SEQ.ID. Nr. 30), (SEQ.ID. Nr. 32), (SEQ.ID. Nr. 34), (SEQ.ID. Nr. 36), Sfa1 (SEQ.ID. Nr. 38), Ypr1 (SEQ.ID. Nr. 40).

Die meisten der genannten Enzyme werden jedoch für eine effiziente Produktion von Isobutanol aus Pyruvat bzw. Zuckern nicht stark genug exprimiert bzw. weisen geringe Enzymaktivitäten auf. Ein anderes Problem ist die Cofaktorspezifität und Redoxbilanz. Bei der Reduktion der zwei aus der Glykolyse stammenden Moleküle Pyruvat zu Isobutanol werden ein Molekül NADPH von der Acetohydroxysäure Reduktoisomerase und ein Molekül NADH oder NADPH von den verzweigtkettigen Alkoholdehydrogenasen benötigt. In der Glykolyse werden aber aus einem Molekül Glucose zwei Moleküle NADH in der Glycerinaldehyd-3-Phosphat Dehydrogenase Reaktion produziert. Daher besteht ein Defizit an NADPH und ein Überschuss an NADH. NADH ist aber nicht ohne weiteres in NADPH zu überführen. Andererseits befinden sich die Enzyme Ilv2/Ilv6, Ilv5 (SEQ.ID. Nr. 6) und Ilv3 (SEQ.ID. Nr. 8) zumindest hauptsächlich in den Mitochondrien der Hefezellen. Deshalb muss zunächst das Pyruvat in die Mitochondrien hinein und letztendlich das 2-Ketoisovalerat aus den Mitochondrien heraus ins Cytosol transportiert werden. Da Transport über Membranen oft limitierend auf Stoffflüsse wirken kann, wäre es deshalb wünschenswert, alle Reaktionen ins Cytosol zu verlagern. Ebenso nachteilig für eine effiziente Produktion von Isobutanol ist, dass einige Intermediate auf dem Weg vom Zucker zum Produkt für andere Stoffwechselreaktionen abgezogen werden. Das gilt vor allen Dingen für das Pyruvat, das durch die Pyruvatdecarboxylasen und Alkoholdehydrogenasen zu einem großen Teil in Ethanol umgewandelt wird. Daher ist es für eine effizientere Produktion von Isobutanol wichtig, diese Nebenreaktionen zu reduzieren bzw. ganz auszuschalten. Aufgabe und Ziel dieser Erfindung ist es daher, einen fermentativen Weg zur Produktion von Isobutanol aus Zuckern zur Verfügung zu stellen, bei dem (i) die eigene Enzymausstattung der Hefe für den Stoffwechselweg vom Pyruvat zum Isobutanol genutzt wird, indem ihre Expression bzw. Aktivitäten gesteigert werden, d.h. ohne dass heterologe Gene in die Hefe eingeführt werden müssen, (ii)a) die Kofaktorspezifität der Acetohydroxysäure Reduktoisomerase so geändert wird, dass dieses Enzym bevorzugt NADH anstelle von NADPH als Kofaktor benutzt, oder (ii)b) die Kofaktorspezifität der Glycerinaldehyd-3-Phosphat Dehydrogenase so geändert wird, dass dieses Enzym bevorzugt NADP⁺ anstelle von NAD⁺ als Kofaktor benutzt bzw. eine heterologe NADP-Glycerinaldehyd-3-Phosphat Dehydrogenase in den Hefezellen exprimiert wird, (iii) die Bildung von Nebenprodukten wie z.B. Ethanol minimiert ist und (iv) bei dem möglichst alle beteiligten Enzyme im Cytosol der Hefezellen lokalisiert sind.

Die Aufgabe wird erfindungsgemäß gelöst durch Überexpression der Enzymaktivitäten von Ilv2 mit oder ohne seinen Aktivator Ilv6, Ilv5, Ilv3, mindestens einer 2-Ketosäure Decarboxylase wie z.B. Aro10 und mindestens einer Alkoholdehydrogenase, die auch Isobutyraldehyd reduzieren kann (bevorzugt Adh1 oder Adh6, aber auch Adh2-5, Sfa1, Ypr1 oder andere). Dieses geschieht zum einen durch den Austausch der jeweiligen Promotoren der entsprechenden Gene gegen stärkere Promotoren, bevorzugt, aber nicht ausschließlich, konstitutive Promotoren. Bevorzugterweise aber nicht ausschließlich sind Promotorsequenzen ausgewählt aus HXT7, verkürzter HXT7, PFK1, FBA1, TPI1, PGK1, PMA1, ADH1, TDH3. Weiterhin werden die entsprechenden Nukleinsäuresequenzen der Gene abgewandelt in codon-optimierte Allele. Jede Aminosäure wird auf Genebene durch ein Codon verschlüsselt. Jedoch gibt es für die meisten Aminosäuren mehrere verschiedene Codons, die für eine einzelne Aminosäure codieren. Der genetische Code ist folglich degeneriert. Die bevorzugte Codonwahl für eine entsprechende Aminosäure ist von Organismus zu Organismus verschieden. So kann es bei heterolog exprimierten Genen zu Problemen kommen, wenn der Wirtsorganismus bzw. die Wirtszelle einen sehr unterschiedlichen Codon-Gebrauch aufweist. Das Gen kann überhaupt nicht oder nur langsam exprimiert werden. Aber auch in Genen von verschiedenen Proteinen und Stoffwechselwegen innerhalb einer Zelle ist ein unterschiedlicher Codon-Gebrauch festzustellen. Von den Glykolyse-Genen aus *S. cerevisiae* ist bekannt, dass sie stark exprimiert werden. Sie weisen einen stark restriktiven Codon-Gebrauch auf, der in etwa den Mengenverhältnissen der entsprechenden tRNAs entspricht. Die Anpassung des Codon-Gebrauchs der Gene *ILV2,* (*ILV6*) (SEQ.ID. Nr. 3), *ILV5, ILV3,* eines der oben genannten 2-Ketosäure Decarboxylase-Gene und eines der oben genannten Alkoholdehydrogenase-Gene an den bevorzugten Codon-Gebrauch von *S. cerevisiae* führt zu einer Verbesserung der Isobutanol-Bildungsrate in Hefe. Der bevorzugte Codon-Gebrauch kann definiert sein wie in Wiedemann und Boles (2008) für die glykolytischen Gene beschrieben, muss aber nicht notwendigerweise auf diese Beispiele beschränkt sein. Die überexprimierten, möglicherweise codon-optimierten Gene können entweder auf Plasmiden kloniert in die Hefezellen eingebracht werden, sie können ins Genom der Hefezellen integriert werden oder sie können die natürlich vorkommenden Allele genomisch ersetzen.

Die vorliegende Erfindung betrifft daher in einer ersten Ausführungsform eine Hefezelle, die Isobutanol produziert, dadurch gekennzeichnet, dass die Zelle einen gesteigerten metabolischen Stofffluss vom Pyruvat über Acetolactat, 2,3-Dihydroxyisovalerat, 2-Ketoisovalerat, Isobutyraldehyd zum Isobutanol aufweist, indem alle Gene, die für die Enzyme kodieren, die an dieser Umsetzung beteiligt sind, überexprimiert werden und dass diese Gene homolog zu der besagten Hefezelle sind, wobei Ilv2 die Acetolactat Synthase Reaktion vom Pyruvat zum Acetolactat katalysiert, Ilv5 die Acetohydroxysäure Reductoisomerase Reaktion vom Acetolactat zum 2,3-Dihydroxyisovalerat katalysiert, Ilv3 die Dihydroxysäure Dehydratase Reaktion vom 2,3-Dihydroxyisovalerat zum 2-Ketoisovalerat katalysiert, eine 2-Ketosäure Decarboxylase die Reaktion vom 2-Ketoisovalerat zum Isobutyraldehyd katalysiert, und eine Alkoholdehydrogenase die Reaktion vom Isobutyraldehyd zum Isobutanol katalysiert, wobei entweder mindestens einer der Promotoren dieser Gene gegen mindestens einen stärkeren Promotor ausgetauscht wird oder die Nukleinsäuresequenzen dieser Gene in Codon-optimierte Allele abgewandelt werden, dadurch gekennzeichnet, dass die Gene *ILV2, ILV5* und *ILV3* ohne die mitochondrielle Targetingsequenz der Enzyme Acetolactat Synthase, Acetohydroxysäure Reductoisomerase und Dihydroxysäure Dehydratase oder mit einer zerstörten, inaktivierten mitochondriellen Targetingsequenz überexpremiert werden, und dadurch gekennzeichnet, dass zusätzlich die Expression der Gene *PDC1* mit SEQ.ID. Nr. 13, *PDC5* mit SEQ.ID. Nr. 15 und *PDC6* mit SEQ.ID. Nr. 17 bzw. die Aktivität der kodierten Enzyme reduziert bzw. ausgeschaltet ist.

In einer bevorzugten Ausführungsform ist die erfindungsgemäße Hefezelle dadurch gekennzeichnet, dass der mindestens eine stärkere Promotor ein konstitutiver Promotor ist.

In einer weiteren bevorzugten Ausführungsform ist die erfindungsgemäße Hefezelle dadurch gekennzeichnet, dass die Promotorsequenz ausgewählt ist aus der Gruppe bestehend aus HXT7, verkürzter HXT7, PFK1, FBA1, TPI1, PGK1, PMA1, ADH1 und TDH3.

In einer weiteren bevorzugten Ausführungsform ist die erfindungsgemäße Hefezelle dadurch gekennzeichnet, dass die 2-Ketosäure Decarboxylase ausgesucht ist aus mindestens einem der Enzyme Aro10 oder Thi3.

In einer weiteren bevorzugten Ausführungsform ist die erfindungsgemäße Hefezelle dadurch gekennzeichnet, dass die Alkoholdeyhdrogenase ausgewählt ist aus mindestens einem der Enzyme Adh1 mit SEQ.ID. Nr. 24, Adh2 mit SEQ.ID. Nr. 26), Adh3 mit SEQ.ID. Nr. 28), Adh4 mit SEQ.ID. Nr. 30, Adh5 mit SEQ.ID. Nr. 32, Adh6 mit SEQ.ID. Nr. 34), Adh7 mit SEQ.ID. Nr. 36, Sfa1 mit SEQ.ID. Nr. 38 oder Ypr1 mit SEQ.ID. Nr. 40.

In einer weiteren bevorzugten Ausführungsform ist die erfindungsgemäße Hefezelle dadurch gekennzeichnet, dass alle diese Gene in codon-optimierten Varianten überexprimiert werden.

In einer weiteren bevorzugten Ausführungsform ist die erfindungsgemäße Hefezelle dadurch gekennzeichnet, dass alle diese Gene in codon-optimierten Varianten überexprimiert werden, wobei die Codon-Optimierung am Codon-Gebrauch der hochexprimierten Glykolysegene der Hefe ausgerichtet ist.

In einer weiteren bevorzugten Ausführungsform ist die erfindungsgemäße Hefezelle dadurch gekennzeichnet, dass die Zelle eine Acetohydroxysäure Reductoisomerase exprimiert, die eine erhöhte Spezifität für NADH gegenüber NADPH aufweist.

In einer weiteren bevorzugten Ausführungsform ist die erfindungsgemäße Hefezelle dadurch gekennzeichnet, dass diese NADH-bevorzugende Acetohydroxysäure Reductoisomerase eine mutierte Variante des Ilv5-Enzyms der Hefe ist.

In einer weiteren bevorzugten Ausführungsform ist die erfindungsgemäße Hefezelle dadurch gekennzeichnet, dass gleichzeitig eine NADH-bevorzugende Alkoholdehydrogenase der Hefe überexprimiert wird, die Isobutyraldehyd in Isobutanol umwandelt.

In einer weiteren bevorzugten Ausführungsform ist die erfindungsgemäße Hefezelle dadurch gekennzeichnet, dass die Zelle zusätzlich eine phosphorylierende Glycerinaldehyd-3-Phosphat Dehydrogenase exprimiert, die eine erhöhte Spezifität für NADP⁺ gegenüber NAD⁺ aufweist.

In einer weiteren bevorzugten Ausführungsform ist die erfindungsgemäße Hefezelle dadurch gekennzeichnet, dass diese NADP-bevorzugende Glycerinaldehyd-3-Phosphat Dehydrogenase heterolog zur Hefewirtszelle ist.

In einer weiteren bevorzugten Ausführungsform ist die erfindungsgemäße Hefezelle dadurch gekennzeichnet, dass diese NADP-Glycerinaldehyd-3-Phosphat Dehydrogenase durch mutierte Allele eines, zweier oder aller drei *TDH1-3* (SEQ.ID. Nr. 41), (SEQ.ID. Nr. 43), (SEQ.ID. Nr. 45) Gene der Hefe kodiert wird. In einer weiteren bevorzugten Ausführungsform ist die erfindungsgemäße Hefezelle dadurch gekennzeichnet, dass diese NADP-Glycerinaldehyd-3-Phosphat Dehydrogenase in einer Hefezelle exprimiert wird, die keine oder eine reduzierte Expression oder Aktivität der NAD-Glycerinaldehyd-3-Phosphat Dehydrogenasen aufweist.

In einer weiteren bevorzugten Ausführungsform ist die erfindungsgemäße Hefezelle dadurch gekennzeichnet, dass gleichzeitig eine NADPH-bevorzugende Alkoholdehydrogenase überexprimiert wird, die Isobutyraldehyd in Isobutanol umwandelt.

In einer weiteren bevorzugten Ausführungsform ist die erfindungsgemäße Hefezelle dadurch gekennzeichnet, dass zusätzlich das Ilv6-Protein im selben Zellkompartiment wie Ilv2 überexprimiert wird.

In einer weiteren bevorzugten Ausführungsform ist die erfindungsgemäße Hefezelle dadurch gekennzeichnet, dass zusätzliche Mutationen die Produktion von Isobutanol steigern. In einer weiteren bevorzugten Ausführungsform ist die erfindungsgemäße Hefezelle dadurch gekennzeichnet, dass zusätzliche Mutationen die Resistenz gegenüber toxischen Konzentrationen von Isobutanol steigern.

In einer weiteren bevorzugten Ausführungsform ist die erfindungsgemäße Hefezelle dadurch gekennzeichnet, dass die Zelle ausgewählt ist aus folgender Gruppe: *Pichia, Candida, Hansenula, Kluyveromyces, Yarrowia* und *Saccharomyces.*

In einer weiteren bevorzugten Ausführungsform ist die erfindungsgemäße Hefezelle dadurch gekennzeichnet, dass die Wirtszelle *Saccharomyces cerevisiae* ist.

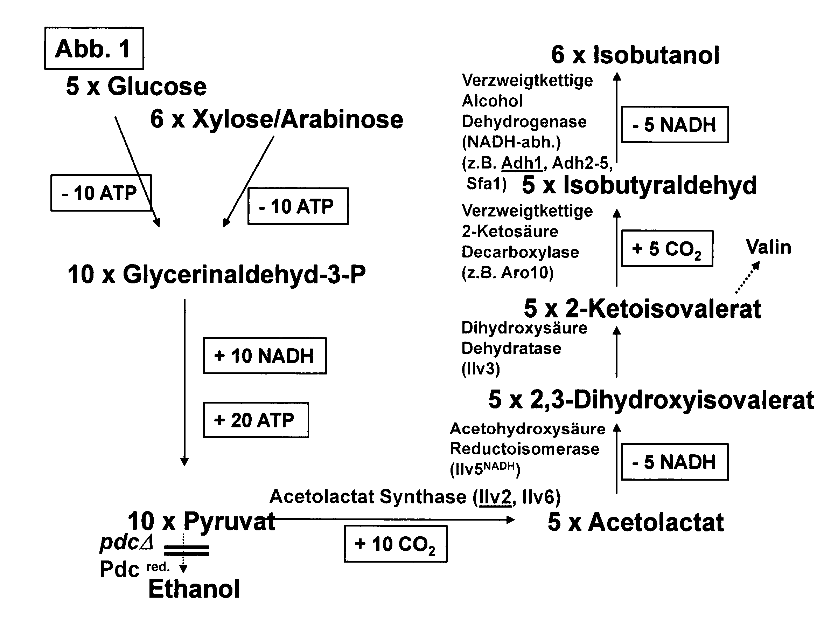

In einer möglichen Ausführungsform der Erfindung (s. Abb. 1) wird das Enzym Ilv5 (Acetohydroxysäure Reduktoisomerase) so verändert, dass es bevorzugt NADH anstelle von NADPH als Cofaktor verwendet. Gleichzeitig wird bevorzugt, aber nicht notwendigerweise, eine Alkoholdehydrogenase überexprimiert, die auch NADH als Cofaktor verwendet (z.B. Adh1 oder Adh2-5 oder Sfa1). Ilv5 katalysiert die Reduktion von Acetolactat zum 2,3-Dihydroxyisovalerat bei gleichzeitiger Oxidation von NADPH+H⁺ zum NADP⁺. Durch den glykolytischen Abbau von Zuckern entsteht jedoch kein oder nur in geringen Mengen NADPH. Es entsteht jedoch NADH. NADH ist aber nicht ohne weiteres in NADPH umzuwandeln (Boles et al., 1993). Deshalb wäre es wünschenswert, die Kofaktorspezifität der Acetohydroxysäure Reduktoisomerase so zu verändern, dass dieses Enzym NADH anstelle von NADPH bevorzugt. Dieses kann erreicht werden, indem bestimmte Aminosäuren von Ilv5, die für die ausschließliche Verwendung des NADPH benötigt werden, durch andere ersetzt werden, die auch oder die bevorzugt eine Verwendung von NADH zulassen. Solche Aminosäuren sind bevorzugt, aber nicht ausschließlich, die Aminosäuren Arg108, Gly111, Ala112 und/oder Ser113 des nicht-prozessierten Vorläuferenzyms, die sich durch einen Vergleich des Hefe-Ilv5 Enzyms mit der Struktur der Acetohydroxysäure Reduktoisomerase von Spinat ableiten lassen (Biou et al., 1997). Arg108 kann dabei bevorzugt, aber nicht ausschließlich, in Met, Trp, Phe, Glu oder Asp umgewandelt werden, Gly111 bevorzugt, aber nicht ausschließlich, in Glu oder Asp, Ala112 bevorzugt, aber nicht ausschließlich, in Ser oder Gly und Ser113 bevorzugt, aber nicht ausschließlich, in Glu oder Asp. Es soll jedoch nicht ausgeschlossen werden, dass auch der Austausch weiterer oder anderer Aminosäuren zu einer Veränderung der Kofaktorspezifität von Ilv5 zugunsten von NADH führt.

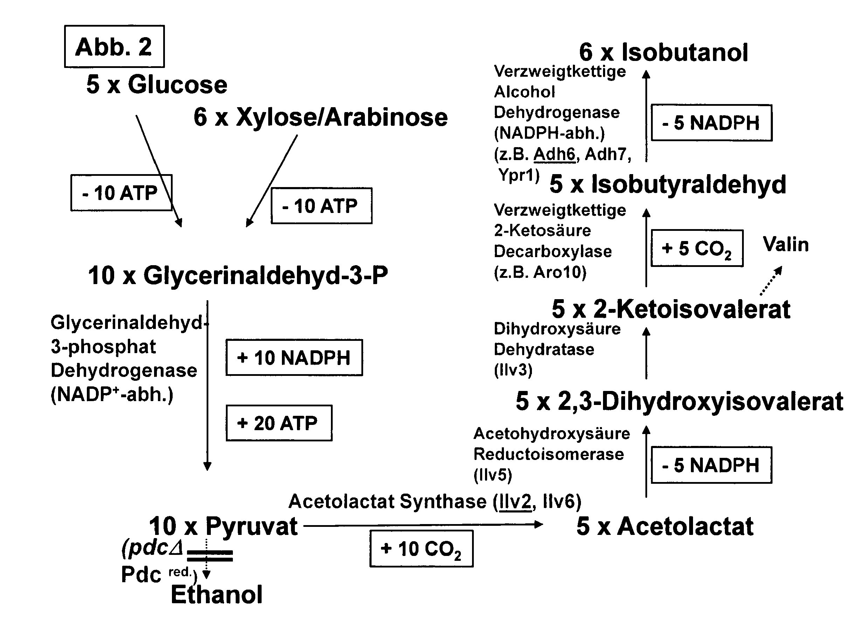

In einer anderen möglichen Ausführungsform der Erfindung (s. Abb. 2) wird die Glycerinaldehyd-3-Phosphat Dehydrogenase (GAPDH) der Hefe so verändert, dass sie NADP⁺ anstelle von NAD⁺ bevorzugt, oder durch eine Glycerinaldehyd-3-Phosphat Dehydrogenase ersetzt bzw. ergänzt, die NADP⁺ gegenüber NAD⁺ bevorzugt. Gleichzeitig wird bevorzugt, aber nicht notwendigerweise, eine Alkoholdehydrogenase überexprimiert, die NADPH als Cofaktor bevorzugt (z.B. Adh6 oder Ypr1). GAPDH wird z.B. in *S. cerevisiae* von den Genen *IDH1* (SEQ.ID. Nr. 41), *TDH2* (SEQ.ID. Nr. 43) und *TDH3* (SEQ.ID. Nr. 45) kodiert und katalysiert die Oxidation von Glycerinaldehyd-3-Phosphat bei gleichzeitiger Phosphorylierung zum 1,3-Diphosphoglycerat. Beim glykolytischen Abbau von Zuckern wird dabei normalerweise NAD⁺ als Kofaktor verwendet, und es entsteht NADH+H⁺. NADH ist nicht ohne weiteres in NADPH umzuwandeln (Boles et al., 1993). Da jedoch die Acetohydroxysäure Reduktoisomerase NADPH als Kofaktor verwendet, wäre es wünschenswert, die Kofaktorspezifität der GAPDH so zu verändern, dass dieses Enzym NADP⁺ anstelle von NAD⁺ bevorzugt. Eine Änderung der Kofaktorspezifität der Hefe-GAPDH kann erreicht werden, indem bestimmte Aminosäuren von Tdh1 (SEQ.ID. Nr. 42), Tdh2 (SEQ.ID. Nr. 44) und/oder Tdh3 (SEQ.ID. Nr. 46), die für die ausschließliche Verwendung des NAD⁺ benötigt werden, durch andere ersetzt werden, die auch oder die bevorzugt eine Verwendung von NADP⁺ zulassen. Solche Aminosäuren sind bevorzugt, aber nicht ausschließlich, die Aminosäuren Asp33 und/oder Gly188-Pro189, die sich durch einen Vergleich der Hefe-GAPDH Enzyme mit der Struktur von NADP⁺-bevorzugenden GAPDH ableiten lassen (Fillinger et al., 2000). Asp33 kann dabei bevorzugt, aber nicht ausschließlich, in Asn, Gly, Ala oder Ser umgewandelt werden, Gly188-Pro189 bevorzugt, aber nicht ausschließlich, in Ala-Ser, Val-Arg, Asn-Pro oder Thr-Lys. Es soll jedoch nicht ausgeschlossen werden, dass auch der Austausch weiterer oder anderer Aminosäuren zu einer Veränderung der Kofaktorspezifität von Tdhl-3 zugunsten von NADP⁺ führt. Alternativ könnte eine heterologe GAPDH in Hefe überexprimiert werden, die bevorzugt NADP⁺ verwendet, z.B. aber nicht ausschließlich Gdp1 (SEQ.ID. Nr. 48) aus *Kluyveromyces lactis* (Verho et al., 2002) oder GapB (SEQ.ID. Nr. 50) aus *Bacillus subtilis* (Fillinger et al., 2000). Die NADP-GAPDH kann in einer bevorzugten Ausführungsform codon-optimiert überexprimiert werden. Die mutierten oder heterologen NADP-GAPDHs können dabei zusätzlich zu den vorhandenen NAD-GAPDHs exprimiert werden oder in einer bevorzugten Ausführung in Hefemutanten mit reduzierter oder ausgeschalteter NAD-GAPDH Expression bzw. Aktivität.

Proteine, die in die mitochondriale Matrix transportiert werden, werden als Vorläuferproteine im Cytosol synthetisiert und dann über Translocasen in die mitochondriale Matrix transportiert. Die N-terminalen Presequenzen werden von einer mitochondrialen Peptidase während der Translocation abgespalten. Im Rahmen der Erfindung werden die Gene *ILV2,* (*ILV6*), *ILV5* bzw. *ILV5^{(NADHmut.)}* und *ILV3* ohne die mitochondrielle Targetingsequenz der entsprechenden Proteine oder mit einer zerstörten, inaktivierten mitochondriellen Targetingsequenz überexprimiert, sodass die produzierten Proteine bevorzugt im Cytosol der Hefezellen lokalisiert sind (Pang and Duggleby, 1999; Omura, 2008). Dieses kann mit den natürlichen oder codon-optimierten Allelen geschehen.

Pyruvat kann in verschiedenen Reaktionswegen weiter umgesetzt werden. Der mengenmäßig stärkste dieser Reaktionswege ist seine Umsetzung zum Ethanol. Dabei wird Pyruvat durch die Pyruvatdecarboxylasen (Pdcs) zu Acetaldehyd decarboxyliert und weiter zum Ethanol umgesetzt. Dabei geht Pyruvat für die Produktion von Isobutanol verloren. In einer bevorzugten Ausführungsform der Erfindung, aber nicht notwendigerweise, wird daher der Fluss des Pyruvat zum Ethanol durch Ausschalten bzw. Verringerung der Pyruvatdecarboxylase-Expression oder - Aktivitäten blockiert bzw. reduziert. Dies geschieht z.B. durch Deletion bzw. Reduktion der Expression der Gene *PDC1, PDC5* und/oder *PDC6.* Da jedoch die Hefe das aus dem Acetaldehyd im Cytosol hergestellte Acetyl-CoA benötigt, muss dieses bei vollständiger Ausschaltung der Pyruvatdecarboxylasen zusätzlich zur Verfügung gestellt werden. Dieses geschieht entweder (i) durch eine nicht vollständige Ausschaltung der Expression oder Aktivität der Pyruvatdecarboxylasen, (ii) durch Expression einer heterologen Pyruvat-Formiat-Lyase mit ihrem aktivierenden Enzym einschließlich der Überexpression einer Formiatdehydrogenase, (iii) durch heterologe Expression eines reversiblen mitochondrialen Carnitin-Carriers oder (iv) durch die Einführung von spontanen Suppressormutationen. Darüberhinaus bleibt die Reduktion oder Ausschaltung weiterer Stoffwechselreaktionen vorbehalten, um den Fluss der Intermediärmetabolite zum Isobutanol zu verstärken.

Weiterhin kann die Produktion von Isobutanol sowie die Resistenz gegenüber toxischen Konzentrationen von Isobutanol in den rekombinanten Hefezellen durch Zufallsmutagenese oder die Methoden des "Evolutionary Engineerings" oder der "Directed Evolution" (Sauer, 2001) noch gesteigert werden.

Die vorliegende Erfindung betrifft desweiteren ein Verfahren für die Produktion von Isobutanol mit Hefezellen, umfassend die Bereitstellung einer Hefezelle wie vorstehend definiert sowie das Inkontaktbringen der Hefezelle mit einer fermentierbaren Kohlenstoffquelle.

In einer bevorzugten Ausführungsform ist das erfindungsgemäße Verfahren dadurch gekennzeichnet, dass die fermentierbare Kohlenstoffquelle eine C3 - C6 Kohlenstoffquelle ist.

In einer weiter bevorzugten Ausführungsform ist das erfindungsgemäße Verfahren dadurch gekennzeichnet, dass die Kohlenstoffquelle zu der Gruppe bestehend aus Monosacchariden, Oligosacchariden oder Polysacchariden gehört.

In einer weiter bevorzugten Ausführungsform ist das erfindungsgemäße Verfahren dadurch gekennzeichnet, dass die Kohlenstoffquelle zu der Gruppe bestehend aus Glucose, Fructose, Mannose, Galactose, Saccharose, Maltose, Xylose oder Arabinose gehört.

In einer weiter bevorzugten Ausführungsform ist das erfindungsgemäße Verfahren dadurch gekennzeichnet, dass die Wirtszelle mit der Kohlenstoffquelle in Kulturmedium in Kontakt gebracht wird.

### Methoden

### 1. Stämme und Medien

### 1.1 Bakterien

- *E. coli* SURE (Stratagene)
- *E.coli* DH5α (Stratagene)

Vollmedium LB 1% Trypton, 0,5 % Hefeextrakt, 0,5% NaCl, pH 7,5 (siehe Maniatis, 1982).
Für die Selektion auf eine plasmidkodierte Antibiotikaresistenz wurde dem Medium nach dem Autoklavieren 40µg/ml Ampicillin zugesetzt. Feste Nährmedien enthielten zusätzlich 2% Agar. Die Anzucht erfolgte bei 37°C.

### 1.2 Hefe

Stämme aus der Serie CEN.PK, Industriehefen
- synthetisches Komplettselektivmedium SC:
   0,67% Yeast nitrogen base w/o amino acids, pH6,3, Aminosäure/Nukleobase-Lösung, Kohlenstoffquelle in der jeweils angegeben Konzentration
- synthetisches Minimalselektivmedium SM:
   0,16% Yeast nitrogen base w/o amino acid and ammonium sulphate, 0,5% Ammoniumsulfat ,20mM Kaliumdihydrogenphosphat, pH6,3, Kohlenstoffquelle in der jeweils angegeben Konzentration
- synthetisches Fermentationsmedium (Mineralmedium) SFM: (Verduyn *et al.,* 1992), pH 5,5
   Salze: (NH₄)₂SO₄, 5g/l; KH₂PO₄, 3g/l; MgSO₄*7H₂O, 0,5g/l
   Spurenelemente: EDTA, 15mg/l, ZnSO₄*4,5mg/l; MnCl₂*4H₂O, 0,1mg/l; CoCl₂*6H₂O, 0,3 mg/l; CuSO₄, 0,192 mg/l; Na₂MoO₄*2H₂O, 0,4 mg/l;CaCl₂*2H₂O, 4,5 mg/l; FeSO₄*7H₂O, 3 mg/l; H₃BO₃, 1 mg/l; KI, 0,1 mg/l
   Vitamine: Biotin, 0,05 mg/l; p-Aminobenzoesäure, 0,2 mg/l; Nicotinsäure, 1 mg/l; Calciumpantothenat, 1 mg/l; Pyridoxin-HCl, 1 mg/l; Thiamin-HCl, 1 mg/l; Minositol, 25 mg/l

Konzentration der Aminosäuren und Nukleobasen im synthetischen Komplettmedium (nach Zimmermann, 1975): Adenin (0,08mM), Arginin (0,22mM), Histidin (0,25mM), Isoleucin (0,44mM), Leucin (0,44mM), Lysin (0,35mM), Methionin (0,26mM), Phenylalanin (0,29mM), Tryptophan (0,19mM), Threonin (0,48mM), Tyrosin (0,34mM), Uracil (0,44mM), Valin (0,49mM). Als Kohlenstoffquelle wurden L-Arabinose und D-Glucose eingesetzt.

Feste Voll- und Selektivmedien enthielten zusätzlich 1,8 % Agar. Die Anzucht der Hefezellen erfolgte bei 30°C. Das für die Fermentationen eingesetzte synthetische Mineralmedium enthielt Salze, Spurenmetalle und Vitamine in den oben aufgelisteten Konzentrationen und L-Arabinose als Kohlenstoffquelle. Von den Spurenmetallen und den Vitaminen wurde eine Stammlösung angesetzt. Beide Lösungen wurden steril-filtriert. Beide wurden bei 4°C gelagert. Für die Herstellung der Spurenmetalllösung war der pH-Wert von entscheidender Rolle. Die verschiedenen Spurenelemente mussten in der obigen Reihenfolge nacheinander in Wasser vollständig gelöst werden. Nach jeder Zugabe musste der pH-Wert mit KOH auf 6,0 eingestellt werden, bevor das nächste Spurenelement zugegeben werden konnte. Am Ende wurde der pH-Wert mit HCl, auf 4,0 justiert. Um Schaumbildung zu vermeiden, wurde dem Medium 200µl Antischaum (Antifoam2004, Sigma) zugegeben. Da die Versuche unter anaeroben Bedingungen durchgeführt wurden, musste dem Medium nach dem Autoklavieren noch 2,5 ml/l einer Tween80-Ergosterol-Lösung zugegeben werden. Diese besteht aus 16,8 g Tween80 und 0,4 g Ergosterol, welche mit Ethanol auf 50 ml aufgefüllt und darin gelöst wurden. Die Lösung wurde steril-filtriert. Die Salze und der Antischaum wurden gemeinsam mit dem kompletten Fermenter autoklaviert. Die Arabinose wurde getrennt vom restlichen Medium autoklaviert. Nach Abkühlen des Mediums wurden die Spurenelemente sowie die Vitamine hinzugegeben.

### 2. Transformation

### 2.1 Transformation von E. coli

Die Transformation der *E. coli* Zellen erfolgte durch die Elektroporationsmethode nach Dower et *al.* (1988) und Wirth (1993) mittels eines Easyject prima Geräts (EQUIBO).

### 2.2 Transformation von S. cerevisiae

Die Transformation von *S. cerevisiae* Stämmen mit Plasmid-DNA bzw. DNA-Fragmenten erfolgte nach der Lithiumacetat-Methode von Gietz und Woods (1994).

### 3. Präparation von DNA

### 3.1 Isolierung von Plasmid-DNA aus E. coli

Die Isolierung von Plasmid-DNA aus *E. coli* erfolgte nach der Methode der alkalischen Lyse von Birnboim und Doly (1979), modifiziert nach Maniatis *et al.* (1982) oder alternativ mit dem "QIAprep Spin Miniprep Kit" der Firma Qiagen. Hochreine Plasmid-DNA für Sequenzierungen wurde mit dem "Plasmid Mini Kit" der Firma Qiagen nach Angaben des Herstellers präpariert.

### 3.2 Isolierung von Plasmid-DNA aus S. cerevisiae

Die Zellen einer stationären Hefekultur (5ml) wurden durch Zentrifugation geerntet, gewaschen und in 400µl Puffer P1 (Plasmid Mini Kit, Firma Qiagen) resuspendiert. Nach Zugabe von 400µl Puffer P2 und 2/3 Volumen Glasperlen (Ø 0,45 mm) erfolgte der Zellaufschluss durch 5-minütiges Schütteln auf einem Vibrax (Vibrax-VXR von Janke & Kunkel oder IKA). Der Überstand wurde mit ½ Volumen Puffer P3 versetzt, gemischt und für 10min auf Eis inkubiert. Nach einer 10-minütigen Zentrifugation bei 13000rpm wurde durch Zugabe von 0,75ml Isopropanol zum Überstand die Plasmid-DNA bei Raumtemperatur gefällt. Die durch Zentrifugation für 30min bei 13000rpm pelletierte DNA wurde mit 70%igem Ethanol gewaschen, getrocknet und in 20µl Wasser resuspendiert. 1µl der DNA wurde für die Transformation in *E. coli* eingesetzt.

### 3.3 Bestimmung der DNA-Konzentration

Die DNA-Konzentration wurde spektralphotometrisch in einem Wellenlängenbereich von 240-300nm gemessen. Liegt die Reinheit der DNA, bestimmt durch den Quotient E₂₆₀ₙₘ/E₂₈₀ₙₘ, bei 1,8, so entspricht die Extinktion E₂₆₀ₙₘ=1,0 einer DNA-Konzentration von 50µg dsDNA/ml (Maniatis *et al.,* 1982).

### 3.4 DNA-Amplifikation mittels PCR

Verwendung des Phusion™ High Fidelity Systems

Die Polymerasekettenreaktion erfolgte in einem Gesamtvolumen von 50µl mit dem "Phusion™ High Fidelity PCR System" der Firma Finnzymes nach Angaben des Herstellers. Jeder Ansatz bestand aus 1-10ng DNA oder 1-2 Hefekolonien als Synthesevorlage, 0,2mM dNTP-Mix, 1xPuffer 2 (enthält 1,5mM MgCl₂), 1U Polymerase und je 100pmol der entsprechenden Oligonukleotidprimer. Die PCR-Reaktion wurde in einem Thermocycler der Firma Techne durchgeführt und die PCR-Bedingungen nach Bedarf wie folgt gewählt:

| | | |
|---|---|---|
| 1 1x | 30sec, 98°C | Denaturierung der DNA |
| 2 30x | 10sec, 98°C | Denaturierung der DNA |
| | 30sec, 56-62°C | Annealing/Bindung der Oligonukleotide an die DNA |
| | 0,5-1min, 72°C | DNA-Synthese/Elongation |
| 3 1x | 7min, 72°C | DNA-Synthese/Elongation |

Nach dem ersten Denaturierungsschritt wurde die Polymerase zugegeben ("hot start PCR"). Die Anzahl der Syntheseschritte, die Annealingtemperatur und die Elongationszeit wurden an die spezifischen Schmelztemperaturen der verwendeten Oligonukleotide bzw. an die Größe des zu erwarteten Produkts angepasst. Die PCR-Produkte wurden durch eine Agarosegelelektrophorese überprüft und anschließend aufgereinigt.

### 3.5 DNA-Aufreinigung von PCR-Produkten

Die Aufreinigung der PCR-Produkte erfolgte mit dem "QIAquick PCR Purification Kit" der Firma Qiagen nach Herstellerangaben.

### 3.6 Gelelektrophoretische Auftrennung von DNA-Fragmenten

Die Auftrennung von DNA-Fragmenten mit einer Größe von 0,15-20kb erfolgte in 0,5-1%igen Agarosegelen mit 0,5µg/ml Ethidiumbromid. Als Gel- und Laufpuffer wurde 1xTAE-Puffer (40mM Tris, 40mM Essigsäure, 2mM EDTA) verwendet (Maniatis et *al.,* 1982). Als Größenstandard diente eine mit den Restriktionsendonukleasen EcoRI und *Hind*III geschnittene Lambda-Phagen-DNA. Die DNA-Proben wurden vor dem Auftragen mit 1/10 Volumen Blaumarker (1xTAE-Puffer, 10% Glycerin, 0,004% Bromphenolblau) versetzt und nach der Auftrennung durch Bestrahlung mit UV-Licht (254nm) sichtbar gemacht.

### 3.7 Isolierung von DNA-Fragmenten aus Agarosegelen

Das gewünschte DNA-Fragment wurde aus dem TAE-Agarosegel unter langwelligem UV-Licht (366nm) ausgeschnitten und mit dem "QIAquick Gel Extraction Kit" der Firma Qiagen nach Angaben des Herstellers isoliert.

### 4. Enzymatische Modifikation von DNA

### 4.1 DNA-Restriktion

Sequenzspezifische Spaltung der DNA mit Restriktionsendonukleasen wurden unter den vom Hersteller empfohlenen Inkubationsbedingungen für 1 Stunde mit 2-5U Enzym pro µg DNA durchgeführt.

Weitere mögliche Expressionsvektoren sind aus der Serie pRS303X, p3RS305X und p3RS306X. Hierbei handelt es sich um integrative Vektoren, die einen dominanten Antibiotika-Marker besitzen. Nähere Angaben zu diesen Vektoren finden sich bei Taxis und Knop (2006).

### 5. Klonierung von DNA-Fragmenten durch in vivo-Rekombination

Für eine *in vivo*-Klonierung von DNA-Fragmenten in *S. cerevisiae* wurde zunächst das entsprechende Gen bzw. die DNA-Sequenz durch eine PCR-Reaktion synthetisiert. Die dabei eingesetzten Oligonukleotide enthalten jeweils im 5'-Bereich 36-39 Nukleotide umfassende spezifische Anhänge, die homolog zu den 5'- bzw. 3'-flankierenden Sequenzen des Integrationsbereiches im Zielvektor sind. Im 3'-Bereich enthalten die Oligonukleotide 20-22 Basen mit Homologie zu den 3'- bzw. 5'-Enden des zu amplifizierenden Gens. Das entstandene PCR-Produkt wurde zusammen mit dem durch Restriktion im Integrationsbereich linearisierten und aufgereinigten Vektor in Hefe transformiert. Die Zellen wurden auf synthetischem Selektivmedium ausplattiert, dem für die Selektion auf den Auxotrophiemarker des Vektors die entsprechende Aminosäure bzw. Nukleotidbase fehlte. Auf diese Weise wurden nur solche Transformanten erhalten, die aufgrund homologer Rekombination des DNA-Fragmentes in den linearisierten Vektor wieder ein stabiles, zirkuläres Plasmid gebildet hatten. Die Plasmide wurden isoliert, in *E. coli* amplifiziert und durch anschließende Restriktionsanalyse oder durch Sequenzierung überprüft.

### 6. Austausch von und Integration in genomische DNA

Dieses wurde wie in Becker und Boles (2003) und Wieczorke et al. (1999) beschrieben, durchgeführt.

### Literatur:

Atsumi S, Hanai T and Liao JC (2008) Non-fermentative pathway for synthesis of branched-chain higher alcohols as biofuels. Nature 451, 86-90.
Bailey JE (1993) Host-vector interactions in Escherichia coli. Adv. Biochem Eng. 48, 29-52
Becker J, Boles E (2003) A modified Saccharomyces cerevisiae strain that consumes L-Arabinose and produces ethanol. Appl Environ Microbiol. 69, 4144-4150.
Biou V, Dumas R, Cohen-Addad C, Douce R, Job D and Pebay-Peyroula E (1997) The crystal structure of plant acetohydroxy acid isomeroreductase complexed with NADPH, two magnesium ions and a herbicidal transition state analog determined at 1.65 A resolution. EMBO J. 16, 3405-3415.
Birnboim HC, Doly J (1979) A rapid alkaline extraction procedure for screening recombinant plasmid DNA. Nucl. Acids Res. 7, 1513-1523
Boles E, Lehnert W and Zimmermann FK (1993) The role of the NAD-dependent glutamate dehydrogenase in restoring growth on glucose of a Saccharomyces cerevisiae phosphoglucose isomerase mutant. Eur. J. Biochem. 217, 469-477.
Dickinson JR, Harrison SJ and Hewlins MJE (1998) An investigation of the metabolism of valine to isobutyl alcohol in Saccharomyces cerevisiae. J. Biol. Chem. 273, 25751-25756.
Dickinson JR, Salgado LEJ and Hewlins MJE (2003) The catabolism of amino acids to long chain and complex alcohols in Saccharomyces cerevisiae. J. Biol. Chem. 278, 8028-8034.
Dower WJ, Miller JF, Ragsdale CW (1988) High efficiency transformation of E. coli by high voltage electroporation. Nucl. Acids Res. 16, 6127-6145
Fillinger S, Boschi-Muller S, Azza S, Dervyn E, Branlant G and Aymerich S (2000) Two glyceraldehyde-3-phosphate dehydrogenases with opposite physiological roles in a nonphotosynthetic bacterium. J. Biol. Chem. 275, 14031-14037.
Gietz RD, Woods RA (1994) High efficiency transformation in yeast. In: Molecular Genetics of Yeast: Practical Approaches, J.A. Johnston (Ed.). Oxford University Press pp. 121-134
Maniatis T, Fritsch EF, Sambrook J (1982) Molecular cloning. A laboratory manual. Cold Spring Harbor Laboratory, New York.
Omura F (2008) Targeting of mitochondrial Saccharomyces cerevisiae Ilv5p to the cytosol and ist effect on vicinal diketone formation in brewing. Appl. Microbiol. Biotechnol. (DOI 10.1007/s00253-007-1333-x)
Pang SS and Duggleby RG (1999) Expression, purification, characterization, and reconstitution of the large and small subunits of yeast acetohydroxyacid synthase. Biochemistry 38, 5222-5231
Patentanmeldung US 2007/0092957 A1 (Fermentive production of four carbon alcohols)
Sauer U (2001) Evolutionary engineering of industrially important microbial phenotypes. Adv. Biochem. Eng. Biotechnol. 73, 129-169.
Taxis C, Knop M (2006) System of centromeric, episomal, and integrative vectors based on drug resistance markers for Saccharomyces cerevisiae. BioTechniques 40, No. 1
Verduyn C, Postma E, Scheffers WA, Van Dijken JP (1992) Effect of benzoic acid on metabolic fluxes in yeasts: a continuous-culture study on the regulation of respiration and alcoholic fermentation. Yeast 8, 501-17
Verho R, Richard P, Jonson PH, Sundqvist L, Londesbrorough J and Penttilä M (2002) Identification of the first fungal NADP-GAPDH from Kluyveromyces lactis. Biochemistry 41, 13833-13838.
Wieczorke R, Krampe S, Weierstall T, Freidel K, Hollenberg CP, Boles E (1999) Concurrent knock-out of at least 20 transporter genes is required to block uptake of hexoses in Saccharomyces cerevisiae. FEBS Lett 464, 123-128.
Wiedemann B und Boles E (2008), Codon-optimized bacterial genes improve L-arabinose fermentation in recombinant Saccharomyces cerevisiae. Appl. Environ. Microbiol. 74, 2043-2050.
Wirth R (1993) Elektroporation: Eine alternative Methode zur Transformation von Bakterien mit Plasmid-DNA. Forum Mikrobiologie 11, 507-515.
Zimmermann FK (1975) Procedures used in the induction of mitotic recombination and mutation in the yeast Saccharomyces cerevisiae. Mutation Res. 31, 71-81.

## Patentansprüche

1. Hefezelle, die Isobutanol produziert, **dadurch gekennzeichnet, dass** die Zelle einen gesteigerten metabolischen Stofffluss vom Pyruvat über Acetolactat, 2,3-Dihydroxyisovalerat, 2-Ketoisovalerat, Isobutyraldehyd zum Isobutanol aufweist, indem alle Gene, die für die Enzyme kodieren, die an dieser Umsetzung beteiligt sind, überexprimiert werden und dass diese Gene homolog zu der besagten Hefezelle sind, wobei Ilv2 mit SEQ.ID. Nr. 2 die Acetolactat Synthase Reaktion vom Pyruvat zum Acetolactat katalysiert, Ilv5 mit SEQ.ID. Nr. 6 die Acetohydroxysäure Reductoisomerase Reaktion vom Acetolactat zum 2,3-Dihydroxyisovalerat katalysiert, Ilv3 mit SEQ.ID. Nr. 8 die Dihydroxysäure Dehydratase Reaktion vom 2,3-Dihydroxyisovalerat zum 2-Ketoisovalerat katalysiert, eine 2-Ketosäure Decarboxylase die Reaktion vom 2-Ketoisovalerat zum Isobutyraldehyd katalysiert, und eine Alkoholdehydrogenase die Reaktion vom Isobutyraldehyd zum Isobutanol katalysiert, wobei entweder mindestens einer der Promotoren dieser Gene gegen mindestens einen stärkeren Promotor ausgetauscht wird oder die Nukleinsäuresequenzen dieser Gene in Codon-optimierte Allele abgewandelt werden,
**dadurch gekennzeichnet, dass** die Gene *ILV2, ILV5* und *ILV3* ohne die mitochondrielle Targetingsequenz der Enzyme Acetolactat Synthase, Acetohydroxysäure Reductoisomerase und Dihydroxysäure Dehydratase oder mit einer zerstörten, inaktivierten mitochondriellen Targetingsequenz überexprimiert werden,
und
**dadurch gekennzeichnet, dass** zusätzlich die Expression der Gene *PDC1* mit SEQ.ID. Nr. 13, *PDC5* mit SEQ.ID. Nr. 15 und *PDC6* mit SEQ.ID. Nr. 17 bzw. die Aktivität der kodierten Enzyme reduziert bzw. ausgeschaltet ist.

2. Hefezelle nach Anspruch 1, **dadurch gekennzeichnet, dass** die 2-Ketosäure Decarboxylase ausgesucht ist aus mindestens einem der Enzyme Aro10 mit SEQ.ID. Nr. 20 oder Thi3 mit SEQ.ID. Nr. 22.

3. Hefezelle nach einem der Ansprüche 1 oder 2, **dadurch gekennzeichnet, dass** die Alkoholdeyhdrogenase ausgewählt ist aus mindestens einem der Enzyme Adh1 mit SEQ.ID. Nr. 24, Adh2 mit SEQ.ID. Nr. 26, Adh3 mit SEQ.ID. Nr. 28, Adh4 mit SEQ.ID. Nr. 30, Adh5 mit SEQ.ID. Nr. 32, Adh6 mit SEQ.ID. Nr. 34, Adh7 mit SEQ.ID. Nr. 36, Sfa1 mit SEQ.ID. Nr. 38 oder Ypr1 mit SEQ.ID. Nr. 40.

4. Hefezelle nach einem der Ansprüche 1 bis 3, **dadurch gekennzeichnet, dass** alle diese Gene in codon-optimierten Varianten überexprimiert werden.

5. Hefezelle nach einem der Ansprüche 1 bis 4, **dadurch gekennzeichnet, dass** die Zelle eine Acetohydroxysäure Reductoisomerase exprimiert, die eine erhöhte Spezifität für NADH gegenüber NADPH aufweist.

6. Hefezelle nach einem der Ansprüche 1 bis 4, **dadurch gekennzeichnet, dass** die Zelle zusätzlich eine phosphorylierende Glycerinaldehyd-3-Phosphat Dehydrogenase exprimiert, die eine erhöhte Spezifität für NADP⁺ gegenüber NAD⁺ aufweist.

7. Hefezelle nach Anspruch 6, **dadurch gekennzeichnet, dass** diese NADP-Glycerinaldehyd-3-Phosphat Dehydrogenase in einer Hefezelle exprimiert wird, die keine oder eine reduzierte Expression oder Aktivität der NAD-Glycerinaldehyd-3-Phosphat Dehydrogenasen aufweist.

8. Hefezelle nach einem der Ansprüche 1 bis 7, **dadurch gekennzeichnet, dass** zusätzlich das Ilv6-Protein mit SEQ.ID. Nr. 4 im selben Zellkompartiment wie Ilv2 überexprimiert wird.

9. Hefezelle nach einem der Ansprüche 1 bis 8, **dadurch gekennzeichnet, dass** die Zelle ausgewählt ist aus folgender Gruppe: *Pichia, Candida, Hansenula, Kluyveromyces, Yarrowia* und *Saccharomyces.*

10. Hefezelle nach Anspruch 9, **dadurch gekennzeichnet, dass** die Wirtszelle *Saccharomyces cerevisiae* ist.

11. Verfahren für die Produktion von Isobutanol mit Hefezellen, umfassend die Bereitstellung einer Hefezelle nach einem der Ansprüche 1 bis 10 sowie das Inkontaktbringen der Hefezelle mit einer fermentierbaren Kohlenstoffquelle.

## Claims

1. Yeast cell producing isobutanol, **characterized in that** the cell has an increased metabolic flow of material from pyruvate via acetolactate, 2,3-dihydroxy isovalerate, 2-ketoisovalerate, isobutyraldehyde to isobutanol, **in that** all of the genes which code for the enzymes which are involved in this conversion are over-expressed and with all of these genes being homologous to the said yeast cell, wherein Ilv2 with SEQ ID No. 2 catalyzes the acetolactate synthase reaction from pyruvate to acetolactate, Ilv5 with SEQ ID No. 6 catalyzes the acetohydroxy acid reducto-isomerase reaction from acetolactate to 2,3-dihydroxy isovalerate, Ilv3 with SEQ ID No. 8 catalyzes the dihydroxy acid dehydratase reaction from 2,3-dihydroxy isovalerate to 2-ketoisovalerate, a 2-keto acid decarboxylase catalyzes the reaction from 2-ketoisovalerate to isobutyraldehyde, and an alcohol dehydrogenase catalyzes the reaction from isobutyraldehyde to isobutanol, wherein either at least one of the promoters of these genes is exchanged for at least one stronger promoter or the nucleic acid sequences of these genes are converted into codon-optimized alleles,
**characterized in that** the genes *ILV2, ILV5* and *ILV3* are overexpressed without the mitochondrial targeting sequence of the enzymes acetolactate synthetase, acetohydroxy acid reducto-isomerase and dihydroxy acid dehydratase or with a destroyed, inactivated mitochondrial targeting sequence, and
**characterized in that** in addition the expression of the genes *PDC1* with SEQ ID No. 13, *PDC5* with SEQ ID No. 15 and *PDC6* with SEQ ID No. 17 or, respectively, the activity of the encoded enzymes is reduced or, respectively, switched off.

2. Yeast cell according to claim 1, **characterized in that** the 2-keto acid decarboxylase is selected from at least one of the enzymes Aro10 with SEQ ID No. 20 or Thi3 with SEQ ID No. 22.

3. Yeast cell according to one of claims 1 or 2, **characterized in that** the alcohol deyhdrogenase is selected from at least one of the enzymes Adh1 with SEQ ID No. 24, Adh2 with SEQ ID No. 26, Adh3 with SEQ ID No. 28, Adh4 with SEQ ID No. 30, Adh5 with SEQ ID No. 32, Adh6 with SEQ ID No. 34, Adh7 with SEQ ID No. 36, Sfa1 with SEQ ID No. 38 or Ypr1 with SEQ ID No. 40.

4. Yeast cell according to one of claims 1 to 3, **characterized in that** all of the over-expressed genes are over-expressed in codon-optimized variants.

5. Yeast cell according to one of claims 1 to 4, **characterized in that** the cell expresses an acetohydroxy acid reducto-isomerase which has an increased specificity for NADH compared with NADPH.

6. Yeast cell according to one of claims 1 to 4, **characterized in that** the cell also expresses a phosphorylative glyceraldehyde-3-phosphate dehydrogenase which has an increased specificity for NADP⁺ compared with NAD⁺.

7. Yeast cell according to claim 6, **characterized in that** this NADP-glyceraldehyde-3-phosphate dehydrogenase is expressed in a yeast cell which displays no or a reduced expression or activity of the NAD-glyceraldehyde-3-phosphate dehydrogenases.

8. Yeast cell according to one of claims 1 to 7, **characterized in that** in addition the Ilv6 protein with SEQ ID No. 4 is over-expressed in the same cell compartment as Ilv2.

9. Yeast cell according to one of claims 1 to 8, **characterized in that** the cell is selected from the following group: *Pichia, Candida, Hansenula, Kluyveromyces, Yarrowia* and *Saccharomyces.*

10. Yeast cell according to claim 9, **characterized in that** the host cell is *Saccharomyces cerevisiae.*

11. Method for the production of isobutanol with yeast cells, comprising the provision of a yeast cell according to one of claims 1 to 10 as well as bringing the yeast cell into contact with a fermentable carbon source.

## Revendications

1. Cellule de levure qui produit de l'isobutanol, **caractérisée en ce que** la cellule présente un flux des matières métabolique accru du pyruvate par le biais de l'acétolactate, du 2,3-dihydroxyisovalérate, du 2-cétoisovalérate, de l'isobutyraldéhyde en isobutanol en ce que tous les gènes qui codent pour les enzymes qui sont impliquées dans cette transformation sont surexprimés et que ces gènes sont homologues à ladite cellule de levure, dans laquelle Ilv2 avec SEQ.ID. N° 2 catalyse la réaction d'acétolactate synthase du pyruvate en acétolactate, Ilv5 avec SEQ.ID. N° 6 catalyse la réaction d'acide acétohydroxylique réductoisomérase de l'acétolactate en 2,3-dihydroxyisovalérate, Ilv3 avec SEQ.ID. N° 8 catalyse la réaction d'acide dihydroxylique déshydratase du 2,3-dihydroxyisovalérate en 2-cétoisovalérate, une acide 2-cétonique décarboxylase catalyse la réaction du 2-cétoisovalérate en isobutyraldéhyde et une alcool déshydrogénase catalyse la réaction de l'isobutyraldéhyde en isobutanol, dans laquelle soit au moins un des promoteurs de ces gènes est échangé contre au moins un promoteur plus fort, soit les séquences d'acide nucléique de ces gènes sont modifiées en allèles optimisés au niveau du codon, **caractérisée en ce que** les gènes ILV2, ILV5 et ILV3 sont surexprimés sans la séquence de ciblage mitochondriale des enzymes acétolactate synthase, acide acétohydroxylique réductoisomérase et acide dihydroxylique déshydratase ou avec une séquence de cible mitochondriale détruite, inactivée,
et
**caractérisée en ce que** l'expression des gènes PDC1 avec SEQ.ID. N° 13, PDC5 avec SEQ.ID. N° 15 et PDC6 avec SEQ.ID. N° 17 ou l'activité des enzymes codées est en outre réduite ou désactivée.

2. Cellule de levure selon la revendication 1, **caractérisée en ce que** l'acide 2-cétonique décarboxylase est sélectionné parmi au moins une des enzymes Aro10 avec SEQ.ID. N° 20 ou Thi3 avec SEQ.ID. N° 22.

3. Cellule de levure selon une des revendications 1 ou 2, **caractérisée en ce que** l'alcool déshydrogénase est sélectionnée parmi au moins une des enzymes Adh1 avec SEQ.ID. N° 24, Adh2 avec SEQ.ID. N° 26, Adh3 avec SEQ.ID. N° 28, Adh4 avec SEQ.ID. N° 30, Adh5 avec SEQ.ID. N° 32, Adh6 avec SEQ.ID. N° 34, Adh7 avec SEQ.ID. N° 36, Sfa1 avec SEQ.ID. N° 38 ou Ypr1 avec SEQ.ID. N° 40.

4. Cellule de levure selon une des revendications 1 à 3, **caractérisée en ce que** tous ces gènes sont surexprimés dans des variantes optimisées au niveau du codon.

5. Cellule de levure selon une des revendications 1 à 4, **caractérisée en ce que** la cellule exprime une acide acétohydroxylique réductoisomérase qui présente une spécificité accrue pour NADH par rapport à NADPH.

6. Cellule de levure selon une des revendications 1 à 4, **caractérisée en ce que** la cellule exprime en outre une glycérinaldéhyde-3-phosphate déshydrogénase phosphorylée qui présente une spécificité accrue pour NADP⁺ par rapport à NAD⁺.

7. Cellule de levure selon la revendication 6, **caractérisée en ce que** cette NADP-glycérinaldéhyde-3-phosphate déshydrogénase est exprimée dans une cellule de levure qui ne présente aucune expression ou activité ou présente une expression ou activité réduite des NAD-glycérinaldéhyde-3-phosphate déshydrogénases.

8. Cellule de levure selon une des revendications 1 à 7, **caractérisée en ce que** la protéine Ilv6 avec SEQ.ID. N° 4 est en outre surexprimée dans le même compartiment cellulaire que Ilv2.

9. Cellule de levure selon une des revendications 1 à 8, **caractérisée en ce que** la cellule est sélectionnée parmi le groupe suivant : Pichia, Candida, Hansenula, Kluyveromyces, Yarrowia et Saccharomyces.

10. Cellule de levure selon la revendication 9, **caractérisée en ce que** la cellule hôte est Saccharomyces cerevisiae.

11. Procédé pour la production d'isobutanol avec des cellules de levure comprenant la mise à disposition d'une cellule de levure selon une des revendications 1 à 10 ainsi que la mise en contact de la cellule de levure avec une source de carbone pouvant être fermentée.


### SEQUENCE LISTING

<110> BUTALCO GmbH
<120> Fermentative Produktion von Isobutanol mit Hefe
<130> 33847-BUT-P-WO
<150> 10 2008 010 121.4
   <151> 20.02.2008
<160> 50
<210> 1
   <211> 2064
   <212> DNA
   <213> Saccharomyces Cerevisiae
<400> 1
   atgatcagac aatctacgct aaaaaacttc gctattaagc gttgctttca acatatagca      60
   taccgcaaca cacctgccat gagatcagta gctctcgcgc agcgctttta tagttcgtct     120
   tcccgttatt acagtgcgtc tccattacca gcctctaaaa ggccagagcc tgctccaagt     180
   ttcaatgttg atccattaga acagcccgct gaaccttcaa aattggctaa gaaactacgc     240
   gctgagcctg acatggatac ctctttcgtc ggtttaactg gtggtcaaat atttaacgaa     300
   atgatgtcca gacaaaacgt tgatactgta tttggttatc caggtggtgc tatcctacct     360
   gtttacgatg ccattcataa cagtgataaa ttcaacttcg ttcttccaaa acacgaacaa     420
   ggtgccggtc acatggcaga aggctacgcc agagcttctg gtaaaccagg tgttgtcttg     480
   gttacttctg ggccaggtgc caccaatgtc gttactccaa tggcagatgc ctttgcagac     540
   gggattccaa tggttgtctt tacagggcaa gtcccaacta gtgctatcgg tactgatgct     600
   ttccaagagg ctgacgtcgt tggtatttct agatcttgta cgaaatggaa tgtcatggtc     660
   aagtccgtgg aagaattgcc attgcgtatt aacgaggctt ttgaaattgc cacgagcggt     720
   agaccgggac cagtcttggt cgatttacca aaggatgtta cagcagctat cttaagaaat     780
   ccaattccaa caaaaacaac tcttccatca aacgcactaa accaattaac cagtcgcgca     840
   caagatgaat ttgtcatgca aagtatcaat aaagcagcag atttgatcaa cttggcaaag     900
   aaacctgtct tatacgtcgg tgctggtatt ttaaaccatg cagatggtcc aagattacta     960
   aaagaattaa gtgaccgtgc tcaaatacct gtcaccacta ctttacaagg tttaggttca    1020
   ttcgaccaag aagatccaaa atcattggat atgcttggta tgcacggttg tgctactgcc    1080
   aacctggcag tgcaaaatgc cgacttgata attgcagttg gtgctagatt cgacgaccgt    1140
   gtcactggta atatttctaa attcgctcca gaagctcgtc gtgcagctgc cgagggtaga    1200
   ggtggtatta ttcatttcga ggttagtcca aaaaacataa acaaggttgt tcaaactcaa    1260
   atagcagtgg aaggtgatgc tacgaccaat ctgggcaaaa tgatgtcaaa gattttccca    1320
   gttaaggaga ggtctgaatg gtttgctcaa ataaataaat ggaagaagga atacccatac    1380
   gcttatatgg aggagactcc aggatctaaa attaaaccac agacggttat aaagaaacta      1440
   tccaaggttg ccaacgacac aggaagacat gtcattgtta caacgggtgt ggggcaacat      1500
   caaatgtggg ctgctcaaca ctggacatgg agaaatccac atactttcat cacatcaggt      1560
   ggtttaggta cgatgggtta cggtctccct gccgccatcg gtgctcaagt tgcaaagcca      1620
   gaatctttgg ttattgacat tgatggtgac gcatccttta acatgactct aacggaattg      1680
   agttctgccg ttcaagctgg tactccagtg aagattttga ttttgaacaa tgaagagcaa      1740
   ggtatggtta ctcaatggca atccctgttc tacgaacatc gttattccca cacacatcaa      1800
   ttgaaccctg atttcataaa actagcggag gctatgggtt taaaaggttt aagagtcaag      1860
   aagcaagagg aattggacgc taagttgaaa gaattcgttt ctaccaaggg cccagttttg      1920
   cttgaagtgg aagttgataa aaaagttcct gttttgccaa tggtggcagg tggtagcggt      1980
   ctagacgagt tcataaattt tgacccagaa gttgaaagac aacagactga attacgtcat      2040
   aagcgtacag gcggtaagca ctga                                              2064
<210> 2
   <211> 687
   <212> PRT
   <213> Saccharomyces Cerevisiae
<400> 2
<210> 3
   <211> 930
   <212> DNA
   <213> Saccharomyces cerevisiae
<400> 3
   atgctgagat cgttattgca aagcggccac cgcagggtgg ttgcttcttc atgtgctacc      60
   atggtgcgtt gcagttcctc gtcgacctcc gcgttggcgt acaagcagat gcacagacac     120
   gcaacaagac ctcccttgcc cacactagac actccttcct ggaatgccaa cagtgccgtt     180
   tcatccatca tttacgaaac accagcgcct tctcgtcaac caagaaaaca gcatgtcttg     240
   aactgtttgg tgcaaaacga acccggtgtc ttgtccagag tctcgggtac gttagctgcc     300
   agaggcttta acatcgattc gttggtcgtg tgcaacaccg aggtcaaaga cctaagtaga     360
   atgaccattg ttttgcaagg gcaagatggc gtagtcgaac aagcacgcag acaaatcgaa     420
   gacttggtcc ccgtctacgc cgtcctagac tataccaatt ctgagatcat caaaagagag     480
   ctagtgatgg ccagaatctc tctattgggt actgaatact tcgaagacct actattgcac     540
   caccacactt ccaccaatgc tggcgccgct gactcccaag aattggtcgc cgaaatcaga     600
   gaaaagcaat tccaccctgc caacttgccc gccagtgagg tattaaggtt gaagcacgag     660
   catttgaacg atatcaccaa cttgaccaac aactttggag gtcgtgtcgt cgacatcagc     720
   gaaacaagct gtattgtgga gttgtctgca aaacccacac gtatctctgc cttcttgaag     780
   ttggtcgagc cattcggtgt cctagagtgt gcaagaagcg gtatgatggc attgccaaga     840
   actcctttga agacaagcac cgaggaagct gccgacgaag acgaaaagat cagcgaaatc     900
   gtcgacattt cccaactacc acctggttag                                     930
<210> 4
   <211> 309
   <212> PRT
   <213> Saccharomyces cerevisiae
<400> 4
<210> 5
   <211> 1188
   <212> DNA
   <213> Saccharomyces cerevisiae
<400> 5
   atgttgagaa ctcaagccgc cagattgatc tgcaactccc gtgtcatcac tgctaagaga 60
   acctttgctt tggccacccg tgctgctgct tacagcagac cagctgcccg tttcgttaag 120
   ccaatgatca ctacccgtgg tttgaagcaa atcaacttcg gtggtactgt tgaaaccgtc 180
   tacgaaagag ctgactggcc aagagaaaag ttgttggact acttcaagaa cgacactttt 240
   gctttgatcg gttacggttc ccaaggttac ggtcaaggtt tgaacttgag agacaacggt 300
   ttgaacgtta tcattggtgt ccgtaaagat ggtgcttctt ggaaggctgc catcgaagac 360
   ggttgggttc caggcaagaa cttgttcact gttgaagatg ctatcaagag aggtagttac 420
   gttatgaact tgttgtccga tgccgctcaa tcagaaacct ggcctgctat caagccattg 480
   ttgaccaagg gtaagacttt gtacttctcc cacggtttct ccccagtctt caaggacttg 540
   actcacgttg aaccaccaaa ggacttagat gttatcttgg ttgctccaaa gggttccggt 600
   agaactgtca gatctttgtt caaggaaggt cgtggtatta actcttctta cgccgtctgg 660
   aacgatgtca ccggtaaggc tcacgaaaag gcccaagctt tggccgttgc cattggttcc 720
   ggttacgttt accaaaccac tttcgaaaga gaagtcaact ctgacttgta cggtgaaaga 780
   ggttgtttaa tgggtggtat ccacggtatg ttcttggctc aatacgacgt cttgagagaa 840
   aacggtcact ccccatctga agctttcaac gaaaccgtcg aagaagctac ccaatctcta 900
   tacccattga tcggtaagta cggtatggat tacatgtacg atgcttgttc caccaccgcc 960
   agaagaggtg ctttggactg gtacccaatc ttcaagaatg ctttgaagcc tgttttccaa 1020
   gacttgtacg aatctaccaa gaacggtacc gaaaccaaga gatctttgga attcaactct 1080
   caacctgact acagagaaaa gctagaaaag gaattagaca ccatcagaaa catggaaatc 1140
   tggaaggttg gtaaggaagt cagaaagttg agaccagaaa accaataa 1188
<210> 6
   <211> 395
   <212> PRT
   <213> Saccharomyces cerevisiae
<400> 6
   Met Leu Arg Thr Gln Ala Ala Arg Leu Ile Cys Asn Ser Arg Val Ile
   1               5                   10                  15
   Thr Ala Lys Arg Thr Phe Ala Leu Ala Thr Arg Ala Ala Ala Tyr Ser
               20                  25                  30
   Arg Pro Ala Ala Arg Phe Val Lys Pro Met Ile Thr Thr Arg Gly Leu
           35                  40                  45
   Lys Gln Ile Asn Phe Gly Gly Thr Val Glu Thr Val Tyr Glu Arg Ala
       50                  55                  60
   Asp Trp Pro Arg Glu Lys Leu Leu Asp Tyr Phe Lys Asn Asp Thr Phe
   65                  70                  75                  80
   Ala Leu Ile Gly Tyr Gly Ser Gln Gly Tyr Gly Gln Gly Leu Asn Leu
                   85                  90                  95
   Arg Asp Asn Gly Leu Asn Val Ile Ile Gly Val Arg Lys Asp Gly Ala
               100                 105                 110
   Ser Trp Lys Ala Ala Ile Glu Asp Gly Trp Val Pro Gly Lys Asn Leu
           115                 120                 125
   Phe Thr Val Glu Asp Ala Ile Lys Arg Gly Ser Tyr Val Met Asn Leu
       130                 135                 140
   Leu Ser Asp Ala Ala Gln Ser Glu Thr Trp Pro Ala Ile Lys Pro Leu
   145                 150                 155                 160
   Leu Thr Lys Gly Lys Thr Leu Tyr Phe Ser His Gly Phe Ser Pro Val
                   165                 170                 175
   Phe Lys Asp Leu Thr His Val Glu Pro Pro Lys Asp Leu Asp Val Ile
               180                 185                 190
   Leu Val Ala Pro Lys Gly Ser Gly Arg Thr Val Arg Ser Leu Phe Lys
           195                 200                 205
   Glu Gly Arg Gly Ile Asn Ser Ser Tyr Ala Val Trp Asn Asp Val Thr
       210                 215                 220
   Gly Lys Ala His Glu Lys Ala Gln Ala Leu Ala Val Ala Ile Gly Ser
   225                 230                 235                 240
   Gly Tyr Val Tyr Gln Thr Thr Phe Glu Arg Glu Val Asn Ser Asp Leu
                   245                 250                 255
   Tyr Gly Glu Arg Gly Cys Leu Met Gly Gly Ile His Gly Met Phe Leu
               260                 265                 270
   Ala Gln Tyr Asp Val Leu Arg Glu Asn Gly His Ser Pro Ser Glu Ala
           275                 280                 285
   Phe Asn Glu Thr Val Glu Glu Ala Thr Gln Ser Leu Tyr Pro Leu Ile
       290                 295                 300
   Gly Lys Tyr Gly Met Asp Tyr Met Tyr Asp Ala Cys Ser Thr Thr Ala
   305                 310                 315                 320
   Arg Arg Gly Ala Leu Asp Trp Tyr Pro Ile Phe Lys Asn Ala Leu Lys
                   325                 330                 335
   Pro Val Phe Gln Asp Leu Tyr Glu Ser Thr Lys Asn Gly Thr Glu Thr
               340                 345                 350
   Lys Arg Ser Leu Glu Phe Asn Ser Gln Pro Asp Tyr Arg Glu Lys Leu
           355                 360                 365
   Glu Lys Glu Leu Asp Thr Ile Arg Asn Met Glu Ile Trp Lys Val Gly
       370                 375                 380
   Lys Glu Val Arg Lys Leu Arg Pro Glu Asn Gln
   385                 390                 395
<210> 7
   <211> 1758
   <212> DNA
   <213> Saccharomyces cerevisiae
<400> 7
   atgggcttgt taacgaaagt tgctacatct agacaattct ctacaacgag atgcgttgca      60
   aagaagctca acaagtactc gtatatcatc actgaaccta agggccaagg tgcgtcccag     120
   gccatgcttt atgccaccgg tttcaagaag gaagatttca agaagcctca agtcggggtt     180
   ggttcctgtt ggtggtccgg taacccatgt aacatgcatc tattggactt gaataacaga     240
   tgttctcaat ccattgaaaa agcgggtttg aaagctatgc agttcaacac catcggtgtt     300
   tcagacggta tctctatggg tactaaaggt atgagatact cgttacaaag tagagaaatc     360
   attgcagact cctttgaaac catcatgatg gcacaacact acgatgctaa catcgccatc     420
   ccatcatgtg acaaaaacat gcccggtgtc atgatggcca tgggtagaca taacagacct     480
   tccatcatgg tatatggtgg tactatcttg cccggtcatc caacatgtgg ttcttcgaag     540
   atctctaaaa acatcgatat cgtctctgcg ttccaatcct acggtgaata tatttccaag     600
   caattcactg aagaagaaag agaagatgtt gtggaacatg catgcccagg tcctggttct     660
   tgtggtggta tgtatactgc caacacaatg gcttctgccg ctgaagtgct aggtttgacc     720
   attccaaact cctcttcctt cccagccgtt tccaaggaga agttagctga gtgtgacaac     780
   attggtgaat acatcaagaa gacaatggaa ttgggtattt tacctcgtga tatcctcaca     840
   aaagaggctt ttgaaaacgc cattacttat gtcgttgcaa ccggtgggtc cactaatgct     900
   gttttgcatt tggtggctgt tgctcactct gcgggtgtca agttgtcacc agatgatttc     960
   caaagaatca gtgatactac accattgatc ggtgacttca aaccttctgg taaatacgtc    1020
   atggccgatt tgattaacgt tggtggtacc caatctgtga ttaagtatct atatgaaaac    1080
   aacatgttgc acggtaacac aatgactgtt accggtgaca ctttggcaga acgtgcaaag    1140
   aaagcaccaa gcctacctga aggacaagag attattaagc cactctccca cccaatcaag    1200
   gccaacggtc acttgcaaat tctgtacggt tcattggcac caggtggagc tgtgggtaaa    1260
   attaccggta aggaaggtac ttacttcaag ggtagagcac gtgtgttcga agaggaaggt    1320
   gcctttattg aagccttgga aagaggtgaa atcaagaagg gtgaaaaaac cgttgttgtt    1380
   atcagatatg aaggtccaag aggtgcacca ggtatgcctg aaatgctaaa gccttcctct    1440
   gctctgatgg gttacggttt gggtaaagat gttgcattgt tgactgatgg tagattctct    1500
   ggtggttctc acgggttctt aatcggccac attgttcccg aagccgctga aggtggtcct    1560
   atcgggttgg tcagagacgg cgatgagatt atcattgatg ctgataataa caagattgac    1620
   ctattagtct ctgataagga aatggctcaa cgtaaacaaa gttgggttgc acctccacct 1680
   cgttacacaa gaggtactct atccaagtat gctaagttgg tttccaacgc ttccaacggt 1740
   tgtgttttag atgcttga 1758
<210> 8
   <211> 585
   <212> PRT
   <213> Saccharomyces cerevisiae
<400> 8
   Met Gly Leu Leu Thr Lys Val Ala Thr Ser Arg Gln Phe Ser Thr Thr
   1               5                   10                  15
   Arg Cys Val Ala Lys Lys Leu Asn Lys Tyr Ser Tyr Ile Ile Thr Glu
               20                  25                  30
   Pro Lys Gly Gln Gly Ala Ser Gln Ala Met Leu Tyr Ala Thr Gly Phe
           35                  40                  45
   Lys Lys Glu Asp Phe Lys Lys Pro Gln Val Gly Val Gly Ser Cys Trp
       50                  55                  60
   Trp Ser Gly Asn Pro Cys Asn Met His Leu Leu Asp Leu Asn Asn Arg
   65                  70                  75                  80
   Cys Ser Gln Ser Ile Glu Lys Ala Gly Leu Lys Ala Met Gln Phe Asn
                   85                  90                  95
   Thr Ile Gly Val Ser Asp Gly Ile Ser Met Gly Thr Lys Gly Met Arg
               100                 105                 110
   Tyr Ser Leu Gln Ser Arg Glu Ile Ile Ala Asp Ser Phe Glu Thr Ile
           115                 120                 125
   Met Met Ala Gln His Tyr Asp Ala Asn Ile Ala Ile Pro Ser Cys Asp
       130                 135                 140
   Lys Asn Met Pro Gly Val Met Met Ala Met Gly Arg His Asn Arg Pro
   145                 150                 155                 160
   Ser Ile Met Val Tyr Gly Gly Thr Ile Leu Pro Gly His Pro Thr Cys
                   165                 170                 175
   Gly Ser Ser Lys Ile Ser Lys Asn Ile Asp Ile Val Ser Ala Phe Gln
               180                 185                 190
   Ser Tyr Gly Glu Tyr Ile Ser Lys Gln Phe Thr Glu Glu Glu Arg Glu
           195                 200                 205
   Asp Val Val Glu His Ala Cys Pro Gly Pro Gly Ser Cys Gly Gly Met
   210 215 220
   Tyr Thr Ala Asn Thr Met Ala Ser Ala Ala Glu Val Leu Gly Leu Thr
   225 230 235 240
   Ile Pro Asn Ser Ser Ser Phe Pro Ala Val Ser Lys Glu Lys Leu Ala
   245 250 255
   Glu Cys Asp Asn Ile Gly Glu Tyr Ile Lys Lys Thr Met Glu Leu Gly
   260 265 270
   Ile Leu Pro Arg Asp Ile Leu Thr Lys Glu Ala Phe Glu Asn Ala Ile
   275 280 285
   Thr Tyr Val Val Ala Thr Gly Gly Ser Thr Asn Ala Val Leu His Leu
   290 295 300
   Val Ala Val Ala His Ser Ala Gly Val Lys Leu Ser Pro Asp Asp Phe
   305 310 315 320
   Gln Arg Ile Ser Asp Thr Thr Pro Leu Ile Gly Asp Phe Lys Pro Ser
   325 330 335
   Gly Lys Tyr Val Met Ala Asp Leu Ile Asn Val Gly Gly Thr Gln Ser
   340 345 350
   Val Ile Lys Tyr Leu Tyr Glu Asn Asn Met Leu His Gly Asn Thr Met
   355 360 365
   Thr Val Thr Gly Asp Thr Leu Ala Glu Arg Ala Lys Lys Ala Pro Ser
   370 375 380
   Leu Pro Glu Gly Gln Glu Ile Ile Lys Pro Leu Ser His Pro Ile Lys
   385 390 395 400
   Ala Asn Gly His Leu Gln Ile Leu Tyr Gly Ser Leu Ala Pro Gly Gly
   405 410 415
   Ala Val Gly Lys Ile Thr Gly Lys Glu Gly Thr Tyr Phe Lys Gly Arg
   420 425 430
   Ala Arg Val Phe Glu Glu Glu Gly Ala Phe Ile Glu Ala Leu Glu Arg
   435 440 445
   Gly Glu Ile Lys Lys Gly Glu Lys Thr Val Val Val Ile Arg Tyr Glu
   450 455 460
   Gly Pro Arg Gly Ala Pro Gly Met Pro Glu Met Leu Lys Pro Ser Ser
   465 470 475 480
   Ala Leu Met Gly Tyr Gly Leu Gly Lys Asp Val Ala Leu Leu Thr Asp
   485 490 495
   Gly Arg Phe Ser Gly Gly Ser His Gly Phe Leu Ile Gly His Ile Val
   500 505 510
   Pro Glu Ala Ala Glu Gly Gly Pro Ile Gly Leu Val Arg Asp Gly Asp
   515 520 525
   Glu Ile Ile Ile Asp Ala Asp Asn Asn Lys Ile Asp Leu Leu Val Ser
   530 535 540
   Asp Lys Glu Met Ala Gln Arg Lys Gln Ser Trp Val Ala Pro Pro Pro
   545 550 555 560
   Arg Tyr Thr Arg Gly Thr Leu Ser Lys Tyr Ala Lys Leu Val Ser Asn
   565 570 575
   Ala Ser Asn Gly Cys Val Leu Asp Ala
   580 585
<210> 9
   <211> 1182
   <212> DNA
   <213> Saccharomyces cerevisiae
<400> 9
   atgttgcaga gacattcctt gaagttgggg aaattctcca tcagaacact cgctactggt 60
   gccccattag atgcatccaa actaaaaatt actagaaacc caaatccatc caagccaaga 120
   ccaaatgaag aattagtgtt cggccagaca ttcaccgatc atatgttgac cattccttgg 180
   tcagccaaag aagggtgggg cactccacac atcaagcctt acggtaatct ttctcttgac 240
   ccatctgctt gtgtattcca ttatgcattt gaattatttg aaggtttgaa agcctacaga 300
   actcctcaaa atactatcac catgttccgt ccggataaga acatggcccg tatgaacaag 360
   tctgccgcta gaatttgttt gccaactttc gaatctgaag aattgatcaa acttaccggg 420
   aaattgatcg aacaagataa acacttggtt cctcaaggta atggttactc attatacatc 480
   agaccaacaa tgattggtac atccaagggt ttaggtgttg gcactccctc cgaggctctt 540
   ctttatgtta ttacttctcc agtcggtcct tattataaga ctggtttcaa agccgtacgt 600
   cttgaagcaa cagactatgc tacaagagct tggccaggtg gtgttggcga caaaaaattg 660
   ggtgctaact atgccccatg catcttacct caactacaag ctgccaaaag agggtaccaa 720
   caaaatctat ggttgttcgg cccagaaaag aacatcactg aggttggtac tatgaacgtg 780
   ttcttcgttt tcctcaacaa agtcactggc aagaaggaat tggttaccgc tccattagat 840
   ggtaccattt tagaaggtgt taccagagac tctgttttaa cattggctcg tgacaaacta 900
   gatcctcaag aatgggacat caacgagcgt tattacacta ttactgaagt cgccactaga 960
   gcaaaacaag gtgaactatt agaagccttc ggttctggta ctgctgctgt cgtttcacct 1020
   atcaaggaaa ttggctggaa caacgaagat attcatgttc cactattgcc tggtgaacaa 1080
   tgtggtgcat tgaccaagca agttgctcaa tggattgctg atatccaata cggtagagtc 1140
   aattatggta actggtcaaa aactgttgcc gacttgaact aa 1182
<210> 10
   <211> 393
   <212> PRT
   <213> Saccharomyces cerevisiae
<400> 10
   Met Leu Gln Arg His Ser Leu Lys Leu Gly Lys Phe Ser Ile Arg Thr
   1               5                   10                  15
   Leu Ala Thr Gly Ala Pro Leu Asp Ala Ser Lys Leu Lys Ile Thr Arg
               20                  25                  30
   Asn Pro Asn Pro Ser Lys Pro Arg Pro Asn Glu Glu Leu Val Phe Gly
           35                  40                  45
   Gln Thr Phe Thr Asp His Met Leu Thr Ile Pro Trp Ser Ala Lys Glu
       50                  55                  60
   Gly Trp Gly Thr Pro His Ile Lys Pro Tyr Gly Asn Leu Ser Leu Asp
   65                  70                  75                  80
   Pro Ser Ala Cys Val Phe His Tyr Ala Phe Glu Leu Phe Glu Gly Leu
                   85                  90                  95
   Lys Ala Tyr Arg Thr Pro Gln Asn Thr Ile Thr Met Phe Arg Pro Asp
               100                 105                 110
   Lys Asn Met Ala Arg Met Asn Lys Ser Ala Ala Arg Ile Cys Leu Pro
           115                 120                 125
   Thr Phe Glu Ser Glu Glu Leu Ile Lys Leu Thr Gly Lys Leu Ile Glu
       130                 135                 140
   Gln Asp Lys His Leu Val Pro Gln Gly Asn Gly Tyr Ser Leu Tyr Ile
   145                 150                 155                 160
   Arg Pro Thr Met Ile Gly Thr Ser Lys Gly Leu Gly Val Gly Thr Pro
                   165                 170                 175
   Ser Glu Ala Leu Leu Tyr Val Ile Thr Ser Pro Val Gly Pro Tyr Tyr
               180                 185                 190
   Lys Thr Gly Phe Lys Ala Val Arg Leu Glu Ala Thr Asp Tyr Ala Thr
           195                 200                 205
   Arg Ala Trp Pro Gly Gly Val Gly Asp Lys Lys Leu Gly Ala Asn Tyr
       210                 215                 220
   Ala Pro Cys Ile Leu Pro Gln Leu Gln Ala Ala Lys Arg Gly Tyr Gln
   225 230 235 240
   Gln Asn Leu Trp Leu Phe Gly Pro Glu Lys Asn Ile Thr Glu Val Gly
   245 250 255
   Thr Met Asn Val Phe Phe Val Phe Leu Asn Lys Val Thr Gly Lys Lys
   260 265 270
   Glu Leu Val Thr Ala Pro Leu Asp Gly Thr Ile Leu Glu Gly Val Thr
   275 280 285
   Arg Asp Ser Val Leu Thr Leu Ala Arg Asp Lys Leu Asp Pro Gln Glu
   290 295 300
   Trp Asp Ile Asn Glu Arg Tyr Tyr Thr Ile Thr Glu Val Ala Thr Arg
   305 310 315 320
   Ala Lys Gln Gly Glu Leu Leu Glu Ala Phe Gly Ser Gly Thr Ala Ala
   325 330 335
   Val Val Ser Pro Ile Lys Glu Ile Gly Trp Asn Asn Glu Asp Ile His
   340 345 350
   Val Pro Leu Leu Pro Gly Glu Gln Cys Gly Ala Leu Thr Lys Gln Val
   355 360 365
   Ala Gln Trp Ile Ala Asp Ile Gln Tyr Gly Arg Val Asn Tyr Gly Asn
   370 375 380
   Trp Ser Lys Thr Val Ala Asp Leu Asn
   385 390
<210> 11
   <211> 1131
   <212> DNA
   <213> Saccharomyces cerevisiae
<400> 11
   atgaccttgg cacccctaga cgcctccaaa gttaagataa ctaccacaca acatgcatct      60
   aagccaaaac cgaacagtga gttagtgttt ggcaagagct tcacggacca catgttaact     120
   gcggaatgga cagctgaaaa agggtggggt accccagaga ttaaacctta tcaaaatctg     180
   tctttagacc cttccgcggt ggttttccat tatgcttttg agctattcga agggatgaag     240
   gcttacagaa cggtggacaa caaaattaca atgtttcgtc cagatatgaa tatgaagcgc     300
   atgaataagt ctgctcagag aatctgtttg ccaacgttcg acccagaaga gttgattacc     360
   ctaattggga aactgatcca gcaagataag tgcttagttc ctgaaggaaa aggttactct     420
   ttatatatca ggcctacatt aatcggcact acggccggtt taggggtttc cacgcctgat     480
   agagccttgc tatatgtcat ttgctgccct gtgggtcctt attacaaaac tggatttaag     540
   gcggtcagac tggaagccac tgattatgcc acaagagctt ggccaggagg ctgtggtgac     600
   aagaaactag gtgcaaacta cgccccctgc gtcctgccac aattgcaagc tgcttcaagg     660
   ggttaccaac aaaatttatg gctatttggt ccaaataaca acattactga agtcggcacc     720
   atgaatgctt ttttcgtgtt taaagatagt aaaacgggca agaaggaact agttactgct     780
   ccactagacg gtaccatttt ggaaggtgtt actagggatt ccattttaaa tcttgctaaa     840
   gaaagactcg aaccaagtga atggaccatt agtgaacgct acttcactat aggcgaagtt     900
   actgagagat ccaagaacgg tgaactactt gaagcctttg gttctggtac tgctgcgatt     960
   gtttctccca ttaaggaaat cggctggaaa ggcgaacaaa ttaatattcc gttgttgccc    1020
   ggcgaacaaa ccggtccatt ggccaaagaa gttgcacaat ggattaatgg aatccaatat    1080
   ggcgagactg agcatggcaa ttggtcaagg gttgttactg atttgaactg a             1131
<210> 12
   <211> 376
   <212> PRT
   <213> Saccharomyces cerevisiae
<400> 12
   Met Thr Leu Ala Pro Leu Asp Ala Ser Lys Val Lys Ile Thr Thr Thr
   1               5                   10                  15
   Gln His Ala Ser Lys Pro Lys Pro Asn Ser Glu Leu Val Phe Gly Lys
               20                  25                  30
   Ser Phe Thr Asp His Met Leu Thr Ala Glu Trp Thr Ala Glu Lys Gly
           35                  40                  45
   Trp Gly Thr Pro Glu Ile Lys Pro Tyr Gln Asn Leu Ser Leu Asp Pro
       50                  55                  60
   Ser Ala Val Val Phe His Tyr Ala Phe Glu Leu Phe Glu Gly Met Lys
   65                  70                  75                  80
   Ala Tyr Arg Thr Val Asp Asn Lys Ile Thr Met Phe Arg Pro Asp Met
                   85                  90                  95
   Asn Met Lys Arg Met Asn Lys Ser Ala Gln Arg Ile Cys Leu Pro Thr
               100                 105                 110
   Phe Asp Pro Glu Glu Leu Ile Thr Leu Ile Gly Lys Leu Ile Gln Gln
           115                 120                 125
   Asp Lys Cys Leu Val Pro Glu Gly Lys Gly Tyr Ser Leu Tyr Ile Arg
       130                 135                 140
   Pro Thr Leu Ile Gly Thr Thr Ala Gly Leu Gly Val Ser Thr Pro Asp
   145                 150                 155                 160
   Arg Ala Leu Leu Tyr Val Ile Cys Cys Pro Val Gly Pro Tyr Tyr Lys
                   165                 170                 175
   Thr Gly Phe Lys Ala Val Arg Leu Glu Ala Thr Asp Tyr Ala Thr Arg
               180                 185                 190
   Ala Trp Pro Gly Gly Cys Gly Asp Lys Lys Leu Gly Ala Asn Tyr Ala
           195                 200                 205
   Pro Cys Val Leu Pro Gln Leu Gln Ala Ala Ser Arg Gly Tyr Gln Gln
       210                 215                 220
   Asn Leu Trp Leu Phe Gly Pro Asn Asn Asn Ile Thr Glu Val Gly Thr
   225                 230                 235                 240
   Met Asn Ala Phe Phe Val Phe Lys Asp Ser Lys Thr Gly Lys Lys Glu
                   245                 250                 255
   Leu Val Thr Ala Pro Leu Asp Gly Thr Ile Leu Glu Gly Val Thr Arg
               260                 265                 270
   Asp Ser Ile Leu Asn Leu Ala Lys Glu Arg Leu Glu Pro Ser Glu Trp
           275                 280                 285
   Thr Ile Ser Glu Arg Tyr Phe Thr Ile Gly Glu Val Thr Glu Arg Ser
       290                 295                 300
   Lys Asn Gly Glu Leu Leu Glu Ala Phe Gly Ser Gly Thr Ala Ala Ile
   305                 310                 315                 320
   Val Ser Pro Ile Lys Glu Ile Gly Trp Lys Gly Glu Gln Ile Asn Ile
                   325                 330                 335
   Pro Leu Leu Pro Gly Glu Gln Thr Gly Pro Leu Ala Lys Glu Val Ala
               340                 345                 350
   Gln Trp Ile Asn Gly Ile Gln Tyr Gly Glu Thr Glu His Gly Asn Trp
           355                 360                 365
   Ser Arg Val Val Thr Asp Leu Asn
       370                 375
<210> 13
   <211> 1692
   <212> DNA
   <213> Saccharomyces cerevisiae
<400> 13
   atgtctgaaa ttactttggg taaatatttg ttcgaaagat taaagcaagt caacgttaac 60
   accgttttcg gtttgccagg tgacttcaac ttgtccttgt tggacaagat ctacgaagtt 120
   gaaggtatga gatgggctgg taacgccaac gaattgaacg ctgcttacgc cgctgatggt 180
   tacgctcgta tcaagggtat gtcttgtatc atcaccacct tcggtgtcgg tgaattgtct 240
   gctttgaacg gtattgccgg ttcttacgct gaacacgtcg gtgttttgca cgttgttggt 300
   gtcccatcca tctctgctca agctaagcaa ttgttgttgc accacacctt gggtaacggt 360
   gacttcactg ttttccacag aatgtctgcc aacatttctg aaaccactgc tatgatcact 420
   gacattgcta ccgccccagc tgaaattgac agatgtatca gaaccactta cgtcacccaa 480
   agaccagtct acttaggttt gccagctaac ttggtcgact tgaacgtccc agctaagttg 540
   ttgcaaactc caattgacat gtctttgaag ccaaacgatg ctgaatccga aaaggaagtc 600
   attgacacca tcttggcttt ggtcaaggat gctaagaacc cagttatctt ggctgatgct 660
   tgttgttcca gacacgacgt caaggctgaa actaagaagt tgattgactt gactcaattc 720
   ccagctttcg tcaccccaat gggtaagggt tccattgacg aacaacaccc aagatacggt 780
   ggtgtttacg tcggtacctt gtccaagcca gaagttaagg aagccgttga atctgctgac 840
   ttgattttgt ctgtcggtgc tttgttgtct gatttcaaca ccggttcttt ctcttactct 900
   tacaagacca agaacattgt cgaattccac tccgaccaca tgaagatcag aaacgccact 960
   ttcccaggtg tccaaatgaa attcgttttg caaaagttgt tgaccactat tgctgacgcc 1020
   gctaagggtt acaagccagt tgctgtccca gctagaactc cagctaacgc tgctgtccca 1080
   gcttctaccc cattgaagca agaatggatg tggaaccaat tgggtaactt cttgcaagaa 1140
   ggtgatgttg tcattgctga aaccggtacc tccgctttcg gtatcaacca aaccactttc 1200
   ccaaacaaca cctacggtat ctctcaagtc ttatggggtt ccattggttt caccactggt 1260
   gctaccttgg gtgctgcttt cgctgctgaa gaaattgatc caaagaagag agttatctta 1320
   ttcattggtg acggttcttt gcaattgact gttcaagaaa tctccaccat gatcagatgg 1380
   ggcttgaagc catacttgtt cgtcttgaac aacgatggtt acaccattga aaagttgatt 1440
   cacggtccaa aggctcaata caacgaaatt caaggttggg accacctatc cttgttgcca 1500
   actttcggtg ctaaggacta tgaaacccac agagtcgcta ccaccggtga atgggacaag 1560
   ttgacccaag acaagtcttt caacgacaac tctaagatca gaatgattga aatcatgttg 1620
   ccagtcttcg atgctccaca aaacttggtt gaacaagcta agttgactgc tgctaccaac 1680
   gctaagcaat aa 1692
<210> 14
   <211> 563
   <212> PRT
   <213> Saccharomyces cerevisiae
<400> 14
   Met Ser Glu Ile Thr Leu Gly Lys Tyr Leu Phe Glu Arg Leu Lys Gln
   1               5                   10                  15
   Val Asn Val Asn Thr Val Phe Gly Leu Pro Gly Asp Phe Asn Leu Ser
               20                  25                  30
   Leu Leu Asp Lys Ile Tyr Glu Val Glu Gly Met Arg Trp Ala Gly Asn
           35                  40                  45
   Ala Asn Glu Leu Asn Ala Ala Tyr Ala Ala Asp Gly Tyr Ala Arg Ile
       50                  55                  60
   Lys Gly Met Ser Cys Ile Ile Thr Thr Phe Gly Val Gly Glu Leu Ser
   65                  70                  75                  80
   Ala Leu Asn Gly Ile Ala Gly Ser Tyr Ala Glu His Val Gly Val Leu
                   85                  90                  95
   His Val Val Gly Val Pro Ser Ile Ser Ala Gln Ala Lys Gln Leu Leu
               100                 105                 110
   Leu His His Thr Leu Gly Asn Gly Asp Phe Thr Val Phe His Arg Met
           115                 120                 125
   Ser Ala Asn Ile Ser Glu Thr Thr Ala Met Ile Thr Asp Ile Ala Thr
       130                 135                 140
   Ala Pro Ala Glu Ile Asp Arg Cys Ile Arg Thr Thr Tyr Val Thr Gln
   145                 150                 155                 160
   Arg Pro Val Tyr Leu Gly Leu Pro Ala Asn Leu Val Asp Leu Asn Val
                   165                 170                 175
   Pro Ala Lys Leu Leu Gln Thr Pro Ile Asp Met Ser Leu Lys Pro Asn
               180                 185                 190
   Asp Ala Glu Ser Glu Lys Glu Val Ile Asp Thr Ile Leu Ala Leu Val
           195                 200                 205
   Lys Asp Ala Lys Asn Pro Val Ile Leu Ala Asp Ala Cys Cys Ser Arg
       210                 215                 220
   His Asp Val Lys Ala Glu Thr Lys Lys Leu Ile Asp Leu Thr Gln Phe
   225                 230                 235                 240
   Pro Ala Phe Val Thr Pro Met Gly Lys Gly Ser Ile Asp Glu Gln His
                   245                 250                 255
   Pro Arg Tyr Gly Gly Val Tyr Val Gly Thr Leu Ser Lys Pro Glu Val
               260                 265                 270
   Lys Glu Ala Val Glu Ser Ala Asp Leu Ile Leu Ser Val Gly Ala Leu
           275                 280                 285
   Leu Ser Asp Phe Asn Thr Gly Ser Phe Ser Tyr Ser Tyr Lys Thr Lys
       290                 295                 300
   Asn Ile Val Glu Phe His Ser Asp His Met Lys Ile Arg Asn Ala Thr
   305                 310                 315                 320
   Phe Pro Gly Val Gln Met Lys Phe Val Leu Gln Lys Leu Leu Thr Thr
                   325                 330                 335
   Ile Ala Asp Ala Ala Lys Gly Tyr Lys Pro Val Ala Val Pro Ala Arg
               340                 345                 350
   Thr Pro Ala Asn Ala Ala Val Pro Ala Ser Thr Pro Leu Lys Gln Glu
           355                 360                 365
   Trp Met Trp Asn Gln Leu Gly Asn Phe Leu Gln Glu Gly Asp Val Val
       370                 375                 380
   Ile Ala Glu Thr Gly Thr Ser Ala Phe Gly Ile Asn Gln Thr Thr Phe
   385                 390                 395                 400
   Pro Asn Asn Thr Tyr Gly Ile Ser Gln Val Leu Trp Gly Ser Ile Gly
                   405                 410                 415
   Phe Thr Thr Gly Ala Thr Leu Gly Ala Ala Phe Ala Ala Glu Glu Ile
               420                 425                 430
   Asp Pro Lys Lys Arg Val Ile Leu Phe Ile Gly Asp Gly Ser Leu Gln
           435                 440                 445
   Leu Thr Val Gln Glu Ile Ser Thr Met Ile Arg Trp Gly Leu Lys Pro
       450                 455                 460
   Tyr Leu Phe Val Leu Asn Asn Asp Gly Tyr Thr Ile Glu Lys Leu Ile
   465                 470                 475                 480
   His Gly Pro Lys Ala Gln Tyr Asn Glu Ile Gln Gly Trp Asp His Leu
                   485                 490                 495
   Ser Leu Leu Pro Thr Phe Gly Ala Lys Asp Tyr Glu Thr His Arg Val
               500                 505                 510
   Ala Thr Thr Gly Glu Trp Asp Lys Leu Thr Gln Asp Lys Ser Phe Asn
           515                 520                 525
   Asp Asn Ser Lys Ile Arg Met Ile Glu Ile Met Leu Pro Val Phe Asp
       530                 535                 540
   Ala Pro Gln Asn Leu Val Glu Gln Ala Lys Leu Thr Ala Ala Thr Asn
   545                 550                 555                 560
   Ala Lys Gln
<210> 15
   <211> 1692
   <212> DNA
   <213> Saccharomyces cerevisiae
<400> 15
   atgtctgaaa taaccttagg taaatattta tttgaaagat tgagccaagt caactgtaac      60
   accgtcttcg gtttgccagg tgactttaac ttgtctcttt tggataagct ttatgaagtc     120
   aaaggtatga gatgggctgg taacgctaac gaattgaacg ctgcctatgc tgctgatggt     180
   tacgctcgta tcaagggtat gtcctgtatt attaccacct tcggtgttgg tgaattgtct     240
   gctttgaatg gtattgccgg ttcttacgct gaacatgtcg gtgttttgca cgttgttggt     300
   gttccatcca tctcttctca agctaagcaa ttgttgttgc atcatacctt gggtaacggt     360
   gacttcactg ttttccacag aatgtctgcc aacatttctg aaaccactgc catgatcact     420
   gatattgcta acgctccagc tgaaattgac agatgtatca gaaccaccta cactacccaa     480
   agaccagtct acttgggttt gccagctaac ttggttgact tgaacgtccc agccaagtta     540
   ttggaaactc caattgactt gtctttgaag ccaaacgacg ctgaagctga agctgaagtt     600
   gttagaactg ttgttgaatt gatcaaggat gctaagaacc cagttatctt ggctgatgct     660
   tgtgcttcta gacatgatgt caaggctgaa actaagaagt tgatggactt gactcaattc     720
   ccagtttacg tcaccccaat gggtaagggt gctattgacg aacaacaccc aagatacggt     780
   ggtgtttacg ttggtacctt gtctagacca gaagttaaga aggctgtaga atctgctgat     840
   ttgatattgt ctatcggtgc tttgttgtct gatttcaata ccggttcttt ctcttactcc     900
   tacaagacca aaaatatcgt tgaattccac tctgaccaca tcaagatcag aaacgccacc     960
   ttcccaggtg ttcaaatgaa atttgccttg caaaaattgt tggatgctat tccagaagtc    1020
   gtcaaggact acaaacctgt tgctgtccca gctagagttc caattaccaa gtctactcca    1080
   gctaacactc caatgaagca agaatggatg tggaaccatt tgggtaactt cttgagagaa    1140
   ggtgatattg ttattgctga aaccggtact tccgccttcg gtattaacca aactactttc    1200
   ccaacagatg tatacgctat cgtccaagtc ttgtggggtt ccattggttt cacagtcggc    1260
   gctctattgg gtgctactat ggccgctgaa gaacttgatc caaagaagag agttatttta    1320
   ttcattggtg acggttctct acaattgact gttcaagaaa tctctaccat gattagatgg    1380
   ggtttgaagc catacatttt tgtcttgaat aacaacggtt acaccattga aaaattgatt    1440
   cacggtcctc atgccgaata taatgaaatt caaggttggg accacttggc cttattgcca    1500
   acttttggtg ctagaaacta cgaaacccac agagttgcta ccactggtga atgggaaaag    1560
   ttgactcaag acaaggactt ccaagacaac tctaagatta gaatgattga agttatgttg    1620
   ccagtctttg atgctccaca aaacttggtt aaacaagctc aattgactgc cgctactaac    1680
   gctaaacaat aa                                                        1692
<210> 16
   <211> 563
   <212> PRT
   <213> Saccharomyces cerevisiae
<400> 16
   Met Ser Glu Ile Thr Leu Gly Lys Tyr Leu Phe Glu Arg Leu Ser Gln
   1               5                   10                  15
   Val Asn Cys Asn Thr Val Phe Gly Leu Pro Gly Asp Phe Asn Leu Ser
               20                  25                  30
   Leu Leu Asp Lys Leu Tyr Glu Val Lys Gly Met Arg Trp Ala Gly Asn
           35                  40                  45
   Ala Asn Glu Leu Asn Ala Ala Tyr Ala Ala Asp Gly Tyr Ala Arg Ile
       50                  55                  60
   Lys Gly Met Ser Cys Ile Ile Thr Thr Phe Gly Val Gly Glu Leu Ser
   65                  70                  75                  80
   Ala Leu Asn Gly Ile Ala Gly Ser Tyr Ala Glu His Val Gly Val Leu
                   85                  90                  95
   His Val Val Gly Val Pro Ser Ile Ser Ser Gln Ala Lys Gln Leu Leu
               100                 105                 110
   Leu His His Thr Leu Gly Asn Gly Asp Phe Thr Val Phe His Arg Met
           115                 120                 125
   Ser Ala Asn Ile Ser Glu Thr Thr Ala Met Ile Thr Asp Ile Ala Asn
       130                 135                 140
   Ala Pro Ala Glu Ile Asp Arg Cys Ile Arg Thr Thr Tyr Thr Thr Gln
   145                 150                 155                 160
   Arg Pro Val Tyr Leu Gly Leu Pro Ala Asn Leu Val Asp Leu Asn Val
                   165                 170                 175
   Pro Ala Lys Leu Leu Glu Thr Pro Ile Asp Leu Ser Leu Lys Pro Asn
               180                 185                 190
   Asp Ala Glu Ala Glu Ala Glu Val Val Arg Thr Val Val Glu Leu Ile
           195                 200                 205
   Lys Asp Ala Lys Asn Pro Val Ile Leu Ala Asp Ala Cys Ala Ser Arg
       210                 215                 220
   His Asp Val Lys Ala Glu Thr Lys Lys Leu Met Asp Leu Thr Gln Phe
   225                 230                 235                 240
   Pro Val Tyr Val Thr Pro Met Gly Lys Gly Ala Ile Asp Glu Gln His
                   245                 250                 255
   Pro Arg Tyr Gly Gly Val Tyr Val Gly Thr Leu Ser Arg Pro Glu Val
               260                 265                 270
   Lys Lys Ala Val Glu Ser Ala Asp Leu Ile Leu Ser Ile Gly Ala Leu
           275                 280                 285
   Leu Ser Asp Phe Asn Thr Gly Ser Phe Ser Tyr Ser Tyr Lys Thr Lys
       290                 295                 300
   Asn Ile Val Glu Phe His Ser Asp His Ile Lys Ile Arg Asn Ala Thr
   305                 310                 315                 320
   Phe Pro Gly Val Gln Met Lys Phe Ala Leu Gln Lys Leu Leu Asp Ala
                   325                 330                 335
   Ile Pro Glu Val Val Lys Asp Tyr Lys Pro Val Ala Val Pro Ala Arg
               340                 345                 350
   Val Pro Ile Thr Lys Ser Thr Pro Ala Asn Thr Pro Met Lys Gln Glu
           355                 360                 365
   Trp Met Trp Asn His Leu Gly Asn Phe Leu Arg Glu Gly Asp Ile Val
       370                 375                 380
   Ile Ala Glu Thr Gly Thr Ser Ala Phe Gly Ile Asn Gln Thr Thr Phe
   385                 390                 395                 400
   Pro Thr Asp Val Tyr Ala Ile Val Gln Val Leu Trp Gly Ser Ile Gly
                   405                 410                 415
   Phe Thr Val Gly Ala Leu Leu Gly Ala Thr Met Ala Ala Glu Glu Leu
               420                 425                 430
   Asp Pro Lys Lys Arg Val Ile Leu Phe Ile Gly Asp Gly Ser Leu Gln
           435                 440                 445
   Leu Thr Val Gln Glu Ile Ser Thr Met Ile Arg Trp Gly Leu Lys Pro
       450                 455                 460
   Tyr Ile Phe Val Leu Asn Asn Asn Gly Tyr Thr Ile Glu Lys Leu Ile
   465                 470                 475                 480
   His Gly Pro His Ala Glu Tyr Asn Glu Ile Gln Gly Trp Asp His Leu
                   485                 490                 495
   Ala Leu Leu Pro Thr Phe Gly Ala Arg Asn Tyr Glu Thr His Arg Val
               500                 505                 510
   Ala Thr Thr Gly Glu Trp Glu Lys Leu Thr Gln Asp Lys Asp Phe Gln
           515                 520                 525
   Asp Asn Ser Lys Ile Arg Met Ile Glu Val Met Leu Pro Val Phe Asp
   530 535 540
   Ala Pro Gln Asn Leu Val Lys Gln Ala Gln Leu Thr Ala Ala Thr Asn
   545 550 555 560
   Ala Lys Gln
<210> 17
   <211> 1692
   <212> DNA
   <213> Saccharomyces cerevisiae
<400> 17
   atgtctgaaa ttactcttgg aaaatactta tttgaaagat tgaagcaagt taatgttaac 60
   accatttttg ggctaccagg cgacttcaac ttgtccctat tggacaagat ttacgaggta 120
   gatggattga gatgggctgg taatgcaaat gagctgaacg ccgcctatgc cgccgatggt 180
   tacgcacgca tcaagggttt atctgtgctg gtaactactt ttggcgtagg tgaattatcc 240
   gccttgaatg gtattgcagg atcgtatgca gaacacgtcg gtgtactgca tgttgttggt 300
   gtcccctcta tctccgctca ggctaagcaa ttgttgttgc atcatacctt gggtaacggt 360
   gattttaccg tttttcacag aatgtccgcc aatatctcag aaactacatc aatgattaca 420
   gacattgcta cagccccttc agaaatcgat aggttgatca ggacaacatt tataacacaa 480
   aggcctagct acttggggtt gccagcgaat ttggtagatc taaaggttcc tggttctctt 540
   ttggaaaaac cgattgatct atcattaaaa cctaacgatc ccgaagctga aaaggaagtt 600
   attgataccg tactagaatt gatccagaat tcgaaaaacc ctgttatact atcggatgcc 660
   tgtgcttcta ggcacaacgt taaaaaagaa acccagaagt taattgattt gacgcaattc 720
   ccagcttttg tgacacctct aggtaaaggg tcaatagatg aacagcatcc cagatatggc 780
   ggtgtttatg tgggaacgct gtccaaacaa gacgtgaaac aggccgttga gtcggctgat 840
   ttgatccttt cggtcggtgc tttgctctct gattttaaca caggttcgtt ttcctactcc 900
   tacaagacta aaaatgtagt ggagtttcat tccgattacg taaaggtgaa gaacgctacg 960
   ttcctcggtg tacaaatgaa atttgcacta caaaacttac tgaaggttat tcccgatgtt 1020
   gttaagggct acaagagcgt tcccgtacca accaaaactc ccgcaaacaa aggtgtacct 1080
   gctagcacgc ccttgaaaca agagtggttg tggaacgaat tgtccaaatt cttgcaagaa 1140
   ggtgatgtta tcatttccga gaccggcacg tctgccttcg gtatcaatca aactatcttt 1200
   cctaaggacg cctacggtat ctcgcaggtg ttgtgggggt ccatcggttt tacaacagga 1260
   gcaactttag gtgctgcctt tgccgctgag gagattgacc ccaacaagag agtcatctta 1320
   ttcataggtg acgggtcttt gcagttaacc gtccaagaaa tctccaccat gatcagatgg 1380
   gggttaaagc cgtatctttt tgtccttaac aacgacggct acactatcga aaagctgatt 1440
   catgggcctc acgcagagta caacgaaatc cagacctggg atcacctcgc cctgttgccc 1500
   gcatttggtg cgaaaaagta cgaaaatcac aagatcgcca ctacgggtga gtgggatgcc 1560
   ttaaccactg attcagagtt ccagaaaaac tcggtgatca gactaattga actgaaactg 1620
   cccgtctttg atgctccgga aagtttgatc aaacaagcgc aattgactgc cgctacaaat 1680
   gccaaacaat aa 1692
<210> 18
   <211> 563
   <212> PRT
   <213> Saccharomyces cerevisiae
<400> 18
   Met Ser Glu Ile Thr Leu Gly Lys Tyr Leu Phe Glu Arg Leu Lys Gln
   1 5 10 15
   Val Asn Val Asn Thr Ile Phe Gly Leu Pro Gly Asp Phe Asn Leu Ser
   20 25 30
   Leu Leu Asp Lys Ile Tyr Glu Val Asp Gly Leu Arg Trp Ala Gly Asn
   35 40 45
   Ala Asn Glu Leu Asn Ala Ala Tyr Ala Ala Asp Gly Tyr Ala Arg Ile
   50 55 60
   Lys Gly Leu Ser Val Leu Val Thr Thr Phe Gly Val Gly Glu Leu Ser
   65 70 75 80
   Ala Leu Asn Gly Ile Ala Gly Ser Tyr Ala Glu His Val Gly Val Leu
   85 90 95
   His Val Val Gly Val Pro Ser Ile Ser Ala Gln Ala Lys Gln Leu Leu
   100 105 110
   Leu His His Thr Leu Gly Asn Gly Asp Phe Thr Val Phe His Arg Met
   115 120 125
   Ser Ala Asn Ile Ser Glu Thr Thr Ser Met Ile Thr Asp Ile Ala Thr
   130 135 140
   Ala Pro Ser Glu Ile Asp Arg Leu Ile Arg Thr Thr Phe Ile Thr Gln
   145 150 155 160
   Arg Pro Ser Tyr Leu Gly Leu Pro Ala Asn Leu Val Asp Leu Lys Val
   165 170 175
   Pro Gly Ser Leu Leu Glu Lys Pro Ile Asp Leu Ser Leu Lys Pro Asn
   180 185 190
   Asp Pro Glu Ala Glu Lys Glu Val Ile Asp Thr Val Leu Glu Leu Ile
   195 200 205
   Gln Asn Ser Lys Asn Pro Val Ile Leu Ser Asp Ala Cys Ala Ser Arg
       210                 215                 220
   His Asn Val Lys Lys Glu Thr Gln Lys Leu Ile Asp Leu Thr Gln Phe
   225                 230                 235                 240
   Pro Ala Phe Val Thr Pro Leu Gly Lys Gly Ser Ile Asp Glu Gln His
                   245                 250                 255
   Pro Arg Tyr Gly Gly Val Tyr Val Gly Thr Leu Ser Lys Gln Asp Val
               260                 265                 270
   Lys Gln Ala Val Glu Ser Ala Asp Leu Ile Leu Ser Val Gly Ala Leu
           275                 280                 285
   Leu Ser Asp Phe Asn Thr Gly Ser Phe Ser Tyr Ser Tyr Lys Thr Lys
       290                 295                 300
   Asn Val Val Glu Phe His Ser Asp Tyr Val Lys Val Lys Asn Ala Thr
   305                 310                 315                 320
   Phe Leu Gly Val Gln Met Lys Phe Ala Leu Gln Asn Leu Leu Lys Val
                   325                 330                 335
   Ile Pro Asp Val Val Lys Gly Tyr Lys Ser Val Pro Val Pro Thr Lys
               340                 345                 350
   Thr Pro Ala Asn Lys Gly Val Pro Ala Ser Thr Pro Leu Lys Gln Glu
           355                 360                 365
   Trp Leu Trp Asn Glu Leu Ser Lys Phe Leu Gln Glu Gly Asp Val Ile
       370                 375                 380
   Ile Ser Glu Thr Gly Thr Ser Ala Phe Gly Ile Asn Gln Thr Ile Phe
   385                 390                 395                 400
   Pro Lys Asp Ala Tyr Gly Ile Ser Gln Val Leu Trp Gly Ser Ile Gly
                   405                 410                 415
   Phe Thr Thr Gly Ala Thr Leu Gly Ala Ala Phe Ala Ala Glu Glu Ile
               420                 425                 430
   Asp Pro Asn Lys Arg Val Ile Leu Phe Ile Gly Asp Gly Ser Leu Gln
           435                 440                 445
   Leu Thr Val Gln Glu Ile Ser Thr Met Ile Arg Trp Gly Leu Lys Pro
       450                 455                 460
   Tyr Leu Phe Val Leu Asn Asn Asp Gly Tyr Thr Ile Glu Lys Leu Ile
   465                 470                 475                 480
   His Gly Pro His Ala Glu Tyr Asn Glu Ile Gln Thr Trp Asp His Leu
   485 490 495
   Ala Leu Leu Pro Ala Phe Gly Ala Lys Lys Tyr Glu Asn His Lys Ile
   500 505 510
   Ala Thr Thr Gly Glu Trp Asp Ala Leu Thr Thr Asp Ser Glu Phe Gln
   515 520 525
   Lys Asn Ser Val Ile Arg Leu Ile Glu Leu Lys Leu Pro Val Phe Asp
   530 535 540
   Ala Pro Glu Ser Leu Ile Lys Gln Ala Gln Leu Thr Ala Ala Thr Asn
   545 550 555 560
   Ala Lys Gln
<210> 19
   <211> 1908
   <212> DNA
   <213> Saccharomyces cerevisiae
<400> 19
   atggcacctg ttacaattga aaagttcgta aatcaagaag aacgacacct tgtttccaac      60
   cgatcagcaa caattccgtt tggtgaatac atatttaaaa gattgttgtc catcgatacg     120
   aaatcagttt tcggtgttcc tggtgacttc aacttatctc tattagaata tctctattca     180
   cctagtgttg aatcagctgg cctaagatgg gtcggcacgt gtaatgaact gaacgccgct     240
   tatgcggccg acggatattc ccgttactct aataagattg gctgtttaat aaccacgtat     300
   ggcgttggtg aattaagcgc cttgaacggt atagccggtt cgttcgctga aaatgtcaaa     360
   gttttgcaca ttgttggtgt ggccaagtcc atagattcgc gttcaagtaa ctttagtgat     420
   cggaacctac atcatttggt cccacagcta catgattcaa attttaaagg gccaaatcat     480
   aaagtatatc atgatatggt aaaagataga gtcgcttgct cggtagccta cttggaggat     540
   attgaaactg catgtgacca agtcgataat gttatccgcg atatttacaa gtattctaaa     600
   cctggttata tttttgttcc tgcagatttt gcggatatgt ctgttacatg tgataatttg     660
   gttaatgttc cacgtatatc tcaacaagat tgtatagtat acccttctga aaaccaattg     720
   tctgacataa tcaacaagat tactagttgg atatattcca gtaaaacacc tgcgatcctt     780
   ggagacgtac tgactgatag gtatggtgtg agtaactttt tgaacaagct tatctgcaaa     840
   actgggattt ggaatttttc cactgttatg ggaaaatctg taattgatga gtcaaaccca     900
   acttatatgg gtcaatataa tggtaaagaa ggtttaaaac aagtctatga acattttgaa     960
   ctgtgcgact tggtcttgca ttttggagtc gacatcaatg aaattaataa tgggcattat    1020
   acttttactt ataaaccaaa tgctaaaatc attcaatttc atccgaatta tattcgcctt    1080
   gtggacacta ggcagggcaa tgagcaaatg ttcaaaggaa tcaattttgc ccctatttta    1140
   aaagaactat acaagcgcat tgacgtttct aaactttctt tgcaatatga ttcaaatgta    1200
   actcaatata cgaacgaaac aatgcggtta gaagatccta ccaatggaca atcaagcatt    1260
   attacacaag ttcacttaca aaagacgatg cctaaatttt tgaaccctgg tgatgttgtc    1320
   gtttgtgaaa caggctcttt tcaattctct gttcgtgatt tcgcgtttcc ttcgcaatta    1380
   aaatatatat cgcaaggatt tttcctttcc attggcatgg cccttcctgc cgccctaggt    1440
   gttggaattg ccatgcaaga ccactcaaac gctcacatca atggtggcaa cgtaaaagag    1500
   gactataagc caagattaat tttgtttgaa ggtgacggtg cagcacagat gacaatccaa    1560
   gaactgagca ccattctgaa gtgcaatatt ccactagaag ttatcatttg gaacaataac    1620
   ggctacacta ttgaaagagc catcatgggc cctaccaggt cgtataacga cgttatgtct    1680
   tggaaatgga ccaaactatt tgaagcattc ggagacttcg acggaaagta tactaatagc    1740
   actctcattc aatgtccctc taaattagca ctgaaattgg aggagcttaa gaattcaaac    1800
   aaaagaagcg ggatagaact tttagaagtc aaattaggcg aattggattt ccccgaacag    1860
   ctaaagtgca tggttgaagc agcggcactt aaaagaaata aaaaatag                  1908
<210> 20
   <211> 635
   <212> PRT
   <213> Saccharomyces cerevisiae
<400> 20
   Met Ala Pro Val Thr Ile Glu Lys Phe Val Asn Gln Glu Glu Arg His
   1               5                   10                  15
   Leu Val Ser Asn Arg Ser Ala Thr Ile Pro Phe Gly Glu Tyr Ile Phe
               20                  25                  30
   Lys Arg Leu Leu Ser Ile Asp Thr Lys Ser Val Phe Gly Val Pro Gly
           35                  40                  45
   Asp Phe Asn Leu Ser Leu Leu Glu Tyr Leu Tyr Ser Pro Ser Val Glu
       50                  55                  60
   Ser Ala Gly Leu Arg Trp Val Gly Thr Cys Asn Glu Leu Asn Ala Ala
   65                  70                  75                  80
   Tyr Ala Ala Asp Gly Tyr Ser Arg Tyr Ser Asn Lys Ile Gly Cys Leu
                   85                  90                  95
   Ile Thr Thr Tyr Gly Val Gly Glu Leu Ser Ala Leu Asn Gly Ile Ala
               100                 105                 110
   Gly Ser Phe Ala Glu Asn Val Lys Val Leu His Ile Val Gly Val Ala
           115                 120                 125
   Lys Ser Ile Asp Ser Arg Ser Ser Asn Phe Ser Asp Arg Asn Leu His
   130 135 140
   His Leu Val Pro Gln Leu His Asp Ser Asn Phe Lys Gly Pro Asn His
   145 150 155 160
   Lys Val Tyr His Asp Met Val Lys Asp Arg Val Ala Cys Ser Val Ala
   165 170 175
   Tyr Leu Glu Asp Ile Glu Thr Ala Cys Asp Gln Val Asp Asn Val Ile
   180 185 190
   Arg Asp Ile Tyr Lys Tyr Ser Lys Pro Gly Tyr Ile Phe Val Pro Ala
   195 200 205
   Asp Phe Ala Asp Met Ser Val Thr Cys Asp Asn Leu Val Asn Val Pro
   210 215 220
   Arg Ile Ser Gln Gln Asp Cys Ile Val Tyr Pro Ser Glu Asn Gln Leu
   225 230 235 240
   Ser Asp Ile Ile Asn Lys Ile Thr Ser Trp Ile Tyr Ser Ser Lys Thr
   245 250 255
   Pro Ala Ile Leu Gly Asp Val Leu Thr Asp Arg Tyr Gly Val Ser Asn
   260 265 270
   Phe Leu Asn Lys Leu Ile Cys Lys Thr Gly Ile Trp Asn Phe Ser Thr
   275 280 285
   Val Met Gly Lys Ser Val Ile Asp Glu Ser Asn Pro Thr Tyr Met Gly
   290 295 300
   Gln Tyr Asn Gly Lys Glu Gly Leu Lys Gln Val Tyr Glu His Phe Glu
   305 310 315 320
   Leu Cys Asp Leu Val Leu His Phe Gly Val Asp Ile Asn Glu Ile Asn
   325 330 335
   Asn Gly His Tyr Thr Phe Thr Tyr Lys Pro Asn Ala Lys Ile Ile Gln
   340 345 350
   Phe His Pro Asn Tyr Ile Arg Leu Val Asp Thr Arg Gln Gly Asn Glu
   355 360 365
   Gln Met Phe Lys Gly Ile Asn Phe Ala Pro Ile Leu Lys Glu Leu Tyr
   370 375 380
   Lys Arg Ile Asp Val Ser Lys Leu Ser Leu Gln Tyr Asp Ser Asn Val
   385 390 395 400
   Thr Gln Tyr Thr Asn Glu Thr Met Arg Leu Glu Asp Pro Thr Asn Gly
                   405                 410                 415
   Gln Ser Ser Ile Ile Thr Gln Val His Leu Gln Lys Thr Met Pro Lys
               420                 425                 430
   Phe Leu Asn Pro Gly Asp Val Val Val Cys Glu Thr Gly Ser Phe Gln
           435                 440                 445
   Phe Ser Val Arg Asp Phe Ala Phe Pro Ser Gln Leu Lys Tyr Ile Ser
       450                 455                 460
   Gln Gly Phe Phe Leu Ser Ile Gly Met Ala Leu Pro Ala Ala Leu Gly
   465                 470                 475                 480
   Val Gly Ile Ala Met Gln Asp His Ser Asn Ala His Ile Asn Gly Gly
                   485                 490                 495
   Asn Val Lys Glu Asp Tyr Lys Pro Arg Leu Ile Leu Phe Glu Gly Asp
               500                 505                 510
   Gly Ala Ala Gln Met Thr Ile Gln Glu Leu Ser Thr Ile Leu Lys Cys
           515                 520                 525
   Asn Ile Pro Leu Glu Val Ile Ile Trp Asn Asn Asn Gly Tyr Thr Ile
       530                 535                 540
   Glu Arg Ala Ile Met Gly Pro Thr Arg Ser Tyr Asn Asp Val Met Ser
   545                 550                 555                 560
   Trp Lys Trp Thr Lys Leu Phe Glu Ala Phe Gly Asp Phe Asp Gly Lys
                   565                 570                 575
   Tyr Thr Asn Ser Thr Leu Ile Gln Cys Pro Ser Lys Leu Ala Leu Lys
               580                 585                 590
   Leu Glu Glu Leu Lys Asn Ser Asn Lys Arg Ser Gly Ile Glu Leu Leu
           595                 600                 605
   Glu Val Lys Leu Gly Glu Leu Asp Phe Pro Glu Gln Leu Lys Cys Met
       610                 615                 620
   Val Glu Ala Ala Ala Leu Lys Arg Asn Lys Lys
   625                 630                 635
<210> 21
   <211> 1830
   <212> DNA
   <213> Saccharomyces cerevisiae
<400> 21
   atgaattcta gctatacaca gagatatgca ctgccgaagt gtatagcaat atcagattat      60
   cttttccatc ggctcaacca gctgaacata cataccatat ttggactctc cggagaattt     120
   agcatgccgt tgctggataa actatacaac attccgaact tacgatgggc cggtaattct     180
   aatgagttaa atgctgccta cgcagcagat ggatactcac gactaaaagg cttgggatgt     240
   ctcataacaa cctttggtgt aggcgaatta tcggcaatca atggcgtggc cggatcttac     300
   gctgaacatg taggaatact tcacatagtg ggtatgccgc caacaagtgc acaaacgaaa     360
   caactactac tgcatcatac tctgggcaat ggtgatttca cggtatttca tagaatagcc     420
   agtgatgtag catgctatac aacattgatt attgactctg aattatgtgc cgacgaagtc     480
   gataagtgca tcaaaaaggc ttggatagaa cagaggccag tatacatggg catgcctgtc     540
   aaccaggtaa atctcccgat tgaatcagca aggcttaata cacctctgga tttacaattg     600
   cataaaaacg acccagacgt agagaaagaa gttatttctc gaatattgag ttttatatac     660
   aaaagccaga atccggcaat catcgtagat gcatgtacta gtcgacagaa tttaatcgag     720
   gagactaaag agctttgtaa taggcttaaa tttccagttt ttgttacacc tatgggtaag     780
   ggtacagtaa acgaaacaga cccgcaattt gggggcgtat tcacgggctc gatatcagcc     840
   ccagaagtaa gagaagtagt tgattttgcc gattttatca tcgtcattgg ttgcatgctc     900
   tccgaattca gcacgtcaac tttccacttc caatataaaa ctaagaattg tgcgctacta     960
   tattctacat ctgtgaaatt gaaaaatgcc acatatcctg acttgagcat taaattacta    1020
   ctacagaaaa tattagcaaa tcttgatgaa tctaaactgt cttaccaacc aagcgaacaa    1080
   cccagtatga tggttccaag accttaccca gcaggaaatg tcctcttgag acaagaatgg    1140
   gtctggaatg aaatatccca ttggttccaa ccaggtgaca taatcataac agaaactggt    1200
   gcttctgcat ttggagttaa ccagaccaga tttccggtaa atacactagg tatttcgcaa    1260
   gctctttggg gatctgtcgg atatacaatg ggggcgtgtc ttggggcaga atttgctgtt    1320
   caagagataa acaaggataa attccccgca actaaacata gagttattct gtttatgggt    1380
   gacggtgctt tccaattgac agttcaagaa ttatccacaa ttgttaagtg gggattgaca    1440
   ccttatattt ttgtgatgaa taaccaaggt tactctgtgg acaggttttt gcatcacagg    1500
   tcagatgcta gttattacga tatccaacct tggaactact tgggattatt gcgagtattt    1560
   ggttgcacga actacgaaac gaaaaaaatt attactgttg gagaattcag atccatgatc    1620
   agtgacccaa actttgcgac caatgacaaa attcggatga tagagattat gctaccacca    1680
   agggatgttc cacaggctct gcttgacagg tgggtggtag aaaaagaaca gagcaaacaa    1740
   gtgcaagagg agaacgaaaa ttctagcgca gtaaatacgc caactccaga attccaacca    1800
   cttctaaaaa aaaatcaagt tggatactga                                     1830
<210> 22
   <211> 609
   <212> PRT
   <213> Saccharomyces cerevisiae
<400> 22
   Met Asn Ser Ser Tyr Thr Gln Arg Tyr Ala Leu Pro Lys Cys Ile Ala
   1               5                   10                  15
   Ile Ser Asp Tyr Leu Phe His Arg Leu Asn Gln Leu Asn Ile His Thr
               20                  25                  30
   Ile Phe Gly Leu Ser Gly Glu Phe Ser Met Pro Leu Leu Asp Lys Leu
           35                  40                  45
   Tyr Asn Ile Pro Asn Leu Arg Trp Ala Gly Asn Ser Asn Glu Leu Asn
       50                  55                  60
   Ala Ala Tyr Ala Ala Asp Gly Tyr Ser Arg Leu Lys Gly Leu Gly Cys
   65                  70                  75                  80
   Leu Ile Thr Thr Phe Gly Val Gly Glu Leu Ser Ala Ile Asn Gly Val
                   85                  90                  95
   Ala Gly Ser Tyr Ala Glu His Val Gly Ile Leu His Ile Val Gly Met
               100                 105                 110
   Pro Pro Thr Ser Ala Gln Thr Lys Gln Leu Leu Leu His His Thr Leu
           115                 120                 125
   Gly Asn Gly Asp Phe Thr Val Phe His Arg Ile Ala Ser Asp Val Ala
       130                 135                 140
   Cys Tyr Thr Thr Leu Ile Ile Asp Ser Glu Leu Cys Ala Asp Glu Val
   145                 150                 155                 160
   Asp Lys Cys Ile Lys Lys Ala Trp Ile Glu Gln Arg Pro Val Tyr Met
                   165                 170                 175
   Gly Met Pro Val Asn Gln Val Asn Leu Pro Ile Glu Ser Ala Arg Leu
               180                 185                 190
   Asn Thr Pro Leu Asp Leu Gln Leu His Lys Asn Asp Pro Asp Val Glu
           195                 200                 205
   Lys Glu Val Ile Ser Arg Ile Leu Ser Phe Ile Tyr Lys Ser Gln Asn
       210                 215                 220
   Pro Ala Ile Ile Val Asp Ala Cys Thr Ser Arg Gln Asn Leu Ile Glu
   225                 230                 235                 240
   Glu Thr Lys Glu Leu Cys Asn Arg Leu Lys Phe Pro Val Phe Val Thr
                   245                 250                 255
   Pro Met Gly Lys Gly Thr Val Asn Glu Thr Asp Pro Gln Phe Gly Gly
               260                 265                 270
   Val Phe Thr Gly Ser Ile Ser Ala Pro Glu Val Arg Glu Val Val Asp
           275                 280                 285
   Phe Ala Asp Phe Ile Ile Val Ile Gly Cys Met Leu Ser Glu Phe Ser
       290                 295                 300
   Thr Ser Thr Phe His Phe Gln Tyr Lys Thr Lys Asn Cys Ala Leu Leu
   305                 310                 315                 320
   Tyr Ser Thr Ser Val Lys Leu Lys Asn Ala Thr Tyr Pro Asp Leu Ser
                   325                 330                 335
   Ile Lys Leu Leu Leu Gln Lys Ile Leu Ala Asn Leu Asp Glu Ser Lys
               340                 345                 350
   Leu Ser Tyr Gln Pro Ser Glu Gln Pro Ser Met Met Val Pro Arg Pro
           355                 360                 365
   Tyr Pro Ala Gly Asn Val Leu Leu Arg Gln Glu Trp Val Trp Asn Glu
       370                 375                 380
   Ile Ser His Trp Phe Gln Pro Gly Asp Ile Ile Ile Thr Glu Thr Gly
   385                 390                 395                 400
   Ala Ser Ala Phe Gly Val Asn Gln Thr Arg Phe Pro Val Asn Thr Leu
                   405                 410                 415
   Gly Ile Ser Gln Ala Leu Trp Gly Ser Val Gly Tyr Thr Met Gly Ala
               420                 425                 430
   Cys Leu Gly Ala Glu Phe Ala Val Gln Glu Ile Asn Lys Asp Lys Phe
           435                 440                 445
   Pro Ala Thr Lys His Arg Val Ile Leu Phe Met Gly Asp Gly Ala Phe
       450                 455                 460
   Gln Leu Thr Val Gln Glu Leu Ser Thr Ile Val Lys Trp Gly Leu Thr
   465                 470                 475                 480
   Pro Tyr Ile Phe Val Met Asn Asn Gln Gly Tyr Ser Val Asp Arg Phe
                   485                 490                 495
   Leu His His Arg Ser Asp Ala Ser Tyr Tyr Asp Ile Gln Pro Trp Asn
               500                 505                 510
   Tyr Leu Gly Leu Leu Arg Val Phe Gly Cys Thr Asn Tyr Glu Thr Lys
           515                 520                 525
   Lys Ile Ile Thr Val Gly Glu Phe Arg Ser Met Ile Ser Asp Pro Asn
       530                 535                 540
   Phe Ala Thr Asn Asp Lys Ile Arg Met Ile Glu Ile Met Leu Pro Pro
   545 550 555 560
   Arg Asp Val Pro Gln Ala Leu Leu Asp Arg Trp Val Val Glu Lys Glu
   565 570 575
   Gln Ser Lys Gln Val Gln Glu Glu Asn Glu Asn Ser Ser Ala Val Asn
   580 585 590
   Thr Pro Thr Pro Glu Phe Gln Pro Leu Leu Lys Lys Asn Gln Val Gly
   595 600 605
   Tyr
<210> 23
   <211> 1047
   <212> DNA
   <213> Saccharomyces cerevisiae
<400> 23
   atgtctatcc cagaaactca aaaaggtgtt atcttctacg aatcccacgg taagttggaa      60
   tacaaagata ttccagttcc aaagccaaag gccaacgaat tgttgatcaa cgttaaatac     120
   tctggtgtct gtcacactga cttgcacgct tggcacggtg actggccatt gccagttaag     180
   ctaccattag tcggtggtca cgaaggtgcc ggtgtcgttg tcggcatggg tgaaaacgtt     240
   aagggctgga agatcggtga ctacgccggt atcaaatggt tgaacggttc ttgtatggcc     300
   tgtgaatact gtgaattggg taacgaatcc aactgtcctc acgctgactt gtctggttac     360
   acccacgacg gttctttcca acaatacgct accgctgacg ctgttcaagc cgctcacatt     420
   cctcaaggta ccgacttggc ccaagtcgcc cccatcttgt gtgctggtat caccgtctac     480
   aaggctttga agtctgctaa cttgatggcc ggtcactggg ttgctatctc cggtgctgct     540
   ggtggtctag gttctttggc tgttcaatac gccaaggcta tgggttacag agtcttgggt     600
   attgacggtg gtgaaggtaa ggaagaatta ttcagatcca tcggtggtga agtcttcatt     660
   gacttcacta aggaaaagga cattgtcggt gctgttctaa aggccactga cggtggtgct     720
   cacggtgtca tcaacgtttc cgtttccgaa gccgctattg aagcttctac cagatacgtt     780
   agagctaacg gtaccaccgt tttggtcggt atgccagctg gtgccaagtg ttgttctgat     840
   gtcttcaacc aagtcgtcaa gtccatctct attgttggtt cttacgtcgg taacagagct     900
   gacaccagag aagctttgga cttcttcgcc agaggtttgg tcaagtctcc aatcaaggtt     960
   gtcggcttgt ctaccttgcc agaaatttac gaaaagatgg aaaagggtca aatcgttggt    1020
   agatacgttg ttgacacttc taaataa                                        1047
<210> 24
   <211> 348
   <212> PRT
   <213> Saccharomyces cerevisiae
<400> 24
   Met Ser Ile Pro Glu Thr Gln Lys Gly Val Ile Phe Tyr Glu Ser His
   1 5 10 15
   Gly Lys Leu Glu Tyr Lys Asp Ile Pro Val Pro Lys Pro Lys Ala Asn
   20 25 30
   Glu Leu Leu Ile Asn Val Lys Tyr Ser Gly Val Cys His Thr Asp Leu
   35 40 45
   His Ala Trp His Gly Asp Trp Pro Leu Pro Val Lys Leu Pro Leu Val
   50 55 60
   Gly Gly His Glu Gly Ala Gly Val Val Val Gly Met Gly Glu Asn Val
   65 70 75 80
   Lys Gly Trp Lys Ile Gly Asp Tyr Ala Gly Ile Lys Trp Leu Asn Gly
   85 90 95
   Ser Cys Met Ala Cys Glu Tyr Cys Glu Leu Gly Asn Glu Ser Asn Cys
   100 105 110
   Pro His Ala Asp Leu Ser Gly Tyr Thr His Asp Gly Ser Phe Gln Gln
   115 120 125
   Tyr Ala Thr Ala Asp Ala Val Gln Ala Ala His Ile Pro Gln Gly Thr
   130 135 140
   Asp Leu Ala Gln Val Ala Pro Ile Leu Cys Ala Gly Ile Thr Val Tyr
   145 150 155 160
   Lys Ala Leu Lys Ser Ala Asn Leu Met Ala Gly His Trp Val Ala Ile
   165 170 175
   Ser Gly Ala Ala Gly Gly Leu Gly Ser Leu Ala Val Gln Tyr Ala Lys
   180 185 190
   Ala Met Gly Tyr Arg Val Leu Gly Ile Asp Gly Gly Glu Gly Lys Glu
   195 200 205
   Glu Leu Phe Arg Ser Ile Gly Gly Glu Val Phe Ile Asp Phe Thr Lys
   210 215 220
   Glu Lys Asp Ile Val Gly Ala Val Leu Lys Ala Thr Asp Gly Gly Ala
   225 230 235 240
   His Gly Val Ile Asn Val Ser Val Ser Glu Ala Ala Ile Glu Ala Ser
   245 250 255
   Thr Arg Tyr Val Arg Ala Asn Gly Thr Thr Val Leu Val Gly Met Pro
   260 265 270
   Ala Gly Ala Lys Cys Cys Ser Asp Val Phe Asn Gln Val Val Lys Ser
           275                 280                 285
   Ile Ser Ile Val Gly Ser Tyr Val Gly Asn Arg Ala Asp Thr Arg Glu
       290                 295                 300
   Ala Leu Asp Phe Phe Ala Arg Gly Leu Val Lys Ser Pro Ile Lys Val
   305                 310                 315                 320
   Val Gly Leu Ser Thr Leu Pro Glu Ile Tyr Glu Lys Met Glu Lys Gly
                   325                 330                 335
   Gln Ile Val Gly Arg Tyr Val Val Asp Thr Ser Lys
               340                 345
<210> 25
   <211> 1047
   <212> DNA
   <213> Saccharomyces cerevisiae
<400> 25
   atgtctattc cagaaactca aaaagccatt atcttctacg aatccaacgg caagttggag      60
   cataaggata tcccagttcc aaagccaaag cccaacgaat tgttaatcaa cgtcaagtac     120
   tctggtgtct gccacaccga tttgcacgct tggcatggtg actggccatt gccaactaag     180
   ttaccattag ttggtggtca cgaaggtgcc ggtgtcgttg tcggcatggg tgaaaacgtt     240
   aagggctgga agatcggtga ctacgccggt atcaaatggt tgaacggttc ttgtatggcc     300
   tgtgaatact gtgaattggg taacgaatcc aactgtcctc acgctgactt gtctggttac     360
   acccacgacg gttctttcca agaatacgct accgctgacg ctgttcaagc cgctcacatt     420
   cctcaaggta ctgacttggc tgaagtcgcg ccaatcttgt gtgctggtat caccgtatac     480
   aaggctttga agtctgccaa cttgagagca ggccactggg cggccatttc tggtgctgct     540
   ggtggtctag gttctttggc tgttcaatat gctaaggcga tgggttacag agtcttaggt     600
   attgatggtg gtccaggaaa ggaagaattg tttacctcgc tcggtggtga agtattcatc     660
   gacttcacca aagagaagga cattgttagc gcagtcgtta aggctaccaa cggcggtgcc     720
   cacggtatca tcaatgtttc cgtttccgaa gccgctatcg aagcttctac cagatactgt     780
   agggcgaacg gtactgttgt cttggttggt ttgccagccg gtgcaaagtg ctcctctgat     840
   gtcttcaacc acgttgtcaa gtctatctcc attgtcggct cttacgtggg gaacagagct     900
   gataccagag aagccttaga tttctttgcc agaggtctag tcaagtctcc aataaaggta     960
   gttggcttat ccagtttacc agaaatttac gaaaagatgg agaagggcca aattgctggt    1020
   agatacgttg ttgacacttc taaataa                                       1047
<210> 26
   <211> 348
   <212> PRT
   <213> Saccharomyces cerevisiae
<400> 26
   Met Ser Ile Pro Glu Thr Gln Lys Ala Ile Ile Phe Tyr Glu Ser Asn
   1               5                   10                  15
   Gly Lys Leu Glu His Lys Asp Ile Pro Val Pro Lys Pro Lys Pro Asn
               20                  25                  30
   Glu Leu Leu Ile Asn Val Lys Tyr Ser Gly Val Cys His Thr Asp Leu
           35                  40                  45
   His Ala Trp His Gly Asp Trp Pro Leu Pro Thr Lys Leu Pro Leu Val
       50                  55                  60
   Gly Gly His Glu Gly Ala Gly Val Val Val Gly Met Gly Glu Asn Val
   65                  70                  75                  80
   Lys Gly Trp Lys Ile Gly Asp Tyr Ala Gly Ile Lys Trp Leu Asn Gly
                   85                  90                  95
   Ser Cys Met Ala Cys Glu Tyr Cys Glu Leu Gly Asn Glu Ser Asn Cys
               100                 105                 110
   Pro His Ala Asp Leu Ser Gly Tyr Thr His Asp Gly Ser Phe Gln Glu
           115                 120                 125
   Tyr Ala Thr Ala Asp Ala Val Gln Ala Ala His Ile Pro Gln Gly Thr
       130                 135                 140
   Asp Leu Ala Glu Val Ala Pro Ile Leu Cys Ala Gly Ile Thr Val Tyr
   145                 150                 155                 160
   Lys Ala Leu Lys Ser Ala Asn Leu Arg Ala Gly His Trp Ala Ala Ile
                   165                 170                 175
   Ser Gly Ala Ala Gly Gly Leu Gly Ser Leu Ala Val Gln Tyr Ala Lys
               180                 185                 190
   Ala Met Gly Tyr Arg Val Leu Gly Ile Asp Gly Gly Pro Gly Lys Glu
           195                 200                 205
   Glu Leu Phe Thr Ser Leu Gly Gly Glu Val Phe Ile Asp Phe Thr Lys
       210                 215                 220
   Glu Lys Asp Ile Val Ser Ala Val Val Lys Ala Thr Asn Gly Gly Ala
   225                 230                 235                 240
   His Gly Ile Ile Asn Val Ser Val Ser Glu Ala Ala Ile Glu Ala Ser
                   245                 250                 255
   Thr Arg Tyr Cys Arg Ala Asn Gly Thr Val Val Leu Val Gly Leu Pro
               260                 265                 270
   Ala Gly Ala Lys Cys Ser Ser Asp Val Phe Asn His Val Val Lys Ser
           275                 280                 285
   Ile Ser Ile Val Gly Ser Tyr Val Gly Asn Arg Ala Asp Thr Arg Glu
       290                 295                 300
   Ala Leu Asp Phe Phe Ala Arg Gly Leu Val Lys Ser Pro Ile Lys Val
   305                 310                 315                 320
   Val Gly Leu Ser Ser Leu Pro Glu Ile Tyr Glu Lys Met Glu Lys Gly
                   325                 330                 335
   Gln Ile Ala Gly Arg Tyr Val Val Asp Thr Ser Lys
               340                 345
<210> 27
   <211> 1128
   <212> DNA
   <213> Saccharomyces cerevisiae
<400> 27
   atgttgagaa cgtcaacatt gttcaccagg cgtgtccaac caagcctatt ttctagaaac      60
   attcttagat tgcaatccac agctgcaatc cctaagactc aaaaaggtgt catcttttat     120
   gagaataagg ggaagctgca ttacaaagat atccctgtcc ccgagcctaa gccaaatgaa     180
   attttaatca acgttaaata ttctggtgta tgtcacaccg atttacatgc ttggcacggc     240
   gattggccat tacctgttaa actaccatta gtaggtggtc atgaaggtgc tggtgtagtt     300
   gtcaaactag gttccaatgt caagggctgg aaagtcggtg atttagcagg tatcaaatgg     360
   ctgaacggtt cttgtatgac atgcgaattc tgtgaatcag gtcatgaatc aaattgtcca     420
   gatgctgatt tatctggtta cactcatgat ggttctttcc aacaatttgc gaccgctgat     480
   gctattcaag ccgccaaaat tcaacagggt accgacttgg ccgaagtagc cccaatatta     540
   tgtgctggtg ttactgtata taaagcacta aaagaggcag acttgaaagc tggtgactgg     600
   gttgccatct ctggtgctgc aggtggcttg ggttccttgg ccgttcaata tgcaactgcg     660
   atgggttaca gagttctagg tattgatgca ggtgaggaaa aggaaaaact tttcaagaaa     720
   ttggggggtg aagtattcat cgactttact aaaacaaaga atatggtttc tgacattcaa     780
   gaagctacca aaggtggccc tcatggtgtc attaacgttt ccgtttctga agccgctatt     840
   tctctatcta cggaatatgt tagaccatgt ggtaccgtcg ttttggttgg tttgcccgct     900
   aacgcctacg ttaaatcaga ggtattctct catgtggtga agtccatcaa tatcaagggt     960
   tcttatgttg gtaacagagc tgatacgaga gaagccttag acttctttag cagaggtttg    1020
   atcaaatcac caatcaaaat tgttggatta tctgaattac caaaggttta tgacttgatg    1080
   gaaaagggca agattttggg tagatacgtc gtcgatacta gtaaataa                1128
<210> 28
   <211> 375
   <212> PRT
   <213> saccharomyces cerevisiae
<400> 28
<210> 29
   <211> 1149
   <212> DNA
   <213> Saccharomyces cerevisiae
<400> 29
   atgtcttccg ttactgggtt ttacattcca ccaatctctt tctttggtga aggtgcttta 60
   gaagaaaccg ctgattacat caaaaacaag gattacaaaa aggctttgat cgttactgat 120
   cctggtattg cagctattgg tctctccggt agagtccaaa agatgttgga agaacgtgac 180
   ttaaacgttg ctatctatga caaaactcaa ccaaacccaa atattgccaa tgtcacagct 240
   ggtttgaagg ttttgaagga acaaaactct gaaattgttg tttccattgg tggtggttct 300
   gctcacgaca atgctaaggc cattgcttta ttggctacta acggtgggga aatcggagac 360
   tatgaaggtg tcaatcaatc taagaaggct gctttaccac tatttgccat caacactact 420
   gctggtactg cttccgaaat gaccagattc actattatct ctaatgaaga aaagaaaatc 480
   aagatggcta tcattgacaa caacgtcact ccagctgttg ctgtcaacga tccatctacc 540
   atgtttggtt tgccacctgc tttgactgct gctactggtc tagatgcttt gactcactgt 600
   atcgaagctt atgtttccac cgcctctaac ccaatcaccg atgcctgtgc tttgaagggt 660
   attgatttga tcaatgaaag cttagtcgct gcatacaaag acggtaaaga caagaaggcc 720
   agaactgaca tgtgttacgc tgaatacttg gcaggtatgg ctttcaacaa tgcttctcta 780
   ggttatgttc atgcccttgc tcatcaactt ggtggtttct accacttgcc tcatggtgtt 840
   tgtaacgctg tcttgttgcc tcatgttcaa gaggccaaca tgcaatgtcc aaaggccaag 900
   aagagattag gtgaaattgc tttgcatttc ggtgcttctc aagaagatcc agaagaaacc 960
   atcaaggctt tgcacgtttt aaacagaacc atgaacattc caagaaactt gaaagaatta 1020
   ggtgttaaaa ccgaagattt tgaaattttg gctgaacacg ccatgcatga tgcctgccat 1080
   ttgactaacc cagttcaatt caccaaagaa caagtggttg ccattatcaa gaaagcctat 1140
   gaatattaa 1149
<210> 30
   <211> 382
   <212> PRT
   <213> Saccharomyces cerevisiae
<400> 30
   Met Ser Ser Val Thr Gly Phe Tyr Ile Pro Pro Ile Ser Phe Phe Gly
   1 5 10 15
   Glu Gly Ala Leu Glu Glu Thr Ala Asp Tyr Ile Lys Asn Lys Asp Tyr
   20 25 30
   Lys Lys Ala Leu Ile Val Thr Asp Pro Gly Ile Ala Ala Ile Gly Leu
   35 40 45
   Ser Gly Arg Val Gln Lys Met Leu Glu Glu Arg Asp Leu Asn Val Ala
   50 55 60
   Ile Tyr Asp Lys Thr Gln Pro Asn Pro Asn Ile Ala Asn Val Thr Ala
   65 70 75 80
   Gly Leu Lys Val Leu Lys Glu Gln Asn Ser Glu Ile Val Val Ser Ile
   85 90 95
   Gly Gly Gly Ser Ala His Asp Asn Ala Lys Ala Ile Ala Leu Leu Ala
   100 105 110
   Thr Asn Gly Gly Glu Ile Gly Asp Tyr Glu Gly Val Asn Gln Ser Lys
   115 120 125
   Lys Ala Ala Leu Pro Leu Phe Ala Ile Asn Thr Thr Ala Gly Thr Ala
   130 135 140
   Ser Glu Met Thr Arg Phe Thr Ile Ile Ser Asn Glu Glu Lys Lys Ile
   145 150 155 160
   Lys Met Ala Ile Ile Asp Asn Asn Val Thr Pro Ala Val Ala Val Asn
   165 170 175
   Asp Pro Ser Thr Met Phe Gly Leu Pro Pro Ala Leu Thr Ala Ala Thr
   180 185 190
   Gly Leu Asp Ala Leu Thr His Cys Ile Glu Ala Tyr Val Ser Thr Ala
   195 200 205
   Ser Asn Pro Ile Thr Asp Ala Cys Ala Leu Lys Gly Ile Asp Leu Ile
   210 215 220
   Asn Glu Ser Leu Val Ala Ala Tyr Lys Asp Gly Lys Asp Lys Lys Ala
   225 230 235 240
   Arg Thr Asp Met Cys Tyr Ala Glu Tyr Leu Ala Gly Met Ala Phe Asn
   245 250 255
   Asn Ala Ser Leu Gly Tyr Val His Ala Leu Ala His Gln Leu Gly Gly
   260 265 270
   Phe Tyr His Leu Pro His Gly Val Cys Asn Ala Val Leu Leu Pro His
   275 280 285
   Val Gln Glu Ala Asn Met Gln Cys Pro Lys Ala Lys Lys Arg Leu Gly
   290 295 300
   Glu Ile Ala Leu His Phe Gly Ala Ser Gln Glu Asp Pro Glu Glu Thr
   305 310 315 320
   Ile Lys Ala Leu His Val Leu Asn Arg Thr Met Asn Ile Pro Arg Asn
   325 330 335
   Leu Lys Glu Leu Gly Val Lys Thr Glu Asp Phe Glu Ile Leu Ala Glu
   340 345 350
   His Ala Met His Asp Ala Cys His Leu Thr Asn Pro Val Gln Phe Thr
   355 360 365
   Lys Glu Gln Val Val Ala Ile Ile Lys Lys Ala Tyr Glu Tyr
   370 375 380
<210> 31
   <211> 1056
   <212> DNA
   <213> Saccharomyces cerevisiae
<400> 31
   atgccttcgc aagtcattcc tgaaaaacaa aaggctattg tcttttatga gacagatgga 60
   aaattggaat ataaagacgt cacagttccg gaacctaagc ctaacgaaat tttagtccac 120
   gttaaatatt ctggtgtttg tcatagtgac ttgcacgcgt ggcacggtga ttggccattt 180
   caattgaaat ttccattaat cggtggtcac gaaggtgctg gtgttgttgt taagttggga 240
   tctaacgtta agggctggaa agtcggtgat tttgcaggta taaaatggtt gaatgggact 300
   tgcatgtcct gtgaatattg tgaagtaggt aatgaatctc aatgtcctta tttggatggt 360
   actggcttca cacatgatgg tacttttcaa gaatacgcaa ctgccgatgc cgttcaagct 420
   gcccatattc caccaaacgt caatcttgct gaagttgccc caatcttgtg tgcaggtatc 480
   actgtttata aggcgttgaa aagagccaat gtgataccag gccaatgggt cactatatcc 540
   ggtgcatgcg gtggcttggg ttctctggca atccaatacg cccttgctat gggttacagg 600
   gtcattggta tcgatggtgg taatgccaag cgaaagttat ttgaacaatt aggcggagaa 660
   atattcatcg atttcacgga agaaaaagac attgttggtg ctataataaa ggccactaat 720
   ggcggttctc atggagttat taatgtgtct gtttctgaag cagctatcga ggcttctacg 780
   aggtattgta ggcccaatgg tactgtcgtc ctggttggta tgccagctca tgcttactgc 840
   aattccgatg ttttcaatca agttgtaaaa tcaatctcca tcgttggatc ttgtgttgga 900
   aatagagctg atacaaggga ggctttagat ttcttcgcca gaggtttgat caaatctccg 960
   atccacttag ctggcctatc ggatgttcct gaaatttttg caaagatgga gaagggtgaa 1020
   attgttggta gatatgttgt tgagacttct aaatga 1056
<210> 32
   <211> 351
   <212> PRT
   <213> Saccharomyces cerevisiae
<400> 32
   Met Pro Ser Gln Val Ile Pro Glu Lys Gln Lys Ala Ile Val Phe Tyr
   1               5                   10                  15
   Glu Thr Asp Gly Lys Leu Glu Tyr Lys Asp Val Thr Val Pro Glu Pro
               20                  25                  30
   Lys Pro Asn Glu Ile Leu Val His Val Lys Tyr Ser Gly Val Cys His
           35                  40                  45
   Ser Asp Leu His Ala Trp His Gly Asp Trp Pro Phe Gln Leu Lys Phe
       50                  55                  60
   Pro Leu Ile Gly Gly His Glu Gly Ala Gly Val Val Val Lys Leu Gly
   65                  70                  75                  80
   Ser Asn Val Lys Gly Trp Lys Val Gly Asp Phe Ala Gly Ile Lys Trp
                   85                  90                  95
   Leu Asn Gly Thr Cys Met Ser Cys Glu Tyr Cys Glu Val Gly Asn Glu
               100                 105                 110
   Ser Gln Cys Pro Tyr Leu Asp Gly Thr Gly Phe Thr His Asp Gly Thr
           115                 120                 125
   Phe Gln Glu Tyr Ala Thr Ala Asp Ala Val Gln Ala Ala His Ile Pro
       130                 135                 140
   Pro Asn Val Asn Leu Ala Glu Val Ala Pro Ile Leu Cys Ala Gly Ile
   145                 150                 155                 160
   Thr Val Tyr Lys Ala Leu Lys Arg Ala Asn Val Ile Pro Gly Gln Trp
                   165                 170                 175
   Val Thr Ile Ser Gly Ala Cys Gly Gly Leu Gly Ser Leu Ala Ile Gln
               180                 185                 190
   Tyr Ala Leu Ala Met Gly Tyr Arg Val Ile Gly Ile Asp Gly Gly Asn
           195                 200                 205
   Ala Lys Arg Lys Leu Phe Glu Gln Leu Gly Gly Glu Ile Phe Ile Asp
       210                 215                 220
   Phe Thr Glu Glu Lys Asp Ile Val Gly Ala Ile Ile Lys Ala Thr Asn
   225                 230                 235                 240
   Gly Gly Ser His Gly Val Ile Asn Val Ser Val Ser Glu Ala Ala Ile
                   245                 250                 255
   Glu Ala Ser Thr Arg Tyr Cys Arg Pro Asn Gly Thr Val Val Leu Val
               260                 265                 270
   Gly Met Pro Ala His Ala Tyr Cys Asn Ser Asp Val Phe Asn Gln Val
           275                 280                 285
   Val Lys Ser Ile Ser Ile Val Gly Ser Cys Val Gly Asn Arg Ala Asp
       290                 295                 300
   Thr Arg Glu Ala Leu Asp Phe Phe Ala Arg Gly Leu Ile Lys Ser Pro
   305                 310                 315                 320
   Ile His Leu Ala Gly Leu Ser Asp Val Pro Glu Ile Phe Ala Lys Met
                   325                 330                 335
   Glu Lys Gly Glu Ile Val Gly Arg Tyr Val Val Glu Thr Ser Lys
               340                 345                 350
<210> 33
   <211> 1083
   <212> DNA
   <213> Saccharomyces cerevisiae
<400> 33
   atgtcttatc ctgagaaatt tgaaggtatc gctattcaat cacacgaaga ttggaaaaac 60
   ccaaagaaga caaagtatga cccaaaacca ttttacgatc atgacattga cattaagatc 120
   gaagcatgtg gtgtctgcgg tagtgatatt cattgtgcag ctggtcattg gggcaatatg 180
   aagatgccgc tagtcgttgg tcatgaaatc gttggtaaag ttgtcaagct agggcccaag 240
   tcaaacagtg ggttgaaagt cggtcaacgt gttggtgtag gtgctcaagt cttttcatgc 300
   ttggaatgtg accgttgtaa gaatgataat gaaccatact gcaccaagtt tgttaccaca 360
   tacagtcagc cttatgaaga cggctatgtg tcgcagggtg gctatgcaaa ctacgtcaga 420
   gttcatgaac attttgtggt gcctatccca gagaatattc catcacattt ggctgctcca 480
   ctattatgtg gtggtttgac tgtgtactct ccattggttc gtaacggttg cggtccaggt 540
   aaaaaagttg gtatagttgg tcttggtggt atcggcagta tgggtacatt gatttccaaa 600
   gccatggggg cagagacgta tgttatttct cgttcttcga gaaaaagaga agatgcaatg 660
   aagatgggcg ccgatcacta cattgctaca ttagaagaag gtgattgggg tgaaaagtac 720
   tttgacacct tcgacctgat tgtagtctgt gcttcctccc ttaccgacat tgacttcaac 780
   attatgccaa aggctatgaa ggttggtggt agaattgtct caatctctat accagaacaa 840
   cacgaaatgt tatcgctaaa gccatatggc ttaaaggctg tctccatttc ttacagtgct 900
   ttaggttcca tcaaagaatt gaaccaactc ttgaaattag tctctgaaaa agatatcaaa 960
   atttgggtgg aaacattacc tgttggtgaa gccggcgtcc atgaagcctt cgaaaggatg 1020
   gaaaagggtg acgttagata tagatttacc ttagtcggct acgacaaaga attttcagac 1080
   tag 1083
<210> 34
   <211> 360
   <212> PRT
   <213> Saccharomyces cerevisiae
<400> 34
   Met Ser Tyr Pro Glu Lys Phe Glu Gly Ile Ala Ile Gln Ser His Glu
   1 5 10 15
   Asp Trp Lys Asn Pro Lys Lys Thr Lys Tyr Asp Pro Lys Pro Phe Tyr
   20 25 30
   Asp His Asp Ile Asp Ile Lys Ile Glu Ala Cys Gly Val Cys Gly Ser
   35 40 45
   Asp Ile His Cys Ala Ala Gly His Trp Gly Asn Met Lys Met Pro Leu
   50 55 60
   Val Val Gly His Glu Ile Val Gly Lys Val Val Lys Leu Gly Pro Lys
   65 70 75 80
   Ser Asn Ser Gly Leu Lys Val Gly Gln Arg Val Gly Val Gly Ala Gln
   85 90 95
   Val Phe Ser Cys Leu Glu Cys Asp Arg Cys Lys Asn Asp Asn Glu Pro
   100 105 110
   Tyr Cys Thr Lys Phe Val Thr Thr Tyr Ser Gln Pro Tyr Glu Asp Gly
   115 120 125
   Tyr Val Ser Gln Gly Gly Tyr Ala Asn Tyr Val Arg Val His Glu His
               130                 135                 140
   Phe Val Val Pro Ile Pro Glu Asn Ile Pro Ser His Leu Ala Ala Pro
   145                 150                 155                 160
   Leu Leu Cys Gly Gly Leu Thr Val Tyr Ser Pro Leu Val Arg Asn Gly
                   165                 170                 175
   Cys Gly Pro Gly Lys Lys Val Gly Ile Val Gly Leu Gly Gly Ile Gly
               180                 185                 190
   Ser Met Gly Thr Leu Ile Ser Lys Ala Met Gly Ala Glu Thr Tyr Val
           195                 200                 205
   Ile Ser Arg Ser Ser Arg Lys Arg Glu Asp Ala Met Lys Met Gly Ala
       210                 215                 220
   Asp His Tyr Ile Ala Thr Leu Glu Glu Gly Asp Trp Gly Glu Lys Tyr
   225                 230                 235                 240
   Phe Asp Thr Phe Asp Leu Ile Val Val Cys Ala Ser Ser Leu Thr Asp
                   245                 250                 255
   Ile Asp Phe Asn Ile Met Pro Lys Ala Met Lys Val Gly Gly Arg Ile
               260                 265                 270
   Val Ser Ile Ser Ile Pro Glu Gln His Glu Met Leu Ser Leu Lys Pro
           275                 280                 285
   Tyr Gly Leu Lys Ala Val Ser Ile Ser Tyr Ser Ala Leu Gly Ser Ile
       290                 295                 300
   Lys Glu Leu Asn Gln Leu Leu Lys Leu Val Ser Glu Lys Asp Ile Lys
   305                 310                 315                 320
   Ile Trp Val Glu Thr Leu Pro Val Gly Glu Ala Gly Val His Glu Ala
                   325                 330                 335
   Phe Glu Arg Met Glu Lys Gly Asp Val Arg Tyr Arg Phe Thr Leu Val
               340                 345                 350
   Gly Tyr Asp Lys Glu Phe Ser Asp
           355                 360
<210> 35
   <211> 1086
   <212> DNA
   <213> Saccharomyces cerevisiae
<400> 35
   atgctttacc cagaaaaatt tcagggcatc ggtatttcca acgcaaagga ttggaagcat 60
   cctaaattag tgagttttga cccaaaaccc tttggcgatc atgacgttga tgttgaaatt 120
   gaagcctgtg gtatctgcgg atctgatttt catatagccg ttggtaattg gggtccagtc 180
   ccagaaaatc aaatccttgg acatgaaata attggccgcg tggtgaaggt tggatccaag 240
   tgccacactg gggtaaaaat cggtgaccgt gttggtgttg gtgcccaagc cttggcgtgt 300
   tttgagtgtg aacgttgcaa aagtgacaac gagcaatact gtaccaatga ccacgttttg 360
   actatgtgga ctccttacaa ggacggctac atttcacaag gaggctttgc ctcccacgtg 420
   aggcttcatg aacactttgc tattcaaata ccagaaaata ttccaagtcc gctagccgct 480
   ccattattgt gtggtggtat tacagttttc tctccactac taagaaatgg ctgtggtcca 540
   ggtaagaggg taggtattgt tggcatcggt ggtattgggc atatggggat tctgttggct 600
   aaagctatgg gagccgaggt ttatgcgttt tcgcgaggcc actccaagcg ggaggattct 660
   atgaaactcg gtgctgatca ctatattgct atgttggagg ataaaggctg gacagaacaa 720
   tactctaacg ctttggacct tcttgtcgtt tgctcatcat ctttgtcgaa agttaatttt 780
   gacagtatcg ttaagattat gaagattgga ggctccatcg tttcaattgc tgctcctgaa 840
   gttaatgaaa agcttgtttt aaaaccgttg ggcctaatgg gagtatcaat ctcaagcagt 900
   gctatcggat ctaggaagga aatcgaacaa ctattgaaat tagtttccga aaagaatgtc 960
   aaaatatggg tggaaaaact tccgatcagc gaagaaggcg tcagccatgc ctttacaagg 1020
   atggaaagcg gagacgtcaa atacagattt actttggtcg attatgataa gaaattccat 1080
   aaatag 1086
<210> 36
   <211> 361
   <212> PRT
   <213> Saccharomyces cerevisiae
<400> 36
   Met Leu Tyr Pro Glu Lys Phe Gln Gly Ile Gly Ile Ser Asn Ala Lys
   1               5                   10                  15
   Asp Trp Lys His Pro Lys Leu Val Ser Phe Asp Pro Lys Pro Phe Gly
               20                  25                  30
   Asp His Asp Val Asp Val Glu Ile Glu Ala Cys Gly Ile Cys Gly Ser
           35                  40                  45
   Asp Phe His Ile Ala Val Gly Asn Trp Gly Pro Val Pro Glu Asn Gln
       50                  55                  60
   Ile Leu Gly His Glu Ile Ile Gly Arg Val Val Lys Val Gly Ser Lys
   65                  70                  75                  80
   Cys His Thr Gly Val Lys Ile Gly Asp Arg Val Gly Val Gly Ala Gln
                   85                  90                  95
   Ala Leu Ala Cys Phe Glu Cys Glu Arg Cys Lys Ser Asp Asn Glu Gln
   100 105 110
   Tyr Cys Thr Asn Asp His Val Leu Thr Met Trp Thr Pro Tyr Lys Asp
   115 120 125
   Gly Tyr Ile Ser Gln Gly Gly Phe Ala Ser His Val Arg Leu His Glu
   130 135 140
   His Phe Ala Ile Gln Ile Pro Glu Asn Ile Pro Ser Pro Leu Ala Ala
   145 150 155 160
   Pro Leu Leu Cys Gly Gly Ile Thr Val Phe Ser Pro Leu Leu Arg Asn
   165 170 175
   Gly Cys Gly Pro Gly Lys Arg Val Gly Ile Val Gly Ile Gly Gly Ile
   180 185 190
   Gly His Met Gly Ile Leu Leu Ala Lys Ala Met Gly Ala Glu Val Tyr
   195 200 205
   Ala Phe Ser Arg Gly His Ser Lys Arg Glu Asp Ser Met Lys Leu Gly
   210 215 220
   Ala Asp His Tyr Ile Ala Met Leu Glu Asp Lys Gly Trp Thr Glu Gln
   225 230 235 240
   Tyr Ser Asn Ala Leu Asp Leu Leu Val Val Cys Ser Ser Ser Leu Ser
   245 250 255
   Lys Val Asn Phe Asp Ser Ile Val Lys Ile Met Lys Ile Gly Gly Ser
   260 265 270
   Ile Val Ser Ile Ala Ala Pro Glu Val Asn Glu Lys Leu Val Leu Lys
   275 280 285
   Pro Leu Gly Leu Met Gly Val Ser Ile Ser Ser Ser Ala Ile Gly Ser
   290 295 300
   Arg Lys Glu Ile Glu Gln Leu Leu Lys Leu Val Ser Glu Lys Asn Val
   305 310 315 320
   Lys Ile Trp Val Glu Lys Leu Pro Ile Ser Glu Glu Gly Val Ser His
   325 330 335
   Ala Phe Thr Arg Met Glu Ser Gly Asp Val Lys Tyr Arg Phe Thr Leu
   340 345 350
   Val Asp Tyr Asp Lys Lys Phe His Lys
   355 360
<210> 37
   <211> 1161
   <212> DNA
   <213> Saccharomyces cerevisiae
<400> 37
   atgtccgccg ctactgttgg taaacctatt aagtgcattg ctgctgttgc gtatgatgcg      60
   aagaaaccat taagtgttga agaaatcacg gtagacgccc caaaagcgca cgaagtacgt     120
   atcaaaattg aatatactgc tgtatgccac actgatgcgt acactttatc aggctctgat     180
   ccagaaggac ttttcccttg cgttctgggc cacgaaggag ccggtatcgt agaatctgta     240
   ggcgatgatg tcataacagt taagcctggt gatcatgtta ttgctttgta cactgctgag     300
   tgtggcaaat gtaagttctg tacttccggt aaaaccaact tatgtggtgc tgttagagct     360
   actcaaggga aaggtgtaat gcctgatggg accacaagat ttcataatgc gaaaggtgaa     420
   gatatatacc atttcatggg ttgctctact ttttccgaat atactgtggt ggcagatgtc     480
   tctgtggttg ccatcgatcc aaaagctccc ttggatgctg cctgtttact gggttgtggt     540
   gttactactg gttttggggc ggctcttaag acagctaatg tgcaaaaagg cgataccgtt     600
   gcagtatttg gctgcgggac tgtaggactc tccgttatcc aaggtgcaaa gttaaggggc     660
   gcttccaaga tcattgccat tgacattaac aataagaaaa aacaatattg ttctcaattt     720
   ggtgccacgg attttgttaa tcccaaggaa gatttggcca aagatcaaac tatcgttgaa     780
   aagttaattg aaatgactga tgggggtctg gattttactt ttgactgtac tggtaatacc     840
   aaaattatga gagatgcttt ggaagcctgt cataaaggtt ggggtcaatc tattatcatt     900
   ggtgtggctg ccgctggtga agaaatttct acaaggccgt tccagctggt cactggtaga     960
   gtgtggaaag gctctgcttt tggtggcatc aaaggtagat ctgaaatggg cggtttaatt    1020
   aaagactatc aaaaaggtgc cttaaaagtc gaagaattta tcactcacag gagaccattc    1080
   aaagaaatca atcaagcctt tgaagatttg cataacggtg attgcttaag aaccgtcttg    1140
   aagtctgatg aaataaaata g                                              1161
<210> 38
   <211> 386
   <212> PRT
   <213> Saccharomyces cerevisiae
<400> 38
   Met Ser Ala Ala Thr Val Gly Lys Pro Ile Lys Cys Ile Ala Ala Val
   1               5                   10                  15
   Ala Tyr Asp Ala Lys Lys Pro Leu Ser Val Glu Glu Ile Thr Val Asp
               20                  25                  30
   Ala Pro Lys Ala His Glu Val Arg Ile Lys Ile Glu Tyr Thr Ala Val
           35                  40                  45
   Cys His Thr Asp Ala Tyr Thr Leu Ser Gly Ser Asp Pro Glu Gly Leu
       50                  55                  60
   Phe Pro Cys Val Leu Gly His Glu Gly Ala Gly Ile Val Glu Ser Val
   65 70 75 80
   Gly Asp Asp Val Ile Thr Val Lys Pro Gly Asp His Val Ile Ala Leu
   85 90 95
   Tyr Thr Ala Glu Cys Gly Lys Cys Lys Phe Cys Thr Ser Gly Lys Thr
   100 105 110
   Asn Leu Cys Gly Ala Val Arg Ala Thr Gln Gly Lys Gly Val Met Pro
   115 120 125
   Asp Gly Thr Thr Arg Phe His Asn Ala Lys Gly Glu Asp Ile Tyr His
   130 135 140
   Phe Met Gly Cys Ser Thr Phe Ser Glu Tyr Thr Val Val Ala Asp Val
   145 150 155 160
   Ser Val Val Ala Ile Asp Pro Lys Ala Pro Leu Asp Ala Ala Cys Leu
   165 170 175
   Leu Gly Cys Gly Val Thr Thr Gly Phe Gly Ala Ala Leu Lys Thr Ala
   180 185 190
   Asn Val Gln Lys Gly Asp Thr Val Ala Val Phe Gly Cys Gly Thr Val
   195 200 205
   Gly Leu Ser Val Ile Gln Gly Ala Lys Leu Arg Gly Ala Ser Lys Ile
   210 215 220
   Ile Ala Ile Asp Ile Asn Asn Lys Lys Lys Gln Tyr Cys Ser Gln Phe
   225 230 235 240
   Gly Ala Thr Asp Phe Val Asn Pro Lys Glu Asp Leu Ala Lys Asp Gln
   245 250 255
   Thr Ile Val Glu Lys Leu Ile Glu Met Thr Asp Gly Gly Leu Asp Phe
   260 265 270
   Thr Phe Asp Cys Thr Gly Asn Thr Lys Ile Met Arg Asp Ala Leu Glu
   275 280 285
   Ala Cys His Lys Gly Trp Gly Gln Ser Ile Ile Ile Gly Val Ala Ala
   290 295 300
   Ala Gly Glu Glu Ile Ser Thr Arg Pro Phe Gln Leu Val Thr Gly Arg
   305 310 315 320
   Val Trp Lys Gly Ser Ala Phe Gly Gly Ile Lys Gly Arg Ser Glu Met
   325 330 335
   Gly Gly Leu Ile Lys Asp Tyr Gln Lys Gly Ala Leu Lys Val Glu Glu
   340 345 350
   Phe Ile Thr His Arg Arg Pro Phe Lys Glu Ile Asn Gln Ala Phe Glu
   355 360 365
   Asp Leu His Asn Gly Asp Cys Leu Arg Thr Val Leu Lys Ser Asp Glu
   370 375 380
   Ile Lys
   385
<210> 39
   <211> 939
   <212> DNA
   <213> Saccharomyces cerevisiae
<400> 39
   atgcctgcta cgttaaagaa ttcttctgct acattaaaac taaatactgg tgcctccatt 60
   ccagtgttgg gtttcggcac ttggcgttcc gttgacaata acggttacca ttctgtaatt 120
   gcagctttga aagctggata cagacacatt gatgctgcgg ctatctattt gaatgaagaa 180
   gaagttggca gggctattaa agattccgga gtccctcgtg aggaaatttt tattactact 240
   aagctttggg gtacggaaca acgtgatccg gaagctgctc taaacaagtc tttgaaaaga 300
   ctaggcttgg attatgttga cctatatctg atgcattggc cagtgccttt gaaaaccgac 360
   agagttactg atggtaacgt tctgtgcatt ccaacattag aagatggcac tgttgacatc 420
   gatactaagg aatggaattt tatcaagacg tgggagttga tgcaagagtt gccaaagacg 480
   ggcaaaacta aagccgttgg tgtctctaat ttttctatta acaacattaa agaattatta 540
   gaatctccaa ataacaaggt ggtaccagct actaatcaaa ttgaaattca tccattgcta 600
   ccacaagacg aattgattgc cttttgtaag gaaaagggta ttgttgttga agcctactca 660
   ccatttggga gtgctaatgc tcctttacta aaagagcaag caattattga tatggctaaa 720
   aagcacggcg ttgagccagc acagcttatt atcagttgga gtattcaaag aggctacgtt 780
   gttctggcca aatcggttaa tcctgaaaga attgtatcca attttaagat tttcactctg 840
   cctgaggatg atttcaagac tattagtaac ctatccaaag tgcatggtac aaagagagtc 900
   gttgatatga agtggggatc cttcccaatt ttccaatga 939
<210> 40
   <211> 312
   <212> PRT
   <213> Saccharomyces cerevisiae
<400> 40
   Met Pro Ala Thr Leu Lys Asn Ser Ser Ala Thr Leu Lys Leu Asn Thr
   1 5 10 15
   Gly Ala Ser Ile Pro Val Leu Gly Phe Gly Thr Trp Arg Ser Val Asp
                   20                  25                  30
   Asn Asn Gly Tyr His Ser Val Ile Ala Ala Leu Lys Ala Gly Tyr Arg
           35                  40                  45
   His Ile Asp Ala Ala Ala Ile Tyr Leu Asn Glu Glu Glu Val Gly Arg
       50                  55                  60
   Ala Ile Lys Asp Ser Gly Val Pro Arg Glu Glu Ile Phe Ile Thr Thr
   65                  70                  75                  80
   Lys Leu Trp Gly Thr Glu Gln Arg Asp Pro Glu Ala Ala Leu Asn Lys
                   85                  90                  95
   Ser Leu Lys Arg Leu Gly Leu Asp Tyr Val Asp Leu Tyr Leu Met His
               100                 105                 110
   Trp Pro Val Pro Leu Lys Thr Asp Arg Val Thr Asp Gly Asn Val Leu
           115                 120                 125
   Cys Ile Pro Thr Leu Glu Asp Gly Thr Val Asp Ile Asp Thr Lys Glu
       130                 135                 140
   Trp Asn Phe Ile Lys Thr Trp Glu Leu Met Gln Glu Leu Pro Lys Thr
   145                 150                 155                 160
   Gly Lys Thr Lys Ala Val Gly Val Ser Asn Phe Ser Ile Asn Asn Ile
                   165                 170                 175
   Lys Glu Leu Leu Glu Ser Pro Asn Asn Lys Val Val Pro Ala Thr Asn
               180                 185                 190
   Gln Ile Glu Ile His Pro Leu Leu Pro Gln Asp Glu Leu Ile Ala Phe
           195                 200                 205
   Cys Lys Glu Lys Gly Ile Val Val Glu Ala Tyr Ser Pro Phe Gly Ser
       210                 215                 220
   Ala Asn Ala Pro Leu Leu Lys Glu Gln Ala Ile Ile Asp Met Ala Lys
   225                 230                 235                 240
   Lys His Gly Val Glu Pro Ala Gln Leu Ile Ile Ser Trp Ser Ile Gln
                   245                 250                 255
   Arg Gly Tyr Val Val Leu Ala Lys Ser Val Asn Pro Glu Arg Ile Val
               260                 265                 270
   Ser Asn Phe Lys Ile Phe Thr Leu Pro Glu Asp Asp Phe Lys Thr Ile
           275                 280                 285
   Ser Asn Leu Ser Lys Val His Gly Thr Lys Arg Val Val Asp Met Lys
   290 295 300
   Trp Gly Ser Phe Pro Ile Phe Gln
   305 310
<210> 41
   <211> 999
   <212> DNA
   <213> Saccharomyces cerevisiae
<400> 41
   atgatcagaa ttgctattaa cggtttcggt agaatcggta gattggtctt gagattggct      60
   ttgcaaagaa aagacattga ggttgttgct gtcaacgatc catttatctc taacgattat     120
   gctgcttaca tggtcaagta cgattctact catggtagat acaagggtac tgtttcccat     180
   gacgacaagc acatcatcat tgatggtgtc aagatcgcta cctaccaaga aagagaccca     240
   gctaacttgc catggggttc tctaaagatc gatgtcgctg ttgactccac tggtgttttc     300
   aaggaattgg acaccgctca aaagcacatt gacgctggtg ccaagaaggt tgtcatcact     360
   gctccatctt cttctgctcc aatgtttgtt gttggtgtta accacactaa atacactcca     420
   gacaagaaga ttgtctccaa cgcttcttgt accaccaact gtttggctcc attggccaag     480
   gttatcaacg atgctttcgg tattgaagaa ggtttgatga ccactgttca ctccatgacc     540
   gccactcaaa agactgttga tggtccatcc cacaaggact ggagaggtgg tagaaccgct     600
   tccggtaaca ttatcccatc ctctaccggt gctgctaagg ctgtcggtaa ggtcttgcca     660
   gaattgcaag gtaagttgac cggtatggct ttcagagtcc caaccgtcga tgtttccgtt     720
   gttgacttga ctgtcaagtt ggaaaaggaa gctacttacg accaaatcaa gaaggctgtt     780
   aaggctgccg ctgaaggtcc aatgaagggt gttttgggtt acaccgaaga tgccgttgtc     840
   tcctctgatt tcttgggtga cactcacgct tccatcttcg atgcctccgc tggtatccaa     900
   ttgtctccaa agttcgtcaa gttgatttcc tggtacgata acgaatacgg ttactccgcc     960
   agagttgttg acttgatcga atatgttgcc aaggcttaa                            999
<210> 42
   <211> 332
   <212> PRT
   <213> Saccharomyces cerevisiae
<400> 42
   Met Ile Arg Ile Ala Ile Asn Gly Phe Gly Arg Ile Gly Arg Leu Val
   1 5 10 15
   Leu Arg Leu Ala Leu Gln Arg Lys Asp Ile Glu Val Val Ala Val Asn
   20 25 30
   Asp Pro Phe Ile Ser Asn Asp Tyr Ala Ala Tyr Met Val Lys Tyr Asp
   35 40 45
   Ser Thr His Gly Arg Tyr Lys Gly Thr Val Ser His Asp Asp Lys His
   50 55 60
   Ile Ile Ile Asp Gly Val Lys Ile Ala Thr Tyr Gln Glu Arg Asp Pro
   65 70 75 80
   Ala Asn Leu Pro Trp Gly Ser Leu Lys Ile Asp Val Ala Val Asp Ser
   85 90 95
   Thr Gly Val Phe Lys Glu Leu Asp Thr Ala Gln Lys His Ile Asp Ala
   100 105 110
   Gly Ala Lys Lys Val Val Ile Thr Ala Pro Ser Ser Ser Ala Pro Met
   115 120 125
   Phe Val Val Gly Val Asn His Thr Lys Tyr Thr Pro Asp Lys Lys Ile
   130 135 140
   Val Ser Asn Ala Ser Cys Thr Thr Asn Cys Leu Ala Pro Leu Ala Lys
   145 150 155 160
   Val Ile Asn Asp Ala Phe Gly Ile Glu Glu Gly Leu Met Thr Thr Val
   165 170 175
   His Ser Met Thr Ala Thr Gln Lys Thr Val Asp Gly Pro Ser His Lys
   180 185 190
   Asp Trp Arg Gly Gly Arg Thr Ala Ser Gly Asn Ile Ile Pro Ser Ser
   195 200 205
   Thr Gly Ala Ala Lys Ala Val Gly Lys Val Leu Pro Glu Leu Gln Gly
   210 215 220
   Lys Leu Thr Gly Met Ala Phe Arg Val Pro Thr Val Asp Val Ser Val
   225 230 235 240
   Val Asp Leu Thr Val Lys Leu Glu Lys Glu Ala Thr Tyr Asp Gln Ile
   245 250 255
   Lys Lys Ala Val Lys Ala Ala Ala Glu Gly Pro Met Lys Gly Val Leu
   260 265 270
   Gly Tyr Thr Glu Asp Ala Val Val Ser Ser Asp Phe Leu Gly Asp Thr
   275 280 285
   His Ala Ser Ile Phe Asp Ala Ser Ala Gly Ile Gln Leu Ser Pro Lys
   290 295 300
   Phe Val Lys Leu Ile Ser Trp Tyr Asp Asn Glu Tyr Gly Tyr Ser Ala
   305 310 315 320
   Arg Val Val Asp Leu Ile Glu Tyr Val Ala Lys Ala
   325 330
<210> 43
   <211> 999
   <212> DNA
   <213> Saccharomyces cerevisiae
<400> 43
   atggttagag ttgctattaa cggtttcggt agaatcggta gattggttat gagaattgct   60
   ttgcaaagaa agaacgtcga agttgttgct ttgaacgatc ctttcatctc taacgactac  120
   tccgcttaca tgttcaagta cgactctact cacggtagat acgctggtga agtttcccac  180
   gatgacaagc acatcatcgt tgatggtcac aagatcgcca ctttccaaga aagagaccca  240
   gctaacttgc catgggcttc tctaaacatt gacatcgcca ttgactccac tggtgttttc  300
   aaggaattgg acactgctca aaagcacatt gacgctggtg ccaagaaggt tgtcatcact  360
   gctccatctt ccaccgcccc aatgttcgtc atgggtgtta acgaagaaaa atacacttct  420
   gacttgaaga ttgtttccaa cgcttcttgt accaccaact gtttggctcc attggccaag  480
   gttatcaacg atgctttcgg tattgaagaa ggtttgatga ccactgttca ctccatgacc  540
   gccacccaaa agactgttga cggtccatcc cacaaggact ggagaggtgg tagaaccgct  600
   tccggtaaca tcatcccatc ctctaccggt gctgctaagg ctgtcggtaa ggtcttgcca  660
   gaattgcaag gtaagttgac cggtatggct ttcagagtcc caaccgtcga tgtttccgtt  720
   gttgacttga ctgtcaagtt gaacaaggaa accacctacg atgaaatcaa gaaggttgtc  780
   aaggctgccg ctgaaggtaa gttgaagggt gtcttgggtt acactgaaga cgctgttgtc  840
   tcctctgact tcttgggtga ctctaactct tccatcttcg atgctgccgc tggtatccaa  900
   ttgtctccaa agttcgtcaa gttggtttcc tggtacgaca acgaatacgg ttactctacc  960
   agagttgtcg acttggttga acacgttgcc aaggcttaa                         999
<210> 44
   <211> 332
   <212> PRT
   <213> Saccharomyces cerevisiae
<400> 44
   Met Val Arg Val Ala Ile Asn Gly Phe Gly Arg Ile Gly Arg Leu Val
   1               5                   10                  15
   Met Arg Ile Ala Leu Gln Arg Lys Asn Val Glu Val Val Ala Leu Asn
               20                  25                  30
   Asp Pro Phe Ile Ser Asn Asp Tyr Ser Ala Tyr Met Phe Lys Tyr Asp
           35                  40                  45
   Ser Thr His Gly Arg Tyr Ala Gly Glu Val Ser His Asp Asp Lys His
       50                  55                  60
   Ile Ile Val Asp Gly His Lys Ile Ala Thr Phe Gln Glu Arg Asp Pro
   65                  70                  75                  80
   Ala Asn Leu Pro Trp Ala Ser Leu Asn Ile Asp Ile Ala Ile Asp Ser
                   85                  90                  95
   Thr Gly Val Phe Lys Glu Leu Asp Thr Ala Gln Lys His Ile Asp Ala
               100                 105                 110
   Gly Ala Lys Lys Val Val Ile Thr Ala Pro Ser Ser Thr Ala Pro Met
           115                 120                 125
   Phe Val Met Gly Val Asn Glu Glu Lys Tyr Thr Ser Asp Leu Lys Ile
       130                 135                 140
   Val Ser Asn Ala Ser Cys Thr Thr Asn Cys Leu Ala Pro Leu Ala Lys
   145                 150                 155                 160
   Val Ile Asn Asp Ala Phe Gly Ile Glu Glu Gly Leu Met Thr Thr Val
                   165                 170                 175
   His Ser Met Thr Ala Thr Gln Lys Thr Val Asp Gly Pro Ser His Lys
               180                 185                 190
   Asp Trp Arg Gly Gly Arg Thr Ala Ser Gly Asn Ile Ile Pro Ser Ser
           195                 200                 205
   Thr Gly Ala Ala Lys Ala Val Gly Lys Val Leu Pro Glu Leu Gln Gly
       210                 215                 220
   Lys Leu Thr Gly Met Ala Phe Arg Val Pro Thr Val Asp Val Ser Val
   225                 230                 235                 240
   Val Asp Leu Thr Val Lys Leu Asn Lys Glu Thr Thr Tyr Asp Glu Ile
                   245                 250                 255
   Lys Lys Val Val Lys Ala Ala Ala Glu Gly Lys Leu Lys Gly Val Leu
               260                 265                 270
   Gly Tyr Thr Glu Asp Ala Val Val Ser Ser Asp Phe Leu Gly Asp Ser
           275                 280                 285
   Asn Ser Ser Ile Phe Asp Ala Ala Ala Gly Ile Gln Leu Ser Pro Lys
       290                 295                 300
   Phe Val Lys Leu Val Ser Trp Tyr Asp Asn Glu Tyr Gly Tyr Ser Thr
   305                 310                 315                 320
   Arg Val Val Asp Leu Val Glu His Val Ala Lys Ala
                   325                 330
<210> 45
   <211> 999
   <212> DNA
   <213> Saccharomyces cerevisiae
<400> 45
   atggttagag ttgctattaa cggtttcggt agaatcggta gattggtcat gagaattgct      60
   ttgtctagac caaacgtcga agttgttgct ttgaacgacc cattcatcac caacgactac     120
   gctgcttaca tgttcaagta cgactccact cacggtagat acgctggtga agtttcccac     180
   gatgacaagc acatcattgt cgatggtaag aagattgcta cttaccaaga aagagaccca     240
   gctaacttgc catggggttc ttccaacgtt gacatcgcca ttgactccac tggtgttttc     300
   aaggaattag acactgctca aaagcacatt gacgctggtg ccaagaaggt tgttatcact     360
   gctccatctt ccaccgcccc aatgttcgtc atgggtgtta acgaagaaaa atacacttct     420
   gacttgaaga ttgtttccaa cgcttcttgt accaccaact gtttggctcc attggccaag     480
   gttatcaacg atgctttcgg tattgaagaa ggtttgatga ccactgtcca ctctttgact     540
   gctactcaaa agactgttga cggtccatcc cacaaggact ggagaggtgg tagaaccgct     600
   tccggtaaca tcatcccatc ctccaccggt gctgctaagg ctgtcggtaa ggtcttgcca     660
   gaattgcaag gtaagttgac cggtatggct ttcagagtcc caaccgtcga tgtctccgtt     720
   gttgacttga ctgtcaagtt gaacaaggaa accacctacg atgaaatcaa gaaggttgtt     780
   aaggctgccg ctgaaggtaa gttgaagggt gttttgggtt acaccgaaga cgctgttgtc     840
   tcctctgact tcttgggtga ctctcactct tccatcttcg atgcttccgc tggtatccaa     900
   ttgtctccaa agttcgtcaa gttggtctcc tggtacgaca acgaatacgg ttactctacc     960
   agagttgtcg acttggttga acacgttgcc aaggcttaa                           999
<210> 46
   <211> 332
   <212> PRT
   <213> Saccharomyces cerevisiae
<400> 46
   Met Val Arg Val Ala Ile Asn Gly Phe Gly Arg Ile Gly Arg Leu Val
   1               5                   10                  15
   Met Arg Ile Ala Leu Ser Arg Pro Asn Val Glu Val Val Ala Leu Asn
               20                  25                  30
   Asp Pro Phe Ile Thr Asn Asp Tyr Ala Ala Tyr Met Phe Lys Tyr Asp
           35                  40                  45
   Ser Thr His Gly Arg Tyr Ala Gly Glu Val Ser His Asp Asp Lys His
       50                  55                  60
   Ile Ile Val Asp Gly Lys Lys Ile Ala Thr Tyr Gln Glu Arg Asp Pro
   65                  70                  75                  80
   Ala Asn Leu Pro Trp Gly Ser Ser Asn Val Asp Ile Ala Ile Asp Ser
   85 90 95
   Thr Gly Val Phe Lys Glu Leu Asp Thr Ala Gln Lys His Ile Asp Ala
   100 105 110
   Gly Ala Lys Lys Val Val Ile Thr Ala Pro Ser Ser Thr Ala Pro Met
   115 120 125
   Phe Val Met Gly Val Asn Glu Glu Lys Tyr Thr Ser Asp Leu Lys Ile
   130 135 140
   Val Ser Asn Ala Ser Cys Thr Thr Asn Cys Leu Ala Pro Leu Ala Lys
   145 150 155 160
   Val Ile Asn Asp Ala Phe Gly Ile Glu Glu Gly Leu Met Thr Thr Val
   165 170 175
   His Ser Leu Thr Ala Thr Gln Lys Thr Val Asp Gly Pro Ser His Lys
   180 185 190
   Asp Trp Arg Gly Gly Arg Thr Ala Ser Gly Asn Ile Ile Pro Ser Ser
   195 200 205
   Thr Gly Ala Ala Lys Ala Val Gly Lys Val Leu Pro Glu Leu Gln Gly
   210 215 220
   Lys Leu Thr Gly Met Ala Phe Arg Val Pro Thr Val Asp Val Ser Val
   225 230 235 240
   Val Asp Leu Thr Val Lys Leu Asn Lys Glu Thr Thr Tyr Asp Glu Ile
   245 250 255
   Lys Lys Val Val Lys Ala Ala Ala Glu Gly Lys Leu Lys Gly Val Leu
   260 265 270
   Gly Tyr Thr Glu Asp Ala Val Val Ser Ser Asp Phe Leu Gly Asp Ser
   275 280 285
   His Ser Ser Ile Phe Asp Ala Ser Ala Gly Ile Gln Leu Ser Pro Lys
   290 295 300
   Phe Val Lys Leu Val Ser Trp Tyr Asp Asn Glu Tyr Gly Tyr Ser Thr
   305 310 315 320
   Arg Val Val Asp Leu Val Glu His Val Ala Lys Ala
   325 330
<210> 47
   <211> 1071
   <212> DNA
   <213> Kluyveromyces lactis
<400> 47
   atgcccgata tgacaaacga atcttcttct aagccagctc aaattaacat tggtatcaat      60
   ggttttggta gaatcggtag attggttcta cgtgctgctt tgacgcaccc agaagttaag     120
   gtcagattaa tcaataatcc atccacaaca ccagaatacg ctgcttattt gttcaaatac     180
   gattctactc acggcaagta tcgtggtgaa gttgaattcg acgatgaacg tatcatcatt     240
   caaaatgacc atgtttcggc tcatatccct ctatctcatt ttagggaacc agagcgtatc     300
   ccatgggctt cctacaacgt cgattatgta attgactcaa ccggtgtctt caaggaagtc     360
   gatacagcct ctagacataa aggtgtcaaa aaagttatca ttactgctcc atcaaagacc     420
   gcgccaatgt acgtctatgg tgttaaccac gttaaataca acccattgac ggatcacgtg     480
   gtctctaatg cctcctgtac taccaactgt ttggctccgt tggttaaggc tttggacgat     540
   gagttcggta tcgaagaagc cttgatgaca actattcatg caactactgc ttctcaaaag     600
   actgtcgatg gtaccagttc tggtggtaag gactggagag gcggtagatc ttgccaggga     660
   aatatcattc cttcatctac tggtgcagct aaggctgtag ggaaaatctt gcctgaactt     720
   aatggtaaga tcaccggtat gtctataaga gtcccaacaa ttaatatttc cctggttgac     780
   ttgacattcc gtacagcaaa gaaaacttct tacgatgaca ttatgaaggc cctagaacaa     840
   agatctcgca gcgatatgaa gggtgttttg ggtgttacca aagacgccgt tgtgtcctct     900
   gacttcacat ccgattcacg ttcatctatt gttgatgcca aggccggtat tgaattgaac     960
   gaccattttt tcaaggtcct ttcttggtat gataatgaat atggttactc ttcaagagtg    1020
   gttgatttat ccattttcat ggctcaaaag gacttcgaag ctggtgttta a             1071
<210> 48
   <211> 356
   <212> PRT
   <213> Kluyveromyces lactis
<400> 48
   Met Pro Asp Met Thr Asn Glu Ser Ser Ser Lys Pro Ala Gln Ile Asn
   1               5                   10                  15
   Ile Gly Ile Asn Gly Phe Gly Arg Ile Gly Arg Leu Val Leu Arg Ala
               20                  25                  30
   Ala Leu Thr His Pro Glu Val Lys Val Arg Leu Ile Asn Asn Pro Ser
           35                  40                  45
   Thr Thr Pro Glu Tyr Ala Ala Tyr Leu Phe Lys Tyr Asp Ser Thr His
       50                  55                  60
   Gly Lys Tyr Arg Gly Glu Val Glu Phe Asp Asp Glu Arg Ile Ile Ile
   65                  70                  75                  80
   Gln Asn Asp His Val Ser Ala His Ile Pro Leu Ser His Phe Arg Glu
                   85                  90                  95
   Pro Glu Arg Ile Pro Trp Ala Ser Tyr Asn Val Asp Tyr Val Ile Asp
   100 105 110
   Ser Thr Gly Val Phe Lys Glu Val Asp Thr Ala Ser Arg His Lys Gly
   115 120 125
   Val Lys Lys Val Ile Ile Thr Ala Pro Ser Lys Thr Ala Pro Met Tyr
   130 135 140
   Val Tyr Gly Val Asn His Val Lys Tyr Asn Pro Leu Thr Asp His Val
   145 150 155 160
   Val Ser Asn Ala Ser Cys Thr Thr Asn Cys Leu Ala Pro Leu Val Lys
   165 170 175
   Ala Leu Asp Asp Glu Phe Gly Ile Glu Glu Ala Leu Met Thr Thr Ile
   180 185 190
   His Ala Thr Thr Ala Ser Gln Lys Thr Val Asp Gly Thr Ser Ser Gly
   195 200 205
   Gly Lys Asp Trp Arg Gly Gly Arg Ser Cys Gln Gly Asn Ile Ile Pro
   210 215 220
   Ser Ser Thr Gly Ala Ala Lys Ala Val Gly Lys Ile Leu Pro Glu Leu
   225 230 235 240
   Asn Gly Lys Ile Thr Gly Met Ser Ile Arg Val Pro Thr Ile Asn Ile
   245 250 255
   Ser Leu Val Asp Leu Thr Phe Arg Thr Ala Lys Lys Thr Ser Tyr Asp
   260 265 270
   Asp Ile Met Lys Ala Leu Glu Gln Arg Ser Arg Ser Asp Met Lys Gly
   275 280 285
   Val Leu Gly Val Thr Lys Asp Ala Val Val Ser Ser Asp Phe Thr Ser
   290 295 300
   Asp Ser Arg Ser Ser Ile Val Asp Ala Lys Ala Gly Ile Glu Leu Asn
   305 310 315 320
   Asp His Phe Phe Lys Val Leu Ser Trp Tyr Asp Asn Glu Tyr Gly Tyr
   325 330 335
   Ser Ser Arg Val Val Asp Leu Ser Ile Phe Met Ala Gln Lys Asp Phe
   340 345 350
   Glu Ala Gly Val
   355
<210> 49
   <211> 1023
   <212> DNA
   <213> Bacillus subtilis
<400> 49
   atgaaggtaa aagtagcgat caacgggttt ggaagaatcg gaagaatggt ttttagaaaa      60
   gcgatgttag acgatcaaat tcaagtagtg gccattaacg ccagctattc cgcagaaacg     120
   ctggctcatt taataaagta tgacacaatt cacggcagat acgacaaaga ggttgtggct     180
   ggtgaagata gcctgatcgt aaatggaaag aaagtgcttt tgttaaacag ccgtgatcca     240
   aaacagctgc cttggcggga atatgatatt gacatagtcg tcgaagcaac agggaagttt     300
   aatgctaaag ataaagcgat gggccatata gaagcaggtg caaaaaaagt gattttgacc     360
   gctccgggaa aaaatgaaga cgttaccatt gtgatgggcg taaatgagga ccaattcgac     420
   gctgagcgcc atgtcattat ttcaaatgcg tcatgcacga caaattgcct tgcgcctgtt     480
   gtaaaagtgc tggatgaaga gtttggcatt gagagcggtc tgatgactac agttcatgcg     540
   tatacgaatg accaaaaaaa tattgataac ccgcacaaag atttgcgccg ggcgcgggct     600
   tgcggtgaat ccatcattcc aacaacaaca ggagcggcaa aggcgctttc gcttgtgctg     660
   ccgcatctga aaggaaaact tcacggcctc gccttgcgtg tccctgttcc gaacgtctca     720
   ttggttgatc tcgttgttga tctgaaaacg gatgttacgg ctgaagaagt aaacgaggca     780
   tttaaacgcg ctgccaaaac gtcgatgtac ggtgtacttg attactcaga tgaaccgctc     840
   gtttcgactg attataatac gaatccgcat tcagcggtca ttgacgggct tacaacaatg     900
   gtaatggaag acaggaaagt aaaggtgctg gcgtggtatg acaacgaatg gggctactcc     960
   tgcagagttg ttgatctaat ccgccatgta gcggcacgaa tgaaacatcc gtctgctgta    1020
   taa                                                                  1023
<210> 50
   <211> 340
   <212> PRT
   <213> Bacillus subtilis
<400> 50
   Met Lys Val Lys Val Ala Ile Asn Gly Phe Gly Arg Ile Gly Arg Met
   1               5                   10                  15
   Val Phe Arg Lys Ala Met Leu Asp Asp Gln Ile Gln Val Val Ala Ile
               20                  25                  30
   Asn Ala Ser Tyr Ser Ala Glu Thr Leu Ala His Leu Ile Lys Tyr Asp
           35                  40                  45
   Thr Ile His Gly Arg Tyr Asp Lys Glu Val Val Ala Gly Glu Asp Ser
       50                  55                  60
   Leu Ile Val Asn Gly Lys Lys Val Leu Leu Leu Asn Ser Arg Asp Pro
   65 70 75 80
   Lys Gln Leu Pro Trp Arg Glu Tyr Asp Ile Asp Ile Val Val Glu Ala
   85 90 95
   Thr Gly Lys Phe Asn Ala Lys Asp Lys Ala Met Gly His Ile Glu Ala
   100 105 110
   Gly Ala Lys Lys Val Ile Leu Thr Ala Pro Gly Lys Asn Glu Asp Val
   115 120 125
   Thr Ile Val Met Gly Val Asn Glu Asp Gln Phe Asp Ala Glu Arg His
   130 135 140
   Val Ile Ile Ser Asn Ala Ser Cys Thr Thr Asn Cys Leu Ala Pro Val
   145 150 155 160
   Val Lys Val Leu Asp Glu Glu Phe Gly Ile Glu Ser Gly Leu Met Thr
   165 170 175
   Thr Val His Ala Tyr Thr Asn Asp Gln Lys Asn Ile Asp Asn Pro His
   180 185 190
   Lys Asp Leu Arg Arg Ala Arg Ala Cys Gly Glu Ser Ile Ile Pro Thr
   195 200 205
   Thr Thr Gly Ala Ala Lys Ala Leu Ser Leu Val Leu Pro His Leu Lys
   210 215 220
   Gly Lys Leu His Gly Leu Ala Leu Arg Val Pro Val Pro Asn Val Ser
   225 230 235 240
   Leu Val Asp Leu Val Val Asp Leu Lys Thr Asp Val Thr Ala Glu Glu
   245 250 255
   Val Asn Glu Ala Phe Lys Arg Ala Ala Lys Thr Ser Met Tyr Gly Val
   260 265 270
   Leu Asp Tyr Ser Asp Glu Pro Leu Val Ser Thr Asp Tyr Asn Thr Asn
   275 280 285
   Pro His Ser Ala Val Ile Asp Gly Leu Thr Thr Met Val Met Glu Asp
   290 295 300
   Arg Lys Val Lys Val Leu Ala Trp Tyr Asp Asn Glu Trp Gly Tyr Ser
   305 310 315 320
   Cys Arg Val Val Asp Leu Ile Arg His Val Ala Ala Arg Met Lys His
   325 330 335
   Pro Ser Ala Val